(19) Europäisches Patentamt
European Patent Office
Office européen des brevets

(11) EP 4 759 813 A1

(12) EUROPEAN PATENT APPLICATION
published in accordance with Art. 153(4) EPC

(43) Date of publication:
**17.06.2026 Bulletin 2026/25**

(21) Application number: **24853705.2**

(22) Date of filing: **09.08.2024**

(51) International Patent Classification (IPC):
***C07D 487/04*** (2006.01) ***C07D 519/00*** (2006.01)
***A61K 31/497*** (2006.01) ***A61K 31/5377*** (2006.01)
***A61K 31/4188*** (2006.01) ***A61K 31/454*** (2006.01)
***A61K 31/501*** (2006.01) ***A61K 31/496*** (2006.01)
***A61P 37/02*** (2006.01) ***A61P 35/00*** (2006.01)
***A61P 3/10*** (2006.01) ***A61P 27/02*** (2006.01)
***A61P 1/00*** (2006.01) ***A61P 37/08*** (2006.01)
***A61P 25/00*** (2006.01) ***A61P 17/00*** (2006.01)
***A61P 37/06*** (2006.01) ***A61P 11/00*** (2006.01)
***A61P 11/06*** (2006.01)

(52) Cooperative Patent Classification (CPC):
**A61K 31/4188; A61K 31/454; A61K 31/496; A61K 31/497; A61K 31/501; A61K 31/5377; A61P 1/00; A61P 3/10; A61P 11/00; A61P 11/06; A61P 17/00; A61P 25/00; A61P 27/02; A61P 35/00; A61P 37/02;** (Cont.)

(86) International application number:
**PCT/CN2024/111108**

(87) International publication number:
**WO 2025/036296 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.08.2023 CN 202311017417**

(71) Applicant: **Medinno Pharmaceutical Technology (Zhuhai) Co., Ltd.**
**Zhuhai, Guangdong 519000 (CN)**

(72) Inventors:
- **LU, Liang**
  **Zhuhai, Guangdong 519000 (CN)**
- **HUANG, Hai**
  **Zhuhai, Guangdong 519000 (CN)**
- **ZHANG, Longzheng**
  **Zhuhai, Guangdong 519000 (CN)**
- **ZHAO, Saisai**
  **Zhuhai, Guangdong 519000 (CN)**
- **ZHANG, Jixuan**
  **Zhuhai, Guangdong 519000 (CN)**
- **ZHU, Junjie**
  **Zhuhai, Guangdong 519000 (CN)**

(74) Representative: **Kraus & Lederer PartGmbB**
**Thomas-Wimmer-Ring 15**
**80539 München (DE)**

# (54) JAK INHIBITOR COMPOUND AND USE THEREOF

(57) The present invention relates to a JAK inhibitor compound and the use thereof. Specifically, disclosed in the present invention is a compound as shown in formula (I), or an optical isomer, a geometric isomer, a tautomer or an isomer mixture thereof, or a pharmaceutically acceptable salt thereof, or a prodrug thereof, or a metabolite thereof. The present invention also relates to the use of the compound in medicine.



Processed by Luminess, 75001 PARIS (FR)

(Cont. next page)

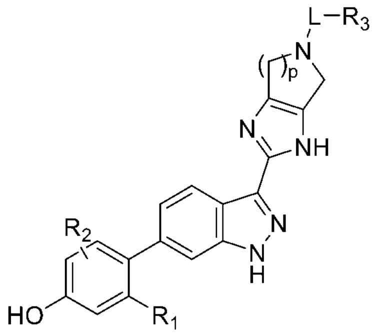

(I)

(52) Cooperative Patent Classification (CPC): (Cont.)
**A61P 37/06; A61P 37/08; C07D 487/04;**
**C07D 519/00**

## Description

## CROSS REFERENCE TO RELATED APPLICATION

This application claims priority to the Chinese Patent Application No. 202311017417.4 entitled "JAK INHIBITOR COMPOUND AND USE THEREOF" and filed on August 11, 2023, which is incorporated herein by reference in its entirety.

## TECHNICAL FIELD

The present disclosure provides a class of novel compounds with pharmacological activity, which can be used to inhibit Janus kinase (JAK). The present disclosure also relates to a composition comprising the compound, and use of the compound and the composition in the preparation of a medicament for the treatment and/or prevention of JAK-related diseases or disorders.

## BACKGROUND

Protein kinases are a family of enzymes that catalyze phosphorylation of specific residues in proteins, and are broadly classified into tyrosine and serine/threonine kinases. Inappropriate kinase activities caused by mutations, overexpression or inappropriate regulation, abnormal regulation or dysregulation, and excessive or insufficient production of growth factors or cytokines are involved in many diseases, including but not limited to cancers, cardiovascular diseases, allergies, asthma and other respiratory diseases, autoimmune diseases, inflammatory diseases, bone diseases, metabolic disorders and neurological and neurodegenerative disorders (such as Alzheimer's disease). Inappropriate kinase activity triggers a variety of biological cell responses associated with cell growth, cell differentiation, cell function, survival, apoptosis, and cell motility related to the aforementioned diseases and other related diseases. Therefore, protein kinases have become an important class of enzymes as targets for therapeutic intervention. In particular, the JAK family of cellular protein tyrosine kinases plays an important role in cytokine signal transduction (Kisseleva et al., Gene, 2002, 285, 1; Yamaoka et al., Genome Biology 2004, 5, 253).

Since the first JAK inhibitor was discovered in the early 1990s, the development of JAK inhibitors has gone through nearly 30 years. JAK is a family of intracellular non-receptor tyrosine kinases, which plays an important role in cytokine receptor signaling pathway by interacting with signal transducer and activator of transcription (STAT). JAK/STAT signaling pathway is involved in many important biological processes such as cell proliferation, differentiation, apoptosis and immune regulation. Compared with other signal pathways, the transmission process of this signal pathway is relatively simple. It is mainly composed of three components, namely tyrosine kinase associated receptor, tyrosine kinase JAK, signal transducer and activator of transcription STAT.

Many cytokines and growth factors transmit signals through the JAK-STAT signal pathway, including interleukins (such as IL-2 to IL-7, IL-9, IL-10, IL-15, IL-21, and the like), GM-CSF ( granulocyte/macrophage colony stimulating factor), GH (growth hormone), EGF (epidermal growth factor), PRL (prolactin), EPO (erythropoietin), TPO (thrombopoietin), PDGF (platelet derived factors) and interferons (including IFN-$\alpha$, IFN-$\beta$, IFN-$\gamma$ and the like) and so on. These cytokines and growth factors have corresponding receptors on the cell membrane. The common feature of these receptors is that the receptor itself does not have kinase activity, but its intracellular segment has a binding site for tyrosine kinase JAK. After the receptor binds to a ligand, tyrosine residues of various target proteins are phosphorylated by activation of JAK that binds to the receptor to realize signal transfer from the extracellular to the intracellular.

JAK is a cytoplasmic tyrosine kinase that transduces cytokine signals from membrane receptors to STAT transcription factors. As mentioned above, JAK is the abbreviation of Janus kinase in English. In Roman mythology, Janus is the double-faced god in charge of the beginning and the end. The reason why it is called Janus kinase is that JAK can phosphorylate cytokine receptors that it binds to, and also phosphorylate multiple signal molecules containing specific SH2 domains. The JAK protein family includes 4 members: JAK1, JAK2, JAK3, and TYK2. They have 7 JAK homology domains (JH) in structure in which the JH1 domain is a kinase domain having the function of encoding kinase proteins; JH2 domain is a "pseudo" kinase domain, which regulates the activity of JH1; and JH3-JH7 constitute a four-in-one domain, which regulates the binding of JAK proteins to receptors.

STAT is a type of cytoplasmic protein that can bind to DNA in the regulatory region of target genes, and is a downstream substrate of JAK. Seven members of the STAT family have been discovered, namely STAT1, STAT2, STAT3, STAT4, STAT5A, STAT5B and STAT6. STAT protein can be divided into the following functional segments in structure including N-terminal conserved sequence, DNA binding region, SH3 domain, SH2 domain and C-terminal transcription activation region. Among them, the segment of the most conserved in sequence and most important in function is the SH2 domain, which has the same core sequence "GTFLLRFSS" as the SH2 domain of tyrosine kinase Src.

JAK-STAT signaling pathway has a wide range of functions and is involved in many important biological processes such as cell proliferation, differentiation, apoptosis, and immune regulation. At present, the research related

to disease and drug innovation mainly focuses on inflammatory diseases and neoplastic diseases in which the inflammatory diseases mainly include rheumatoid arthritis, canine dermatitis, psoriasis, ulcerative colitis and Crohn's disease; and the neoplastic diseases mainly involve myelofibrosis, polycythemia vera and primary platelets hyperplasia. In addition, mutations in JAK molecule itself can also cause acute myeloid leukemia (AML), acute lymphocytic leukemia (ALL), ductal breast carcinoma and non-small cell lung cancer (NSCLC), polycythemia vera (PV), essential thrombocythemia (ET), idiopathic myelofibrosis (IMF), chronic myeloid leukemia (CML), and the like.

JAK is a very important drug target. JAK inhibitors developed for this target are mainly used to screen therapeutic drugs for blood system diseases, tumors, rheumatoid arthritis and psoriasis. JAK-1, JAK-2 and TYK-2 are expressed in various tissue cells of human body. JAK-3 is mainly expressed in various hematopoietic tissue cells, mainly in bone marrow cells, thymocytes, NK cells and activated B lymphocytes and T lymphocytes. Studies have shown that JAK2 inhibitors are suitable for myeloproliferative diseases (Santos et al., Blood, 2010, 115:1131; Barosi G. and Rosti V., Curr. Opin. Hematol., 2009, 16:129; Atallah E. and Versotvsek S., 2009 Exp. Rev. Anticancer Ther. 9:663), and JAK3 inhibitors are suitable as immunosuppressive agents (such as, US Patent No. 6,313, 129; Borie et al., Curr. Opin. Investigational Drugs, 2003, 4 :1297).

Currently, JAK inhibitors approved by the FDA and EMA include Tofacitinib, Ruxolitinib, and Oclacitinib. JAK inhibitors in the middle and late stages of clinical research include Filgotinib, Peficitinib and so on.

Tofacitinib, a JAK3 inhibitor, was developed by Pfizer and was approved by the FDA in November 2012 for the treatment of moderate to severe rheumatoid arthritis (RA) due to inadequate response or intolerance to methotrexate in adult patients. It is the first oral JAK inhibitor approved for RA treatment. After that, it was approved by Japan PMDA for listing in March 2013 under the trade name Xeljanz. On March 16, 2017, Pfizer China announced that the CFDA had formally approved Pfizer's application for the marketing of the oral JAK inhibitor. It was reported that the drug was approved for the treatment of adult patients with moderate to severe rheumatoid arthritis having inadequate response or intolerance to methotrexate. At present, Tofacitinib is close to being approved for indications such as psoriasis, ulcerative colitis, juvenile idiopathic arthritis; and clinical trials for the treatment of indications such as Crohn's disease and alopecia areata have also entered the mid- to late-stage. The main side effects of Tofacitinib are serious infection rate and increased low-density lipoprotein level. The most common adverse effects are upper respiratory tract infection, headache, diarrhea, nasal congestion, sore throat and nasopharyngitis. In addition, it has been have reported that Tofacitinib can cause side effects such as anemia and neutropenia in clinical studies.

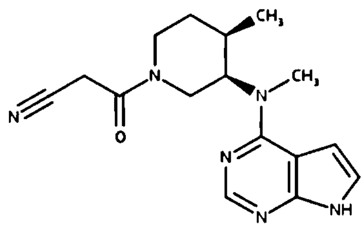

Tofacitinib

Ruxolitinib, a JAK1 and JAK2 inhibitor, was jointly developed by Incyte and Novartis and was approved by the FDA of US in November 2011. It is also the first approved drug specifically for the treatment of myelofibrosis. It was approved by EMA in August 2012 and approved by Japan PMDA for listing in July 2014. The drug is sold by Incyte in the United States under the trade name Jakafi; and is sold by Novartis in Europe and Japan under the trade name Jakavi. Ruxolitinib is under a number of clinical trials in the middle and late stages, wherein the indications include a variety of cancers, GVHD (rejection reaction), alopecia areata, allergic dermatitis, rheumatoid arthritis, vitiligo, psoriasis, and the like. The most common hematological adverse effects with an incidence of > 20% of Ruxolitinib are low platelet counts and anemia. The most common non-hematological adverse effects with an incidence of > 10% are ecchymosis, dizziness and headache.

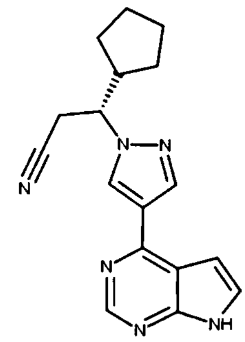

Ruxolitinib

Olatinib, approved by the FDA of US in 2013, is used to control itching and atopic dermatitis caused by canine allergic dermatitis. Olatinib is a new type of JAK and JAK1-dependent cytokine inhibitor. Olatinib is not only a very effective JAK1 inhibitor, but can also inhibit the function of JAK1-dependent cytokines in some anti-allergic, inflammation and pruritic reactions. It has little effect on cytokines that are not involved in activation of JAK1. Oral administration of 0.4-0.6 mg/kg Olatinib twice a day is safe and effective for the treatment of itching caused by allergic dermatitis. During the treatment, Olatinib can relieve itching within 24 hours. In experiments, more than 70% of experimental animals (dogs) alleviated the itching response by more than 50% on the 7th day. However, Olatinib cannot yet be used to treat human diseases.

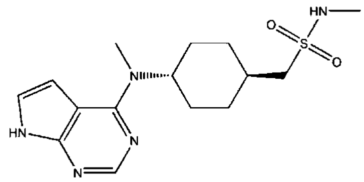

Oclacitinib

Filgotinib, a JAK1 inhibitor, passed Phase III clinical trials in September 2018 for the treatment of rheumatoid arthritis. At the same time, the study of Filgotinib for the treatment of ulcerative colitis and Crohn's disease is currently in clinical phase II/III trials. Filgotinib is a selective JAK1 inhibitor with IC50 of 10 nM, 28 nM, 810 nM and 116 nM for JAK1, JAK2, JAK3 and TYK2, respectively.

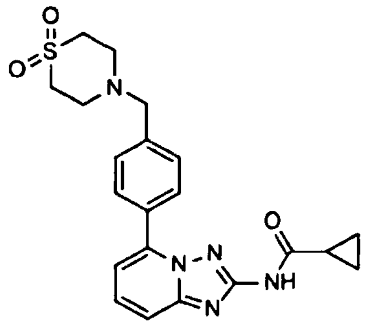

Filgotinib

Peficitinib, a JAK1 and JAK3 inhibitor, developed by Astellas, is currently in Phase III clinical trial for the treatment of rheumatoid arthritis. The Phase II clinical study for the treatment of psoriasis has been completed. Peficitinib is a new oral JAK inhibitor. Peficitinib inhibits the enzyme activities of JAK1, JAK2, JAK3 and TYK2 with IC50 of 3.9 nM, 5.0 nM, 0.71 nM and 4.8 nM, respectively.

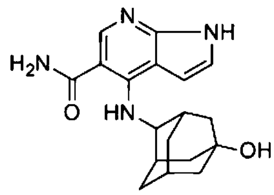

Peficitinib

Although some JAK inhibitors have been approved for listing, and a large number of JAK inhibitors are still in clinical research, these JAK inhibitors are not satisfactory in terms of efficacy or safety. Therefore, there is always a need for JAK inhibitors with better efficacy and/or fewer side effects.

## SUMMARY

It is one object of the present disclosure to provide a novel JAK inhibitor alternative to existing JAK inhibitors, so as to provide more options for the treatment of JAK-related diseases.

A further object of the present disclosure is to provide a novel JAK inhibitor with better efficacy and/or better safety than existing JAK inhibitors.

In a first aspect, the present disclosure provides a compound of Formula (I) as a JAK inhibitor

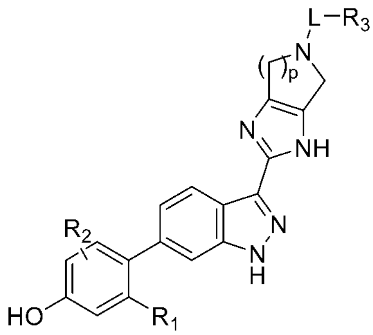

(I)

or an optical isomer thereof, a geometric isomer thereof, a tautomer thereof or a mixture of various isomers, or a pharmaceutically acceptable salt thereof, or a prodrug thereof, or a metabolite thereof,

wherein

L is C=O, O=S=O, $CH_2$ or a covalent bond; and

$R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on R1 is substituted by a D atom; and

$R_3$ is H, -N($R_{17}$)($R_{18}$), $C_{1-6}$ alkoxy, -$SR_{12}$, -$OR_{12}$, -CN, halogen, -$NO_2$, -$SF_5$, -S-$C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, or 5-11 membered bicyclic heteroalkyl, and $R_{13}$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, and 5-11 membered bicyclic heteroalkyl, and are optionally substituted by one or more substituents each independently selected from the group consisting of: -OH, -CN, -SH, halogen, -$NO_2$, -$SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, - N($R_9$)($R_{10}$), -N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(=O)$_2$-N($R_9$)($R_{10}$), -$SR_{12}$, and -$OR_{12}$, wherein the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are

each optionally substituted by 1, 2, or 3 substituents each independently selected from the group consisting of: halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), - C(=O)-O$R_{12}$, -C(=O)-H, -C(=O)-$R_{12}$, -C(=O)-N($R_9$)($R_{10}$), -N($R_{11}$)(S(=O)$_2R_{12}$), -S(=O)$_2$-N($R_9$)($R_{10}$), -S$R_{12}$, and -O$R_{12}$, and -OR12; or $R_{17}$, $R_{18}$, and the nitrogen atom to which they are attached together form a 3-14 membered ring.

0, 1, 2, 3 or 4 $R_2$(s) are present in formula (I), and $R_2$ is selected from H, halogen, -OH, -$NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), -N($R_{11}$)(C(=O)$R_{12}$), - C(=O)-N($R_9$)($R_{10}$), -C(=O)-$R_{12}$, -C(=O)-O$R_{12}$, -OC(=O)$R_{12}$, -N($R_{11}$)(S(=O)$_2R_{12}$), -S(=O)$_2$-N($R_9$)($R_{10}$), -S$R_{12}$ and -O$R_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-O$R_{12}$, -C(=O)H, -C(=O)$R_{12}$, - C(=O)-N($R_9$)($R_{10}$), -N($R_{11}$)(S(=O)$_2R_{12}$), -S(=O)$_2$-N($R_9$)($R_{10}$), -S$R_{12}$ and -O$R_{12}$; and

p is 1, 2 or 3; and

$R_a$ is selected from H, halogen, -OH, -$NO_2$, -CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15membered tricyclyl, $C_{5-11}$bicycloalkyl, 5-11 membered bicyclic heteroalkyl, -N($R_9$)($R_{10}$), - N($R_{11}$)(C(=O)$R_{12}$), -C(=O)-N($R_9$)($R_{10}$), -C(=O)-$R_{12}$, -C(=O)-O$R_{12}$, -OC(=O)$R_{12}$, - N($R_{11}$)(S(=O)$_2R_{12}$), -S(=O)$_2$-N($R_9$)($R_{10}$), -S$R_{12}$ and -O$R_{12}$, in which the -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_b$(s); and

$R_b$ is selected from H, halogen, -OH, -$NO_2$, -CN, -$SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, -N($R_5$)($R_6$), - N($R_{11}$)(C(=O)$R_{12}$), -CON($R_7$)($R_8$), -C(=O)-$R_{12}$, -C(=O)-O$R_{12}$, -OC(=O)$R_{12}$, - N($R_{11}$)(S(=O)$_2R_{12}$), -S(=O)$_2$-N($R_9$)($R_{10}$), -S$R_{12}$ and -O$R_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen, - CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-O$R_{12}$, -C(=O)H, -C(=O)$R_{12}$, -C(=O)-N($R_9$)($R_{10}$), - N($R_{11}$)(S(=O)$_2R_{12}$), -S(=O)$_2$-N($R_9$)($R_{10}$), -S$R_{12}$ and -O$R_{12}$; and

$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option in the group is optionally substituted with 1, 2, 3 or 4 substituent(s) each independently selected from the group consisting of halogen, -$CF_3$, -OH, -$NH_2$, - NH($CH_3$), -N($CH_3$)$_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy and $C_{1-4}$ haloalkoxy.

In some preferred embodiments of the present disclosure, in formula (I), L is C=O, O=S=O, or $CH_2$. In some particularly preferred embodiments of the present disclosure, in formula (I), L is C=O. In some particularly preferred embodiments of the present disclosure, in formula (I), L is O=S=O. In some particularly preferred embodiments of the present disclosure, in formula (I), L is $CH_2$. In other embodiments of the present disclosure, in formula (I), L is a covalent bond.

In some preferred embodiments of the present disclosure, in formula (I), $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R^1$ is replaced by a D atom. In some more preferred embodiments of the present disclosure, in formula (I), $R_1$ is -S-$C_{1-4}$ alkyl, $C_{1-6}$ alkyl, $C_{1-6}$ alkoxy, or $C_{1-6}$ haloalkoxy, wherein at least one H atom on $R^1$ is replaced by a D atom, preferably all H atoms on $R^1$ are replaced by D atoms. In some even more preferred embodiments of the present disclosure, in formula (I), $R_1$ is $C_{1-6}$ alkyl, wherein at least one H atom on $R^1$ is replaced by a D atom, preferably all H atoms on $R^1$ are replaced by D atoms. In some particularly preferred embodiments of the present disclosure, in formula (I), $R_1$ is $C_{1-6}$ alkyl, wherein all H atoms on $R^1$ are replaced by D atoms, for example, $R^1$ is $CD_3$, $C_2D_6$, $C_3D_8$, or $C_4D_{10}$.

In the above embodiments of the present disclosure, substituting H atoms on $R_1$ of the compound of formula (I) with deuterium (i.e., $^2H$, also abbreviated as D), a heavier isotope, may provide certain unexpected therapeutic benefits. The inventors of the present application speculate that these benefits may arise from significantly greater metabolic stability (e.g., substantially increased *in vivo* half-life or substantially reduced dosage requirements). Furthermore, the inventors of the present application have discovered that after substituting H atoms on $R_1$ of the compound of formula (I) with deuterium, the activity of the compound of formula (I) in inhibiting JAK1, JAK2, JAK3, and/or TYK2 enzymes is surprisingly enhanced, for example, manifested as the $IC_{50}$ value of the deuterated compound being reduced to one-half, one-third, one-quarter, or even lower compared to the $IC_{50}$ of the corresponding non-deuterated compound. It is generally believed in the field that simple deuteration does not have such a dramatic impact on pharmacological activity.

In some preferred embodiments of the present disclosure, in formula (I), $R_3$ is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, or 5-11 membered bicyclic heteroalkyl, and $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted by one or more substituents selected from -OH, - CN, -SH, halogen, $-NO_2$, and $-SF_5$ (wherein $R_3$ is optionally substituted with 1, 2, 3, or 4 $R_a$(s)). In some preferred embodiments of the present disclosure, in formula (I), R3 is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11-15 membered tricyclyl, and $R_{17}$ and $R_{18}$ are as defined above (wherein $R_3$ is optionally substituted with 1, 2, 3, or 4 $R_a$(s)). In some preferred embodiments of the present disclosure, in formula (I), R3 is H, $-N(R_{17})(R_{18})$, $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_{17}$ and $R_{18}$ are as defined above (wherein $R_3$ is optionally substituted with 1, 2, 3, or 4 $R_a$(s)). In some preferred embodiments of the present disclosure, in formula (I), $R_3$ is - $N(R_{17})(R_{18})$, $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, $C_{5-11}$ bicycloalkyl, $C_{7-11}$ bicyclic aryl, 7-11 membered bicyclic heteroaryl, or 5-11 membered bicyclic heteroalkyl, and $R_{17}$ and $R_{18}$ are as defined above (wherein $R_3$ is optionally substituted with 1, 2, 3, or 4 $R_a$(s)). In some preferred embodiments of the present disclosure, in formula (I), R3 is $-N(R_{17})(R_{18})$, $C_{1-6}$ alkoxy, $C_{1-6}$ alkyl, $C_{3-7}$ cycloalkyl, 3-7 membered heterocycloalkyl, phenyl, or 5-6 membered heteroaryl, and $R_{17}$ and $R_{18}$ are as defined above (wherein $R_3$ is optionally substituted with 1, 2, or 3 $R_a$(s)). In some preferred embodiments of the present disclosure, in formula (I), $R_3$ is $-N(R_{17})(R_{18})$, $C_{1-6}$ alkoxy, $C_{1-6}$ alkyl, $C_{3-7}$ cycloalkyl, or 3-7 membered heterocycloalkyl, and $R_{17}$ and $R_{18}$ are as defined above (wherein $R_3$ is optionally substituted with 1, 2, or 3 $R_a$(s)). In some preferred embodiments of the present disclosure, in formula (I), $R_3$ is $-N(R_{17})(R_{18})$, $C_{1-3}$ alkoxy, $C_{3-6}$ cycloalkyl, 3-7 membered heterocycloalkyl, phenyl, 5-6 membered heteroaryl, or $C_{1-4}$ alkyl, and $R_{17}$ and $R_{18}$ are as defined above (wherein $R_3$ is optionally substituted with 1, 2, or 3 $R_a$(s)). In some preferred embodiments of the present disclosure, in formula (I), $R_3$ is - N(H)($C_{1-3}$ alkyl), -N(H)(3-6 membered cycloalkyl), -N(H)(3-7 membered heterocycloalkyl), -N($C_{1-3}$ alkyl)($C_{1-3}$ alkyl), $C_{1-3}$ alkoxy, $C_{3-6}$ cycloalkyl, 4-6 membered azacycloalkyl or oxacycloalkyl, phenyl, 5-6 membered azaaryl, or $C_{1-4}$ alkyl; or $R_3$ is $-N(R_{17})(R_{18})$, and $R_{17}$, $R_{18}$, and the nitrogen atom to which they are attached together form a 4-10 membered ring (wherein $R_3$ is optionally substituted with 1, 2, 3, or 4 $R_a$(s)). In some particularly preferred embodiments of the present disclosure, in formula (I), $R_3$ is cyclopropyl, cyclobutyl, cyclopentyl, cyclohexyl, methyl, ethyl, propyl, butyl, methoxy, ethoxy, propoxy, $-N(H)(CH_3)$, $-N(H)(CH_2CH_3)$, $-N(H)(CH_2CH_2OH)$, $-N(H)(CH_2CH_2CH_2OH)$, $-N(H)(CH_2CH(CH_3)OH)$, - $N(H)(CH_2CH_2CN)$, $-N(CH_3)(CH_3)$, -N(H)(cyclopropyl), -N(H)(cyclobutyl), - N(H)(tetrahydrofuranyl), pyrazinyl, pyridazinyl, pyrrolidinyl, pyrazolyl, piperidinyl, phenyl, azetidinyl, morpholinyl, piperazinyl, or tetrahydropyranyl; or $R_3$ is $-N(R_{17})(R_{18})$, and $R_{17}$, $R_{18}$, and the nitrogen atom to which they are attached together form a 5-membered ring (wherein $R_3$ is optionally substituted with 1, 2, or 3 $R_a$(s)). In some particularly preferred embodiments of the present disclosure, in formula (I), $R_3$ is cyclopropyl. In some particularly preferred embodiments of the present disclosure, in formula (I), $R_3$ is cyclobutyl. In some particularly preferred embodiments of the present disclosure, in formula (I), $R_3$ is cyclopentyl. In some particularly preferred embodiments of the present disclosure, in formula (I), $R_3$ is cyclohexyl. In some particularly preferred embodiments of the present disclosure, in formula (I), $R_3$ is methyl. In some particularly preferred embodiments of the present disclosure, in formula (I), $R_3$ is ethyl. In some particularly preferred embodiments of the present disclosure, in formula (I), $R_3$ is propyl. In some particularly preferred embodiments of the present disclosure, in formula (I), $R_3$ is butyl. In some particularly preferred embodiments of the present disclosure, in formula (I), $R_3$ is pyrazinyl. In some particularly preferred embodiments of the present disclosure, in formula (I), $R_3$ is pyridazinyl. In some particularly preferred embodiments of the present disclosure, in formula (I), $R_3$ is pyrrolidinyl. In some particularly preferred embodiments of the present disclosure, in formula (I), $R_3$ is pyrazolyl. In some particularly preferred embodiments of the present disclosure, in formula (I), $R_3$ is piperidinyl. In some particularly preferred embodiments of the present disclosure, in formula (I), $R_3$ is phenyl. In some particularly preferred embodiments of the present disclosure, in formula (I), $R_3$ is azetidinyl. In some particularly preferred embodiments of the present disclosure, in formula (I), $R_3$ is morpholinyl. In some particularly preferred embodiments of the present disclosure, in formula (I), $R_3$ is piperazinyl. In some particularly preferred embodiments of the present disclosure, in formula (I), $R_3$ is tetrahydropyranyl. In some particularly preferred embodiments of the present disclosure, in formula (I), $R_3$ is methoxy. In some particularly

preferred embodiments of the present disclosure, in formula (I), $R_3$ is ethoxy. In some particularly preferred embodiments of the present disclosure, in formula (I), $R_3$ is -N(H)($CH_3$). In some particularly preferred embodiments of the present disclosure, in formula (I), $R_3$ is - N(H)($CH_2CH_3$). In some particularly preferred embodiments of the present disclosure, in formula (I), $R_3$ is -N(H)($CH_2CH_2OH$). In some particularly preferred embodiments of the present disclosure, in formula (I), $R_3$ is -N(H)($CH_2CH_2CH_2OH$). In some particularly preferred embodiments of the present disclosure, in formula (I), $R_3$ is -N(H)($CH_2CH(CH_3)OH$). In some particularly preferred embodiments of the present disclosure, in formula (I), $R_3$ is -N(H)($CH_2CH_2CN$). In some particularly preferred embodiments of the present disclosure, in formula (I), $R_3$ is -N($CH_3$)($CH_3$). In some particularly preferred embodiments of the present disclosure, in formula (I), $R_3$ is - N(H)(cyclopropyl). In some particularly preferred embodiments of the present disclosure, in formula (I), $R_3$ is -N(H)(cyclobutyl). In some particularly preferred embodiments of the present disclosure, in formula (I), $R_3$ is -N(H)(tetrahydrofuranyl). In some particularly preferred embodiments of the present disclosure, in formula (I), $R_3$ is -N($R_{17}$)($R_{18}$), and $R_{17}$, $R_{18}$, and the nitrogen atom to which they are attached together form a 5-membered ring, and the ring is optionally substituted by one or more substituents selected from -OH, -CN, -SH, halogen, -$NO_2$, and -$SF_5$.

In some preferred embodiments of the present disclosure, in formula (I), $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted by one or more substituents selected from -OH, -CN, -SH, halogen, -$NO_2$, and -$SF_5$. In some preferred embodiments of the present disclosure, in formula (I), $R_{17}$ and $R_{18}$ are each independently selected from H, $C_{1-6}$ alkyl, $C_{3-7}$ cycloalkyl, and $C_{3-7}$ heterocycloalkyl, and are optionally substituted by one or more substituents selected from -OH, -CN, -SH, halogen, - $NO_2$, and -$SF_5$. In some preferred embodiments of the present disclosure, in formula (I), $R_{17}$ and $R_{18}$ are each independently selected from H, $C_{1-6}$ alkyl, $C_{3-7}$ cycloalkyl, and $C_{3-7}$ heterocycloalkyl, and are optionally substituted by one or more substituents selected from - OH and -CN. In some preferred embodiments of the present disclosure, in formula (I), $R_{17}$ and $R_{18}$ are each independently selected from H, methyl, ethyl, propyl, isopropyl, 3-membered cycloalkyl, 4-membered cycloalkyl, 5-membered cycloalkyl, 5-membered heterocycloalkyl, and 6-membered heterocycloalkyl, and are optionally substituted by one or more substituents selected from -OH and -CN. In some preferred embodiments of the present disclosure, in formula (I), $R_{17}$, $R_{18}$, and the nitrogen atom to which they are attached together form a 4-10 membered ring, and the ring is optionally substituted by one or more substituents selected from -OH, -CN, -SH, halogen, -$NO_2$, and -$SF_5$. In some preferred embodiments of the present disclosure, in formula (I), $R_{17}$, $R_{18}$ and the nitrogen atom to which they are attached together form a 5-membered ring, and the ring is optionally substituted by one or more substituents selected from -OH, -CN, -SH, halogen, -$NO_2$, and -$SF_5$.

In some particularly preferred embodiments of the present disclosure, in formula (I), L is C=O, and $R_3$ is -N($R_{17}$)($R_{18}$), $C_{3-7}$ cycloalkyl, 3-7 membered heterocycloalkyl, $C_{1-6}$ alkoxy, -OH, -SH, -CN, halogen, -$NO_2$, -$SF_5$, or -S-$C_{1-4}$ alkyl, and $R_3$ is substituted with 0, 1, 2, 3, or 4 $R^a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted by one or more substituents selected from -OH, -CN, -SH, halogen, -$NO_2$, and -$SF_5$; or $R_{17}$, $R_{18}$, and the nitrogen atom to which they are attached together form a 3-14 membered ring, and the ring is optionally substituted by one or more substituents selected from -OH, -CN, -SH, halogen, -$NO_2$, and -$SF_5$. In some particularly preferred embodiments of the present disclosure, in formula (I), L is C=O, and $R_3$ is -N($R_{17}$)($R_{18}$), $C_{3-7}$ cycloalkyl, 3-7 membered heterocycloalkyl, or $C_{1-6}$ alkoxy; wherein $R_{17}$ and $R_{18}$ are each independently selected from H, $C_{1-6}$ alkyl, $C_{3-7}$ cycloalkyl, and $C_{3-7}$ heterocycloalkyl, and are optionally substituted by one or more substituents selected from -OH, -CN, -SH, halogen, - $NO_2$, and -$SF_5$; or $R_{17}$, $R_{18}$ and the nitrogen atom to which they are attached together form a 3-10 membered ring, and the ring is optionally substituted by one or more substituents selected from -OH, -CN, -SH, halogen, -$NO_2$, and -$SF_5$. In some particularly preferred embodiments of the present disclosure, in formula (I), L is C=O, and $R_3$ is methoxy, ethoxy, propoxy, cyclopropyl, cyclobutyl, cyclopentyl, cyclohexyl, pyrrolidinyl, pyridinyl, morpholinyl, -N(H)($CH_3$), -N(H)($CH_2CH_3$), -N(H)($CH_2CH_2OH$), -N(H)($CH_2CH_2CH_2OH$), - N(H)($CH_2CH(CH_3)OH$), -N(H)($CH_2CH_2CN$), -N($CH_3$)($CH_3$), -N(H)(cyclopropyl), - N(H)(cyclobutyl), or -N(H)(tetrahydrofuranyl); or $R_3$ is -N($R_{17}$)($R_{18}$), and $R_{17}$, $R_{18}$ and the nitrogen atom to which they are attached together form a 5-membered ring, and the ring is optionally substituted by one or more substituents selected from -OH, -CN, -SH, halogen, - $NO_2$, and -$SF_5$. In some particularly preferred embodiments of the present disclosure, in formula (I), L is C=O, and $R_3$ is methoxy. In some particularly preferred embodiments of the present disclosure, in formula (I), L is C=O, and $R_3$ is ethoxy. In some particularly preferred embodiments of the present disclosure, in formula (I), L is C=O, and $R_3$ is cyclopropyl. In some particularly preferred embodiments of the present disclosure, in formula (I), L is C=O, and $R_3$ is morpholinyl. In some particularly preferred embodiments of the present disclosure, in formula (I), L is C=O, and $R_3$ is -N(H)($CH_3$). In some particularly preferred embodiments of the present disclosure, in formula (I), L is C=O, and $R_3$ is -N(H)($CH_2CH_3$). In some particularly preferred embodiments of the present disclosure, in formula (I), L is C=O, and $R_3$ is -N(H)($CH_2CH_2OH$). In some particularly preferred embodiments of the present disclosure, in formula (I), L is C=O, and $R_3$ is -N(H)($CH_2CH_2CN$). In some particularly preferred embodiments of the present disclosure, in formula (I), L is C=O, and $R_3$ is - N(H)($CH_2CH_2CH_2OH$). In some particularly preferred embodiments of the present disclosure, in formula (I), L is C=O, and $R_3$ is -N(H)($CH_2CH(CH_3)OH$). In some particularly preferred embodiments of the present disclosure, in formula (I), L is C=O, and $R_3$ is -N($CH_3$)($CH_3$). In some particularly preferred embodiments of the present disclosure, in formula (I), L is

C=O, and $R_3$ is -N(H)(cyclopropyl). In some particularly preferred embodiments of the present disclosure, in formula (I), L is C=O, and $R_3$ is -N(H)(cyclobutyl). In some particularly preferred embodiments of the present disclosure, in formula (I), L is C=O, and $R_3$ is - N(H)(tetrahydrofuranyl). In some particularly preferred embodiments of the present disclosure, in formula (I), L is C=O, and $R_3$ is -N($R_{17}$)($R_{18}$), and $R_{17}$, $R_{18}$ and the nitrogen atom to which they are attached together form a 5-membered ring, and the ring is optionally substituted by one or more substituents selected from -OH, -CN, -SH, halogen, -$NO_2$, and - $SF_5$.

In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; $R_3$ is - N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, $C_{5-11}$ bicyclic alkyl, $C_{7-11}$ bicyclic aryl, 7-11 membered bicyclic heteroaryl, 5-11 membered bicyclic heteroalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, or 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, and $R_3$ is substituted with 0, 1, 2, 3, or 4 $R_a$(s); wherein $R_{17}$, $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, and $R_3$ is substituted with 0, 1, 2, 3, or 4 $R_a$(s); wherein $R_{17}$, $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; and $R_3$ is - N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s) and $R_3$ is -N($R_{17}$)($R_{18}$), $C_{3-7}$ cycloalkyl or $C_{1-6}$ alkoxy; wherein $R_{17}$, $R_{18}$ are each independently selected from H, $C_{1-6}$ alkyl, $C_{3-7}$ cycloalkyl, and $C_{3-7}$ heterocycloalkyl, and optionally substituted with one or more of -OH, -CN, -SH, halogen, - $NO_2$, and -$SF_5$; or $R_{17}$, $R_{18}$, and the N atom to which they are attached together form a 3-10 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is methoxy, ethoxy, propoxy, cyclopropyl, cyclobutyl, cyclopentyl, cyclohexyl, pyrrolidinyl, piperidinyl, morpholinyl, - N(H)($CH_3$), -N(H)($CH_2CH_3$), -N(H)($CH_2CH_2OH$), -N(H)($CH_2CH_2CH_2OH$), - N(H)($CH_2CH(CH_3)OH$), -N(H)($CH_2CH_2CN$), -N($CH_3$)($CH_3$), -N(H)(cyclopropyl), - N(H)(cyclobutyl), -N(H)(tetrahydrofuranyl); or $R_3$ is -N($R_{17}$)($R_{18}$), and $R_{17}$, $R_{18}$, and the N atom to which they are attached together form a 5-membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, $C_{1-6}$ alkoxy, and $R_3$ is substituted with 0, 1, 2, 3, or 4 $R_a$(s); and $R_3$ is methoxy. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl,

wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is cyclopropyl. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is morpholinyl. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N(H)($CH_3$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N(H)($CH_2CH_3$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N(H)($CH_2CH_2OH$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is - N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N(H)($CH_2CH_2CN$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N(H)($CH_2CH_2CH_2OH$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is - N(H)($CH_2CH(CH_3)OH$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N($CH_3$)($CH_3$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is - N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N(H)(cyclopropyl). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N(H)(cyclobutyl). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is - N(H)(tetrahydrofuranyl). In some particularly preferred embodiments of the present disclosure, in Formula (I), L is C=O; $R_1$

is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom, preferably all H atoms on $R_1$ are replaced by D atoms; and $R_3$ is -N($R_{17}$)($R_{18}$), $C_{3-7}$ cycloalkyl, 3-7 membered heterocycloalkyl, or $C_{1-6}$ alkoxy, and $R_3$ is substituted with 0, 1, 2, 3, or 4 $R_a$(s); and $R_3$ is -N($R_{17}$)($R_{18}$), and $R_{17}$, $R_{18}$, and the N atom to which they are attached together form a 5-membered ring, and the ring is optionally substituted with one or more of -OH, -CN, -SH, halogen, -$NO_2$, and -$SF_5$.

In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms; $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, $C_{5-11}$ bicyclic alkyl, $C_{7-11}$ bicyclic aryl, 7-11 membered bicyclic heteroaryl, or 5-11 membered bicyclic heteroalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, $C_{1-6}$ haloalkoxy, wherein at least one H atom on $R_1$ is replaced by a D atom, preferably all H atoms on $R_1$ are replaced by D atoms; $R_3$ is - N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$.

In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N($R_{17}$)($R_{18}$), $C_{1-6}$ alkoxy, $C_{1-6}$ alkyl, $C_{3-7}$ cycloalkyl, or 3-7 membered heterocycloalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N($R_{17}$)($R_{18}$), $C_{3-7}$ cycloalkyl, 3-7 membered heterocycloalkyl or $C_{1-6}$ alkoxy; wherein $R_{17}$, $R_{18}$ are each independently selected from H, $C_{1-6}$ alkyl, $C_{3-7}$ cycloalkyl, and $C_{3-7}$ heterocycloalkyl, and optionally substituted with one or more of -OH, -CN, -SH, halogen, -$NO_2$, and -$SF_5$; or $R_{17}$, $R_{18}$, and the N atom to which they are attached together form a 3-10 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is methoxy, ethoxy, propoxy, cyclopropyl, cyclobutyl, cyclopentyl, cyclohexyl, pyrrolidinyl, piperidinyl, morpholinyl, -N(H)($CH_3$), -N(H)($CH_2CH_3$), -N(H)($CH_2CH_2OH$), -N(H)($CH_2CH_2CH_2OH$), - N(H)($CH_2CH(CH_3)OH$), -N(H)($CH_2CH_2CN$), -N($CH_3$)($CH_3$), -N(H)(cyclopropyl), - N(H)(cyclobutyl), -N(H)(tetrahydrofuranyl); or $R_3$ is -N($R_{17}$)($R_{18}$), and $R_{17}$, $R_{18}$, and the N atom to which they are attached together form a 5-membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is methoxy. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is ethoxy. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is cyclopropyl. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is cyclopropyl. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is morpholinyl. In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N(H)($CH_3$). In some particularly preferred embodiments of the present disclosure, in Formula (I), L is C=O; $R_1$ is -S-$C_{1-4}$ alkyl, $C_{1-6}$ alkyl, $C_{1-6}$

alkoxy, or $C_{1-6}$ haloalkoxy, wherein at least one H atom on $R_1$ is replaced by a D atom, preferably all H atoms on $R_1$ are replaced by D atoms, and $R_3$ is - N(H)($CH_2CH_3$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N(H)($CH_2CH_2OH$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N(H)($CH_2CH_2CN$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is - N(H)($CH_2CH_2CH_2OH$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N(H)($CH_2CH(CH_3)OH$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N($CH_3$)($CH_3$). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is - N(H)(cyclopropyl). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N(H)(cyclobutyl). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N(H)(tetrahydrofuranyl). In some particularly preferred embodiments of the present disclosure, in Formula (I), L is C=O; $R_1$ is -S-$C_{1-4}$ alkyl, $C_{1-6}$ alkyl, $C_{1-6}$ alkoxy, or $C_{1-6}$ haloalkoxy, wherein at least one H atom on $R_1$ is replaced by a D atom, preferably all H atoms on $R_1$ are replaced by D atoms, and $R_3$ is -N($R_{17}$)($R_{18}$), and $R_{17}$, $R_{18}$, and the N atom to which they are attached together form a 5-membered ring, and said ring is optionally substituted with one or more of -OH, -CN, -SH, halogen, -$NO_2$, and - $SF_5$.

In some particularly preferred embodiments of the present disclosure, in Formula (I), L is C=O; $R_1$ is $C_{1-6}$ alkyl, wherein at least one H atom on $R_1$ is replaced by a D atom, preferably all H atoms on $R_1$ are replaced by D atoms; $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, $C_{5-11}$ bicyclic alkyl, $C_{7-11}$ bicyclic aryl, 7-11 membered bicyclic heteroaryl, or 5-11 membered bicyclic heteroalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O; $R_1$ is $C_{1-6}$ alkyl, wherein at least one H atom on $R_1$ is replaced by a D atom, preferably all H atoms on $R_1$ are replaced by D atoms; $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N($R_{17}$)($R_{18}$), $C_{1-6}$ alkoxy, $C_{1-6}$ alkyl, $C_{3-7}$ cycloalkyl, or 3-7 membered heterocycloalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, or 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N($R_{17}$)($R_{18}$), $C_{3-7}$ cycloalkyl, 3-7 membered heterocycloalkyl, or $C_{1-6}$ alkoxy; wherein $R_{17}$ and $R_{18}$ are each independently selected from H, $C_{1-6}$ alkyl, $C_{3-7}$ cycloalkyl, and $C_{3-7}$ heterocycloalkyl, and optionally substituted with one or more of -OH, - CN, -SH, halogen, -$NO_2$, and -$SF_5$; or $R_{17}$, $R_{18}$, and the N atom to which they are attached together form a 3-10 membered ring, and the ring is optionally substituted with one or more of -OH, -CN, -SH, halogen, -$NO_2$, and -$SF_5$. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is methoxy, ethoxy, propoxy, cyclopropyl, cyclobutyl, cyclopentyl, cyclohexyl, pyrrolidinyl, piperidinyl, morpholinyl, -N(H)($CH_3$), -N(H)($CH_2CH_3$), -N(H)($CH_2CH_2OH$), - N(H)($CH_2CH_2CH_2OH$), -N(H)($CH_2CH(CH_3)OH$), -N(H)($CH_2CH_2CN$), -N($CH_3$)($CH_3$), - N(H)(cyclopropyl), -N(H)(cyclobutyl),

-N(H)(tetrahydrofuranyl); or $R_3$ is -N($R_{17}$)($R_{18}$), and $R_{17}$, $R_{18}$, and the N atom to which they are attached together form a 5-membered ring, and said ring is optionally substituted with one or more of -OH, -CN, -SH, halogen, -$NO_2$, and - $SF_5$. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is methoxy. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is ethoxy. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is cyclopropyl. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is morpholinyl. In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N(H)($CH_3$). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N(H)($CH_2CH_3$). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is - N(H)($CH_2CH_2OH$). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N(H)($CH_2CH_2CN$). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N(H)($CH_2CH_2CH_2OH$). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N(H)($CH_2CH(CH_3)OH$). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N($CH_3$)($CH_3$). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is - N(H)(cyclopropyl). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N(H)(cyclobutyl). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N(H)(tetrahydrofuranyl). In some particularly preferred embodiments of the present disclosure, in Formula (I), L is C=O, $R_1$ is $C_{1-6}$ alkyl, wherein at least one H atom on $R_1$ is replaced by a D atom, preferably all H atoms on $R_1$ are replaced by D atoms, and $R_3$ is -N($R_{17}$)($R_{18}$), and $R_{17}$, $R_{18}$, and the N atom to which they are attached together form a 5-membered ring, and said ring is optionally substituted with one or more of -OH, -CN, -SH, halogen, -$NO_2$, and -$SF_5$.

In some particularly preferred embodiments of the present disclosure, in Formula (I), L is C=O; $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$; $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, $C_{5-11}$ bicyclic alkyl, $C_{7-11}$ bicyclic aryl, 7-11 membered bicyclic heteroaryl, or 5-11 membered bicyclic heteroalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and-$SF_5$. In some particularly preferred embodiments of the present disclosure, in Formula (I), L is C=O; $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$; $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N($R_{17}$)($R_{18}$), $C_{1-6}$ alkoxy, $C_{1-6}$ alkyl, $C_{3-7}$ cycloalkyl, or 3-7 membered heterocycloalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and optionally substituted with one or more of -OH, -CN, -SH, halogen, -$NO_2$, and -$SF_5$; or $R_{17}$, $R_{18}$, and the N atom to which they are attached together form a 3-14 membered ring, and the ring is optionally substituted with one or more of -OH, -CN, -SH, halogen, -$NO_2$, and -$SF_5$. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is

-N($R_{17}$)($R_{18}$), 3-7 membered heterocycloalkyl, $C_{3-7}$ cycloalkyl or $C_{1-6}$ alkoxy; wherein $R_{17}$ and $R_{18}$ are each independently selected from H, $C_{1-6}$ alkyl, $C_{3-7}$ cycloalkyl, and $C_{3-7}$ heterocycloalkyl, and optionally substituted with one or more of -OH, -CN, -SH, halogen, -$NO_2$, and -$SF_5$; or $R_{17}$, $R_{18}$, and the N atom to which they are attached together form a 3-10 membered ring, and the ring is optionally substituted with one or more of -OH, -CN, -SH, halogen, -$NO_2$, and -$SF_5$. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is methoxy, ethoxy, propoxy, cyclopropyl, cyclobutyl, cyclopentyl, cyclohexyl, pyrrolidinyl, piperidinyl, morpholinyl, -N(H)($CH_3$), -N(H)($CH_2CH_3$), -N(H)($CH_2CH_2OH$), -N(H)($CH_2CH_2CH_2OH$), - N(H)($CH_2CH(CH_3)OH$), -N(H)($CH_2CH_2CN$), -N($CH_3$)($CH_3$), -N(H)(cyclopropyl), - N(H)(cyclobutyl), -N(H)(tetrahydrofuranyl); or $R_3$ is -N($R_{17}$)($R_{18}$), and $R_{17}$, $R_{18}$, and the N atom to which they are attached together form a 5-membered ring, and the ring is optionally substituted with one or more of -OH, -CN, -SH, halogen, -$NO_2$, -$SF_5$. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is methoxy. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is ethoxy. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is cyclopropyl. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is morpholinyl. In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)($CH_3$). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)($CH_2CH_3$). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)($CH_2CH_2OH$). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)($CH_2CH_2CN$). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)($CH_2CH_2CH_2OH$). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)($CH_2CH(CH_3)OH$). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N($CH_3$)($CH_3$). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)(cyclopropyl). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N(H)(cyclobutyl). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is - N(H)(tetrahydrofuranyl). In some particularly preferred embodiments of the present disclosure, in Formula (I), L is C=O, $R_1$ is $C_{1-6}$ alkyl, wherein all H atoms on $R_1$ are replaced by D atoms, for example $R_1$ is $CD_3$, $C_2D_6$, $C_3D_8$ or $C_4D_{10}$, and $R_3$ is -N($R_{17}$)($R_{18}$), and $R_{17}$, $R_{18}$, and the N atom to which they are attached together form a 5-membered ring, and the ring is optionally substituted with one or more of -OH, -CN, -SH, halogen, -$NO_2$, and -$SF_5$.

In some particularly preferred embodiments of the present disclosure, in Formula (I), one, two or three $R_2$(s) are present and $R_2$ is selected from H, halogen, -OH, -$NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the -S-$C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, -$NH_2$, -NH($CH_3$), -N($CH_3$)$_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in Formula (I), one, two or three $R_2$(s) are present and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, -$NH_2$, -NH($CH_3$), -N($CH_3$)$_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in Formula (I), one, two or three $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, -$NH_2$, -NH($CH_3$), -N($CH_3$)$_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in Formula (I), one, or two $R_2$(s) are present and $R_2$ is selected from halogen, and $C_{1-6}$ alkyl. In some particularly preferred embodiments of the present disclosure, in formula (I), one or two $R_2$(s) are present and $R_2$ is selected from fluorine, chlorine, bromine, methyl, ethyl, n-propyl, isopropyl, n-butyl, and isobutyl. In some preferred embodiments of the present disclosure, in Formula (I), one or two $R_2$(s) are present, and $R_2$ is selected from fluorine, chlorine, methyl, ethyl, n-propyl, and isopropyl. In some preferred embodiments of the present disclosure, in Formula (I), one or two $R_2$(s) are present, and each $R_2$ is selected from fluorine, methyl, and ethyl. In some preferred embodiments of the present disclosure, in Formula (I), one or two $R_2$ (s) are present, and each $R_2$ is selected from fluorine and ethyl. In some preferred embodiments of the present disclosure, in formula (I), one $R_2$ is present, and $R_2$ is selected from fluorine and ethyl. In some preferred embodiments of the present disclosure, in Formula (I), two $R_2$(s) are present, and $R_2$ is selected from fluorine and ethyl. In some particularly preferred embodiments of the present disclosure, in Formula (I), two $R_2$(s) are present which are respectively fluorine and ethyl. In some particularly preferred embodiments of the present disclosure, in formula (I), one $R_2$ is present, and $R_2$ is an ethyl group. In some particularly preferred embodiments of the present disclosure, in Formula (I), one $R_2$ is present, and $R_2$ is fluoro.

In some preferred embodiments of the present disclosure, in Formula (I), $R_3$ is substituted with 0, 1, 2, 3 or 4 $R_a$(s), and each $R_a$ is independently selected from H, halogen, -OH, -$NO_2$,- CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3 -10 membered heterocycloalkyl, $C_{5-7}$ aryl, 5-7 membered heteroaryl, $C_{7-11}$ bicyclic aryl, and 7-11 membered bicyclic heteroaryl, wherein the -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3 -10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_b$(s). In some preferred embodiments of the present disclosure, in Formula (I), $R_3$ is substituted with 0, 1, 2, 3, or 4 $R_a$(s), and each $R_a$ is independently selected from H, halogen, -OH, -$NO_2$, -CN, -$SF_5$, -SH, -S-$C_{1-4}$ alkyl, $C_{1-8}$ alkyl, $C_{3-7}$ cycloalkyl, 3-7 membered heterocycloalkyl, $C_{5-7}$ aryl, and 5-7 membered heteroaryl, wherein the -S-$C_{1-4}$ alkyl, $C_{1-8}$ alkyl, $C_{3-7}$ cycloalkyl, 3-7 membered heterocycloalkyl, $C_{5-7}$ aryl, and 5-7 membered heteroaryl are optionally substituted with 1, 2, 3, or 4 $R_b$(s). In some preferred embodiments of the present disclosure, in Formula (I), $R_3$ is substituted with 0, 1, 2, 3, or 4 $R_a$(s), and each $R_a$ is independently selected from halogen, -OH, -CN, $C_{1-8}$ alkyl, $C_{3-7}$ cycloalkyl, 3-7 membered heterocycloalkyl, $C_{5-7}$ aryl, and 5-7 membered heteroaryl, wherein the $C_{1-8}$ alkyl, $C_{3-7}$ cycloalkyl, 3-7 membered heterocycloalkyl, $C_{5-7}$ aryl, and 5-7 membered heteroaryl are optionally substituted with 1, 2, 3, or 4 $R_b$(s). In some preferred embodiments of the present disclosure, in Formula (I), $R_3$ is substituted with 0, 1, 2, 3, or 4 $R_a$(s), and each $R_a$ is independently selected from halogen, -OH, -CN, $C_{1-8}$ alkyl, $C_{3-7}$ cycloalkyl, and 3-7 membered heterocycloalkyl, wherein the $C_{1-8}$ alkyl, $C_{3-7}$ cycloalkyl, and 3-7 membered heterocycloalkyl are optionally substituted with 1, 2, or 3 $R_b$(s). In some preferred embodiments of the present disclosure, in Formula (I), $R_3$ is substituted with 0 or 1 $R_a$, and each $R_a$ is independently selected from halogen, -OH, -CN, $C_{1-6}$ alkyl, $C_{3-7}$ cycloalkyl, and 5-7 membered heterocycloalkyl, wherein the $C_{1-6}$ alkyl, $C_{3-7}$ cycloalkyl, 5-7 membered heterocycloalkyl are optionally substituted with 1, 2, or 3 $R_b$(s). In some preferred embodiments of the present disclosure, in Formula (I), $R_3$ is substituted with 0 or 1 $R_a$, and each $R_a$ is independently selected from halogen, -OH, -CN, $C_{1-4}$ alkyl, $C_{3-6}$ cycloalkyl, and 5-7 membered heterocycloalkyl, wherein the $C_{1-4}$ alkyl, $C_{3-6}$ cycloalkyl, and 5-7 membered heterocycloalkyl are optionally substituted with 1, 2, or 3 $R_b$(s). In some particularly preferred embodiments of the present disclosure, in Formula (I), $R_3$ is substituted with 0 or 1 $R_a$, and each $R_a$ is independently selected from methyl, ethyl, hydroxyl, -CN, piperidinyl, morpholinyl, piperazinyl, and cyclopropyl, wherein piperidinyl, morpholinyl, and piperazinyl are optionally substituted with 1, 2, 3, or 4 $C_{1-3}$ alkyl(s). In some particularly preferred embodiments of the present disclosure, in Formula (I), $R_3$ is substituted with 0 or 1 $R_a$, and each $R_a$ is independently selected from methyl, ethyl, hydroxyl, -CN, piperidinyl, morpholinyl, 1-methylpiperazinyl, and cyclopropyl. In some particularly preferred embodiments of the present disclosure, in Formula (I), $R_a$ is absent. In some particularly preferred embodiments of the present disclosure, in Formula (I), $R_a$ is 1-methylpiperazinyl. In some particularly preferred embodiments of the present disclosure, in Formula (I), $R_a$ is methyl. In some particularly preferred embodiments of the present disclosure, in Formula (I), $R_a$ is ethyl. In some particularly preferred embodiments of the present disclosure, in Formula (I), $R_a$ is piperidinyl. In some particularly preferred embodiments of the present disclosure, in Formula (I), $R_a$ is morpholinyl. In some particularly preferred embodiments of the present disclosure, in Formula (I), $R_a$ is hydroxyl. In some particularly preferred embodiments of the present disclosure, in Formula (I), $R_a$ is -CN. In some particularly preferred embodiments of the present disclosure, in Formula (I), $R_a$ is cyclopropyl.

The preferred options of the respective substituents mentioned in the above various preferred embodiments can be combined with each other in any way, and various combinations thereof are within the scope of the present disclosure. In the most preferred embodiments of the present disclosure, the compound of formula (I) is each specific compound shown in Examples herein. That is, the compound of formula (I) is selected from

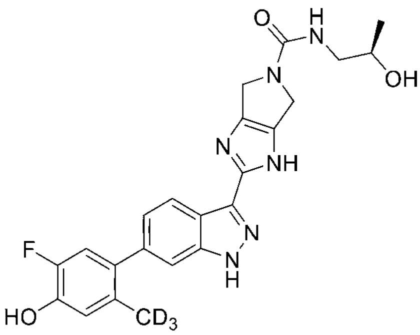

1 ,

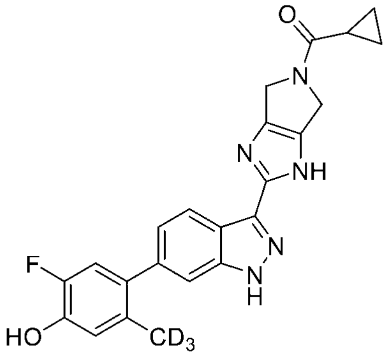

2 ,

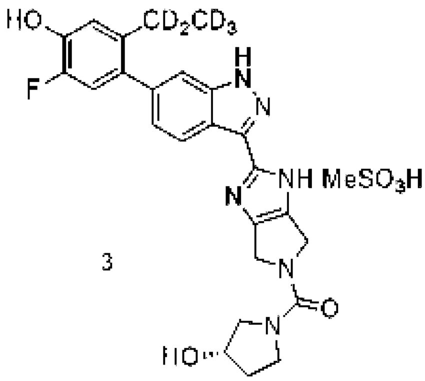

3 ,

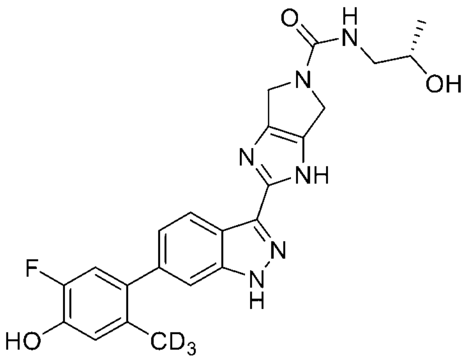

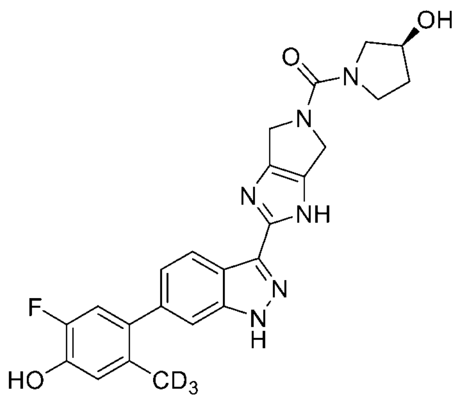

,

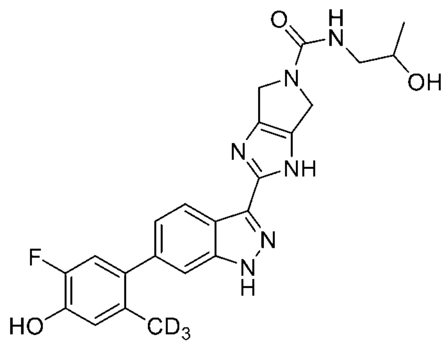

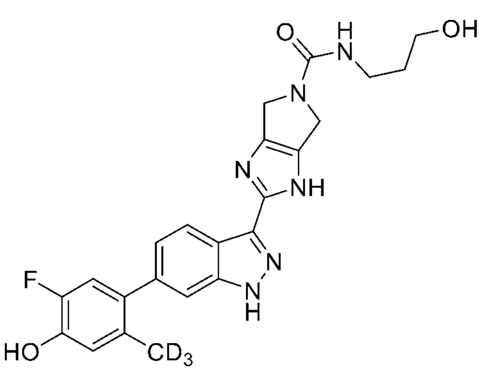

,

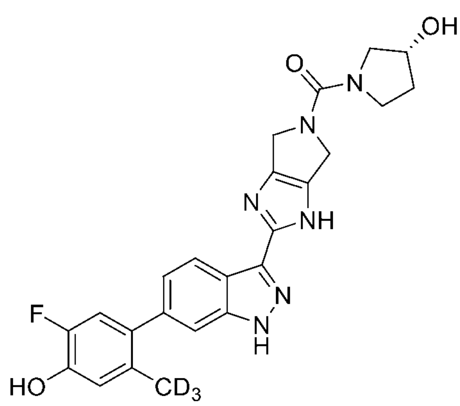

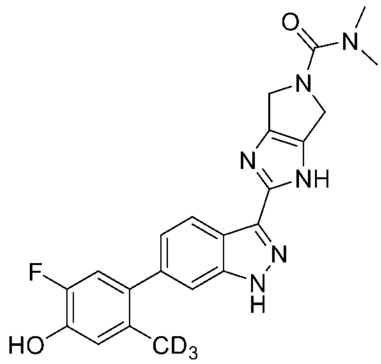, 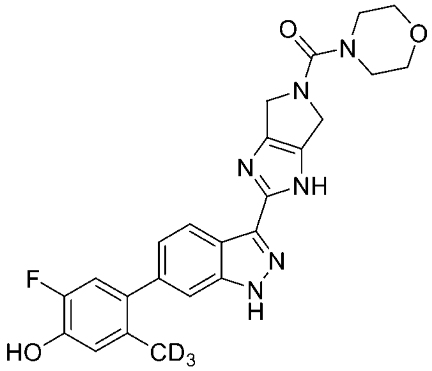,

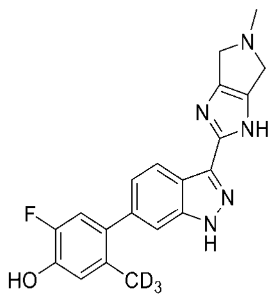
12, 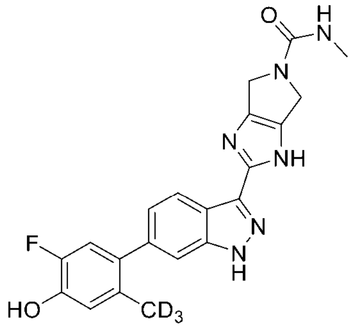, 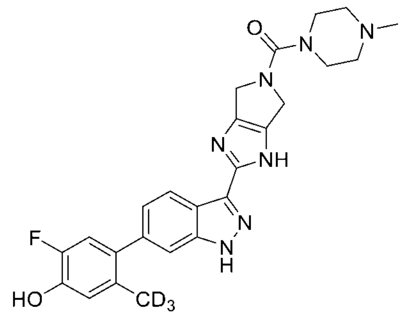,

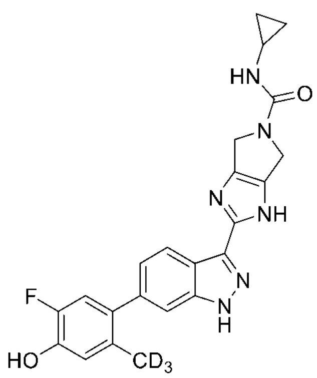, 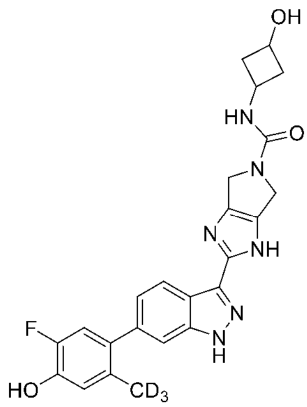, 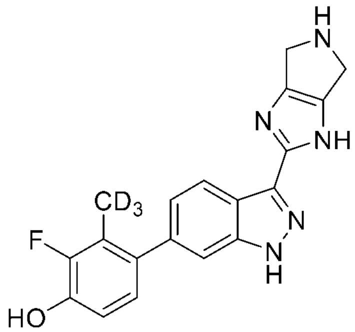,

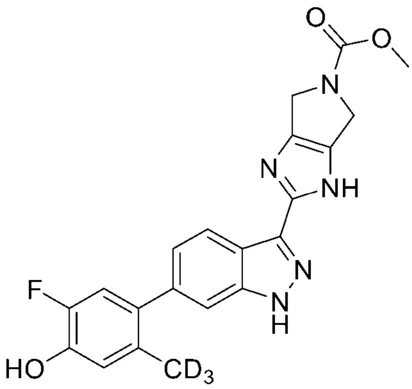, 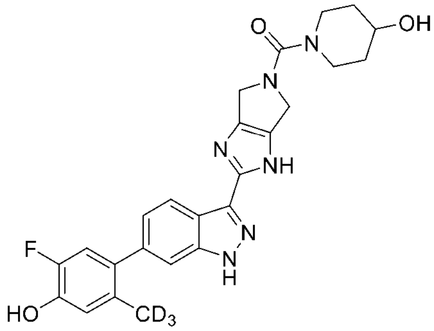,

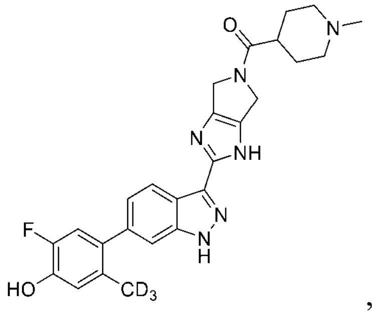, 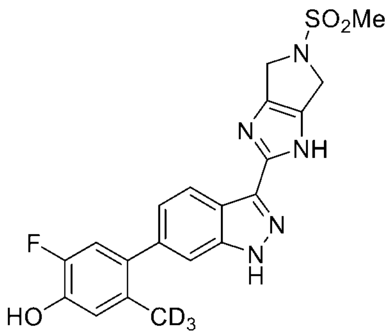, 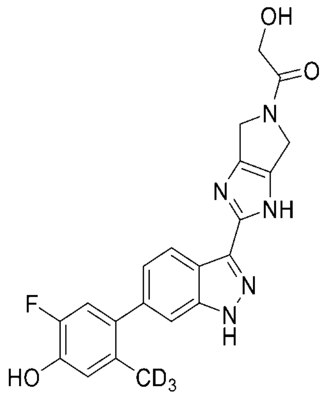,

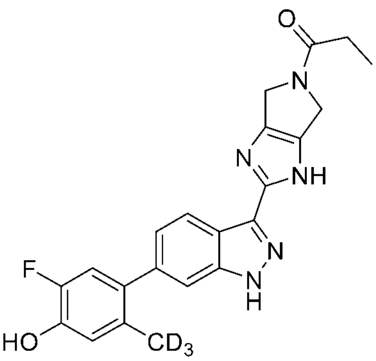, 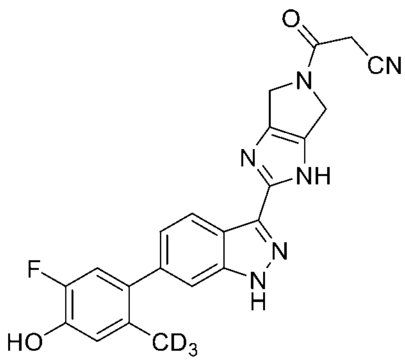,

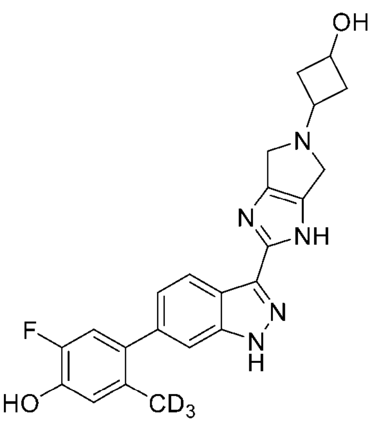, 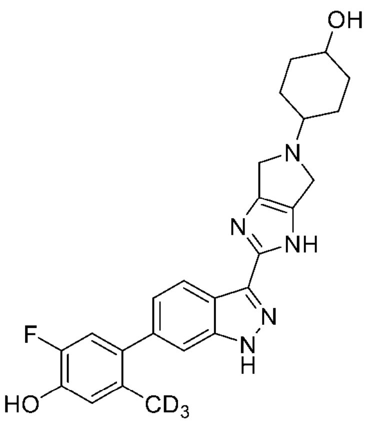, 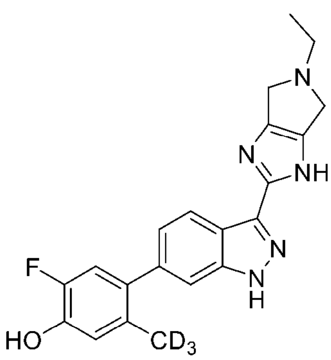,

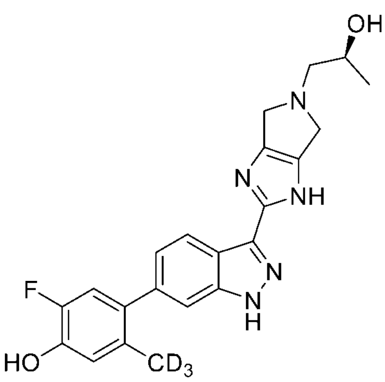, 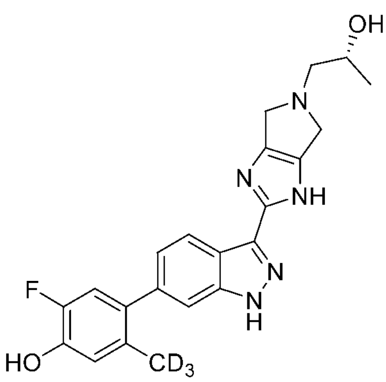

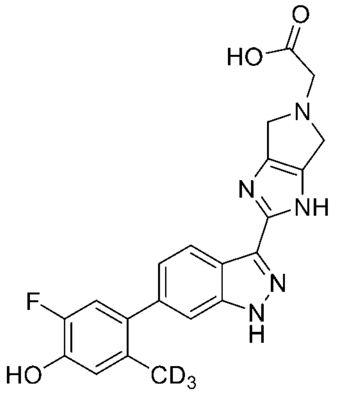

,

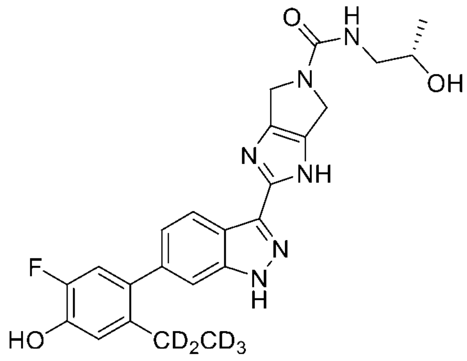

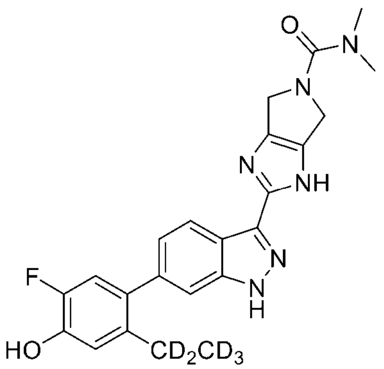

,

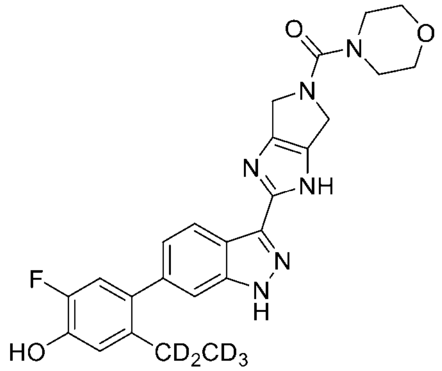

,

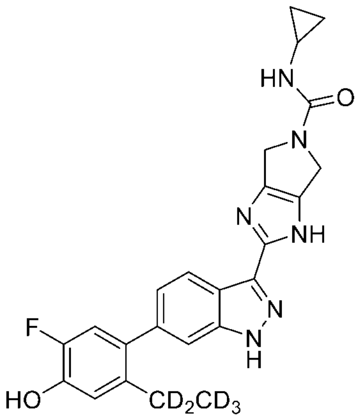

,

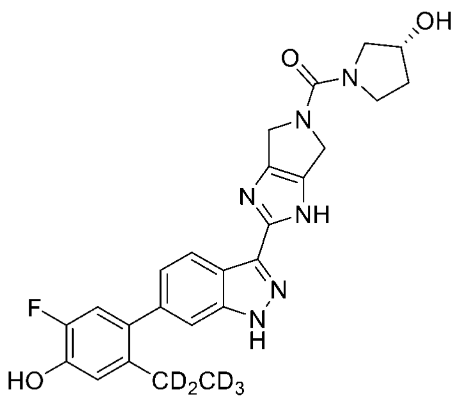

and

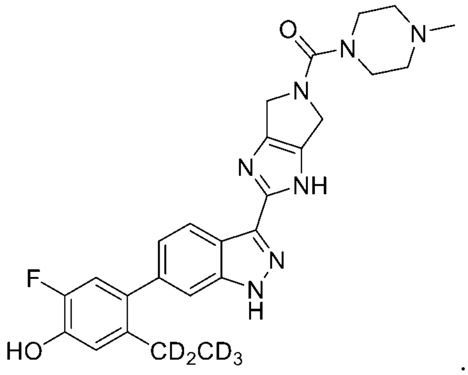

.

In addition to the embodiments of the present invention in which $R_1$ in Formula (I) is an isotopically labeled substituent, the above-described embodiments of the present invention also encompass embodiments in which other substituents in Formula (I) besides $R_1$, including but not limited to $R_2$ and/or $R_3$, are either isotopically labeled substituents or non-isotopically labeled substituents.

For simplicity, hereinafter, the term "a compound as shown by Formula (I)" or "a compound of Formula (I)" or "a compound of the disclosure" or "a compound according to the disclosure" also encompasses any optical isomer, geometric isomer, tautomer or a mixture of various isomers of the compound of Formula (I).

The term "optical isomer" refers that when a compound has one or more chiral centers, each chiral center may have an R configuration or an S configuration, and the various isomers thus constituted are known as an optical isomer. Optical isomers comprise all diastereomers, enantiomers, meso forms, racemates or mixtures thereof. For example, optical isomers can be separated by a chiral chromatography or by chiral synthesis.

The term "geometric isomer" refers that when a double bond is present in a compound, the compound may exist as a cis isomer, a trans isomer, an E isomer, or a Z isomer. A geometric isomer comprises a cis isomer, trans isomer, E isomer, Z isomer, or a mixture thereof.

The term "tautomer" refers to an isomer that is formed by rapid movement of an atom at two positions in a single molecule. It will be understood by those skilled in the art that tautomers can be mutually transformed, and in a certain state, may coexist by reaching an equilibrium state. As used herein, the term "a compound as shown by Formula (I)" also encompasses any tautomer of the compound of Formula (I).

Unless otherwise indicated, reference to "a compound as shown by Formula (I)" or "a compound of Formula (I)" or "a compound of the disclosure" or "a compound according to the disclosure" herein also encompasses isotopically-labeled compounds obtained by replacing any atom of the compound with its isotopic atom.

The disclosure comprises all pharmaceutically acceptable isotopically-labeled compounds of Formula (I) wherein one or more atoms are replaced by atoms having the same atomic number but different atomic mass or mass number than those normally found in nature.

Examples of isotopes suitable for inclusion in the compounds of the disclosure include isotopes of hydrogen, such as $^{2}H$ (D) and $^{3}H$ (T), of carbon, such as $^{11}C$, $^{13}C$ and $^{14}C$, of chlorine, such as $^{36}Cl$, of fluorine, such as $^{18}F$, of iodine, such as $^{123}I$ and $^{125}I$, of nitrogen, such as $^{13}N$ and $^{15}N$, of oxygen, such as $^{15}O$, $^{17}O$ and $^{18}O$, and of sulphur, such as $^{35}S$.

Certain isotopically-labelled compounds of formula (I), for example, those incorporating a radioactive isotope, are useful in drug and/or substrate tissue distribution studies. The radioactive isotopes deuterium, i.e. $^{2}H$, also abbreciated as D and carbon-14, i.e. $^{14}C$, are particularly useful for this purpose in view of their ease of incorporation and ready means of detection.

Substitution with heavier isotopes such as deuterium, i.e. $^{2}H$, also abbreciated as D may afford certain therapeutic advantages resulting from greater metabolic stability, for example, increased in vivo half-life or reduced dosage requirements, and hence is preferred.

Substitution with positron emitting isotopes, such as $^{11}C$, $^{18}F$, $^{15}O$ and $^{13}N$, can be useful in Positron Emission Topography (PET) studies for examining substrate receptor occupancy.

Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

The compound of formula (I) may exist in the form of a pharmaceutically acceptable salt, for example, an acid addition salt and/or a base addition salt of the compound of formula (I). Unless otherwise indicated, "a pharmaceutically acceptable salt" as used herein includes acid addition salts or base addition salts that may appear in the compound of formula (I).

The pharmaceutically acceptable salt of the compound of formula (I) include acid addition salts and base addition salts thereof. Suitable acid addition salts are formed from acids that form non-toxic salts. Examples include but are not limited to: acetate, adipate, aspartate, benzoate, benzenesulfonate, bicarbonate/carbonate, bisulfate/sulfate, borate, camphor sulfonate, citrate, cyclohexamine sulfonate, ethanedisulfonate, formate, fumarate, glucoheptonate, gluconate, glucuronate, hexafluorophosphate, 2-(4-hydroxybenzyl) benzoate, hydrochloride/chloride, hydrobromide/bromide, hydroiodide/iodide, 2-isethionate, lactate, malate, maleate, malonate, methanesulfonate, methyl sulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate and trifluoroacetate. Suitable base addition salts are formed from bases that form non-toxic salts. Examples thereof include, but are not limited to: aluminum, arginine, calcium, choline, diethylamine, diethanolamine, glycine, lysine, magnesium, meglumine, ethanolamine, potassium, sodium, tromethamine, and zinc salts. It is also possible to form half salts of acids and bases, such as hemisulfate and hemicalcium salts. For a review of suitable salts, please refer to Handbook of Pharmaceutical Salts: Properties, Selection and Use by Stahl and Wermuth (Wiley-VCH, 2002). Methods for preparing pharmaceutically acceptable salts of the compounds described herein are known to those skilled in the art.

Certain compounds of the disclosure may exist in unsolvated form as well as solvated forms, including hydrated

forms. In general, the compounds of formula (I), whether present in solvated form or in unsolvated form, are included within the scope of the disclosure.

Certain compounds of the disclosure may exist in different crystalline or amorphous forms, and the compounds of formula (I) present in any forms, are included within the scope of the disclosure.

To avoid ambiguity, the definitions of some terms used herein are given below. Unless otherwise stated, the meanings of the terms used herein are as follows.

The term "pharmaceutically acceptable" means that the corresponding compound, carrier or molecule is suitable for administration to humans. Preferably, the term refers to it is approved by regulatory agencies such as CFDA (China), EMEA (Europe), FDA (United States), and other national regulatory agencies to be suitable for mammals, preferably humans.

The "prodrug" refers to a derivative that is converted into a compound of the present disclosure by a reaction with enzymes, gastric acid, and the like in the living body under physiological conditions, for example, through oxidation, reduction, hydrolysis, and the like catalyzed by enzymes.

The "metabolite" refers to all molecules derived from any compound of the present disclosure in a cell or organism, preferably a human.

The term "hydroxy" refers to -OH.

The term "halogen" or "halo" refers to -F, -Cl, -Br, or -I.

The term "cyano" refers to -CN.

In the present disclosure, when there are multiple substituents of a certain type, each substituent is independently selected from each other, and these substituents may be the same or different. For example, when there are 2, 3, or 4 $R_1$s, these $R_1$s may be the same or different. For example, when there are 2, 3, or 4 $R_2$s, these $R_2$s may be the same or different. For example, when $R_1$ and $R_2$ are both -N($R_9$)($R_{10}$), $R_9$ and $R_{10}$ contained in $R_1$ and $R_2$ can be independently selected, that is, $R_9$ in $R_1$ and $R_9$ in $R_2$ can be the same or different, and $R_{10}$ in $R_1$ and $R_{10}$ in $R_2$ may be the same or different. For example, when there are two $R_1$s, and the two $R_1$s are both -N($R_9$)($R_{10}$), $R_9$ and $R_{10}$ in the two $R_1$s can be selected independently, that is, $R_9$ in the first $R_1$ and $R_9$ in the second $R_1$ may be the same or different, and $R_{10}$ in the first $R_1$ and $R_{10}$ in the second $R_1$ may be the same or different. The above statement applies to $R_1$, $R_2$, $R_3$, $R_4$, $R_5$, $R_6$, $R_7$, $R_8$, $R_9$, $R_{10}$, $R_{11}$, $R_{12}$, $R_3$, $R_{14}$, $R_{15}$, $R_{16}$, $R_{17}$, $R_{18}$.

As used herein, the term "substituted" means that one or more (preferably 1 to 5, more preferably 1 to 3) hydrogen atoms in a group are independently replaced by a corresponding number of substituents.

As used herein, the term "independently" means that when the number of substituents is more than one, these substituents may be the same or different.

As used herein, the term "optional" or "optionally" means that the event described therein may or may not occur. For example, an "optionally substituted" group means that the group may be unsubstituted or substituted.

As used herein, the term "heteroatom" as used herein refers to oxygen (O), nitrogen (N), or $S(O)_m$in which m may be 0, 1 or 2, i.e. a sulfur atom S, or a sulfoxide group SO, or a sulfonyl group $S(O)_2$).

As used herein, the term "alkyl" refers to saturated aliphatic hydrocarbons, including straight and branched chains. In some embodiments, the alkyl group has 1-8, or 1-6, or 1-3 carbon atoms. For example, the term "$C_{1-8}$ alkyl" refers to a straight or branched chain group of atoms having 1-8 carbon atoms. The term "$C_{1-8}$ alkyl" includes the terms "$C_{1-6}$ alkyl", "$C_1$-$C_3$ alkyl" and "$C_1$-$C_4$ alkyl" in its definition. Examples of alkyl groups include, but are not limited to, methyl, ethyl, propyl, isopropyl, butyl, isobutyl, sec-butyl, tert-butyl, pentyl, 2-pentyl, 3-pentyl, isopentyl, neopentyl, (R)-2-methylbutyl, (S)-2-methylbutyl, 3-methylbutyl, 2,3-dimethylpropyl, 2,3-dimethylbutyl, hexyl, and the like. The alkyl group may be optionally substituted with one or more (for example, 1 to 5) suitable substituent(s).

As used herein, the term "alkenyl" refers to an aliphatic hydrocarbon having at least one carbon-carbon double bond, including straight and branched chains having at least one carbon-carbon double bond. In some embodiments, alkenyl groups have 2-8 carbon atoms, 2-6 carbon atoms, 3-6 carbon atoms, or 2-4 carbon atoms. For example, the term "$C_{2-8}$ alkenyl" refers to a linear or branched unsaturated atomic group (having at least one carbon-carbon double bond) having 2-8 carbon atoms. The double bond may or may not be the point of attachment of another group. Alkenyl groups include, but are not limited to, vinyl, 1-propenyl, 2-propenyl, 2-methyl-2-propenyl, butenyl, pentenyl, 3-hexenyl, and the like. Alkenyl groups may be optionally substituted with one or more (for example, 1 to 5) suitable substituent(s). When the compound of formula (I) contains an alkenyl group, the alkenyl group may be present in the pure E form, the pure Z form, or any mixture thereof.

As used herein, the term "alkynyl" refers to an aliphatic hydrocarbon having at least one carbon-carbon triple bond, including straight and branched chains having at least one carbon-carbon triple bond. In some embodiments, an alkynyl group has 2-8 carbon atoms, 2-6 carbon atoms, 3-6 carbon atoms, or 2-4 carbon atoms. For example, the term "$C_{2-8}$ alkynyl" refers to a linear or branched unsaturated atomic group (having at least one carbon-carbon triple bond) having 2-8 carbon atoms. The triple bond may or may not be the point of attachment of another group. Alkynyl groups include, but are not limited to, ethynyl, 1-propynyl, 2-propynyl, 2-methyl-2-propynyl, butynyl, pentynyl, 3-hexynyl, and the like. The alkynyl group may be optionally substituted with one or more (for example, 1 to 5) suitable substituent(s).

As used herein, the term "$C_{3-7}$ cycloalkyl" refers to a cycloalkyl group having 3-7 carbon atoms forming a ring, such as cyclopropyl, cyclobutyl, cyclopentyl, cyclohexyl, cyclohexenyl, cycloheptyl. The cycloalkyl may be optionally substituted with one or more suitable substituent(s).

As used herein, the term "n-membered heterocycloalkyl" refers to a cycloalkyl group having m ring-forming carbon atoms and (n-m) ring-forming heteroatoms, the heteroatoms being selected from O, S and N. For example, 3-7 membered heterocycloalkyl includes, but not limited to, oxetane, thietane, azetidine, tetrahydrofuran, tetrahydrothiophene, pyrrolidine, tetrahydropyran, tetrahydrothiopyran, piperidine, morpholine, piperazine, oxepane, thiepane, and azepine. The heterocycloalkyl may be optionally substituted with one or more suitable substituent(s).

As used herein, the term "$C_{5-7}$ aryl" refers to an aryl group having an aromatic ring containing 5-7 carbon atoms, preferably phenyl.

As used herein, the term "n-membered heteroaryl" refers to a heteroaryl group having m carbon atoms forming an aromatic ring and (n-m) heteroatoms forming an aromatic ring, the heteroatoms being selected from O, S and N. For example, 5-7 membered heteroaryl includes but not limited to pyrazine, pyrazole, pyrrole, furan, thiophene, thiazole, and pyridine. The heteroaryl may be optionally substituted with one or more suitable substituent(s).

As used herein, the term "$C_{7-11}$ bicyclic aryl" refers to a bicyclic aryl group having 7-11 carbon atoms, such as naphthalene, indene and the like. The bicyclic aryl may be optionally substituted with one or more suitable substituent(s).

As used herein, the term "n-membered bicyclic heteroaryl" refers to a bicyclic heteroaryl group having m carbon atoms forming an aromatic bicyclic ring and (n-m) heteroatoms forming an aromatic bicyclic ring, and the heteroatoms are selected from O, S and N. For example, 7-11 membered bicyclic heteroaryl includes, but not limited to, quinoline, isoquinoline, benzothiazole, and the like. The bicyclic heteroaryl may be optionally substituted with one or more suitable substituent(s).

As used herein, the term "11-15 membered tricyclyl" includes but not limited to acridine and the like. The 11-15 membered tricyclyl may be optionally substituted with one or more suitable substituent(s).

As used herein, the term "haloalkyl" refers to an alkyl group having one or more halogen substituent(s) (up to perhaloalkyl, that is, each hydrogen atom of the alkyl is replaced by a halogen atom). For example, the term "$C_{1-6}$ haloalkyl" refers to a $C_{1-6}$ alkyl group with one or more halogen substituent(s) (up to perhaloalkyl, that is, each hydrogen atom of the alkyl group is replaced by a halogen atom). As another example, the term "$C_{1-4}$ haloalkyl" refers to a $C_{1-4}$ alkyl group with one or more halogen substituent(s) (up to perhaloalkyl, that is, each hydrogen atom of the alkyl group is replaced by a halogen atom); the term "$C_{1-3}$ haloalkyl" refers to a $C_{1-3}$ alkyl group with one or more halogen substituent(s) (up to perhaloalkyl, that is, each hydrogen atom of the alkyl group is replaced by a halogen atom); and the term "$C_{1-2}$ haloalkyl" refers to a $C_{1-2}$ alkyl group (i.e. methyl or ethyl) with one or more halogen substituent(s) (up to perhaloalkyl, that is, each hydrogen atom of the alkyl group is replaced by a halogen atom). As another example, the term "$C_1$ haloalkyl" refers to a methyl group with 1, 2, or 3 halogen substituent(s). Examples of haloalkyl groups include: $CF_3$, $C_2F_5$, $CHF_2$, $CH_2F$, $CH_2CF_3$, $CH_2Cl$, and the like.

As used herein, the term "alkoxy" refers to alkyl with a single bond attached to an oxygen atom. The point of attachment of the alkoxy group to a molecule is through the oxygen atom. Alkoxy can be described as alkyl-O-. The term "$C_{1-6}$ alkoxy" refers to a linear or branched alkoxy group containing 1 to 6 carbon atoms. The term "$C_{1-6}$ alkoxy" includes the term "$C_{1-3}$ alkoxy" in its definition. Alkoxy includes, but not limited to, methoxy, ethoxy, propoxy, isopropoxy, butoxy, hexoxy, and the like. The alkoxy group may be optionally substituted with one or more suitable substituent(s).

Herein, a numerical range relating to the number of substituents, the number of carbon atoms, or the number of ring members represents an enumeration of all integers in the range, and the range is only a simplified representation thereof. For example:

"1-4 substituent(s)" means 1, 2, 3 or 4 substituent(s);

"1-3 substituent(s)" means a 1, 2 or 3 substituent(s);

"3 to 12-membered ring" means a 3, 4, 5, 6, 7, 8, 9, 10, 11 or 12-membered ring;

"3 to 14-membered ring" means a 3, 4, 5, 6, 7, 8, 9, 10, 11, 12, 13 or 14-membered ring;

"3 to 8 membered ring" means a 3, 4, 5, 6, 7, or 8 membered ring;

"1-12 carbon atoms" or "$C_{1-12}$" means 1 ($C_1$), 2 ($C_2$), 3 ($C_3$), 4 ($C_4$), 5 ($C_5$), 6 ($C_6$), 7 ($C_7$), 8 ($C_8$), 9 ($C_9$), 10 ($C_{10}$), 11 ($C_{11}$) or 12 ($C_{12}$) carbon atoms;

"1-6 carbon atoms" or "$C_{1-6}$" means 1 ($C_1$), 2 ($C_2$), 3 ($C_3$), 4 ($C_4$), 5 ($C_5$) or 6 ($C_6$) carbon atoms;

"1-4 carbon atoms" or "$C_{1-4}$" means 1 ($C_1$), 2 ($C_2$), 3 ($C_3$), 4 ($C_4$) carbon atoms;

"2-6 carbon atoms" or "$C_{2-6}$" means 2 ($C_2$), 3 ($C_3$), 4 ($C_4$), 5 ($C_5$) or 6 ($C_6$) carbon atoms;

"$C_{3-8}$" means 3 ($C_3$), 4 ($C_4$), 5 ($C_5$), 6 ($C_6$), 7 ($C_7$), 8 ($C_8$) carbon atoms; and

"3 to 8 ring members" means 3, 4, 5, 6, 7, or 8 ring members.

Thus, a numerical range associated with the number of substituents, the number of carbon atoms, or the number of ring members also encompasses any one of its subranges, and each subrange is also considered to be disclosed herein.

In a second aspect, the present disclosure provides a pharmaceutical composition comprising the above mentioned compounds, or an optical isomer thereof, a geometric isomer thereof, a tautomer thereof or a mixture of various isomers, or a pharmaceutically acceptable salt thereof, or a prodrug thereof, or a metabolite thereof, and one or more pharmaceutically acceptable carriers, adjuvants or excipients.

The pharmaceutical compositions of the disclosure may be formulated as suitable dosage forms for oral, external (including not limited to external application, spraying, and the like), parenteral (including subcutaneous, intramuscular, intradermal and intravenous), bronchial or nasal administration as desire. Preferably, the pharmaceutical compositions of the disclosure may be formulated as suitable dosage forms for oral or external administration. More preferably, the pharmaceutical compositions of the disclosure may be formulated as suitable dosage forms for oral administration.

If a solid carrier is used, the preparation may be tableted, placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the preparation may be in the form of a syrup, emulsion, paste, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. The pharmaceutical compositions are prepared by conventional techniques appropriate to the desired preparation containing appropriate amounts of the active ingredient, that is, the compound of Formula (I) according to the present disclosure.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil), and injectable organic esters such as ethyl oleate.

These compositions may also contain excipients such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules.In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed

manner. Examples of embedding compositions which can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylatedisostearyl alcohols, polyoxyethylene sorbitol and sorbitan esters, microcrystalline cellulose, aluminum metahydroxide, bentonite, agar-agar and tragacanth, or mixtures of these substances, and the like.

Dosage forms for topical administration of a compound of the disclosure include paste, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

The external dosage form of the compound of the present disclosure may be in the form of a water-in-oil (W/O) or oil-in-water (O/W) emulsion, a multi-emulsion form, such as a water-in-oil-in-water (W/O/W) form or an oil-in-water-oil (O/W/O) emulsion, or in the form of water dispersion or lipid dispersion, gel or aerosol.

The external dosage form of the compound of the present disclosure may contain additives and aids, such as emulsifiers, thickeners, gelling agents, water fixatives, spreading agents, stabilizers, dyes, fragrances, and preservatives. Suitable emulsifiers include stearic acid, triethanolamine and PEG-40-stearate. Suitable thickeners include glyceryl monostearate and PEG600. Suitable preservatives include propyl paraben and chlorocresol. Suitable spreading agents include dimethicone and polydimethylcyclosiloxane. Suitable water fixatives include polyethylene glycol, preferably polyethylene glycol 600.

The external dosage form of the compound of the present disclosure may include pastes, lotions, gels, emulsions, microemulsions, sprays, skin patches, and the like, which can be applied topically to treat atopic dermatitis, eczema, psoriasis, and scleroderma, itching, vitiligo, hair loss and other skin diseases. In particular, the external dosage form of the compound of the present disclosure is pastes, which can be applied topically to treat skin diseases such as atopic dermatitis, eczema, psoriasis, scleroderma, itching, vitiligo, and hair loss and other skin diseases.

The amount of the compound of formula (I) in the pharmaceutical composition and dosage form can be appropriately determined by those skilled in the art as needed. For example, the compound of formula (I) can be present in the pharmaceutical composition or dosage form in a therapeutically effective amount.

In a third aspect, the present disclosure provides use of the compound as described above, or an optical isomer thereof, a geometric isomer thereof, a tautomer thereof or a mixture of various isomers, or a pharmaceutically acceptable salt thereof, or a prodrug thereof, or a metabolite thereof, or the composition as described above in the preparation of a medicament for the treatment and/or prevention of JAK-related diseases or disorders.

"Diseases or disorders related to JAK" include but not limited to:

Arthritis, including rheumatoid arthritis, juvenile arthritis and psoriatic arthritis;

Autoimmune diseases or disorders, including single organ or single cell type autoimmune disorders, such as Hashimoto’s thyroiditis, autoimmune hemolytic anemia, pernicious anemia of autoimmune atrophic gastritis, autoimmune encephalomyelitis, autoimmune orchitis, Goodpasture’s disease, autoimmune thrombocytopenia, sympathetic ophthalmia, myasthenia gravis, Graves’ disease, primary biliary cirrhosis, chronic aggressive hepatitis, ulcerative colitis and membranous glomerulopathy, those involving systemic autoimmune disorders (e.g. systemic lupus erythematosus, rheumatoid arthritis, Sjogren’s syndrome, Reiter’s syndrome, polymyositis-dermatomyositis, systemic sclerosis, polyarteritis nodosa, multiple sclerosis and bullous pemphigoid) and other O-cell (humoral) or T-cell autoimmune diseases (including Kogan syndrome), ankylosing spondylitis, Wegener’s Granuloma, autoimmune alopecia, type I diabetes or juvenile-onset diabetes or thyroiditis;

Cancer or tumor, including digestive/gastrointestinal cancer, colorectal cancer, liver cancer, skin cancer (including mast cell tumor and squamous cell carcinoma), breast cancer, ovarian cancer, prostate cancer, lymphoma, leukemia (including acute myeloid leukemia and chronic myeloid leukemia), kidney cancer, lung cancer, muscle cancer, bone cancer, bladder cancer, brain cancer, melanoma (including oral and metastatic melanoma), Kaposi’s sarcoma, myeloma (including multiple myeloma), myeloproliferative disorders, proliferative diabetic retinopathy or disorders

related to angiogenesis (including solid tumors);

Diabetes, including type I diabetes or diabetic complications;

Eye diseases, disorders or conditions, including autoimmune diseases of eyes, keratoconjunctivitis, vernal conjunctivitis, uveitis (including uveitis and lens uveitis related to Behcet's disease), keratitis, herpetic keratitis, keratitis conus, corneal epithelial dystrophy, leukoplakia, ocular pemphigus, Moran ulcer, scleritis, Grave's eye disease, Vogt-Koyanagi-Harada syndrome, keratoconjunctivitis sicca (dry eye) , blisters, iridocyclitis, sarcoidosis, endocrine ophthalmopathy, sympathetic ophthalmia, allergic conjunctivitis, or ocular neovascularization;

Intestinal inflammation, allergies or conditions, including Crohn's disease and/or ulcerative colitis, inflammatory bowel disease, celiac disease, proctitis, eosinophilic gastroenteritis or mastocytosis;

Neurodegenerative diseases, including motor neuron disease, Alzheimer's disease, Parkinson's disease, amyotrophic lateral sclerosis, Huntington's disease, neurodegenerative disease caused by cerebral ischemia or traumatic injury, stroke, glutamate neurotoxicity or hypoxia; stroke ischemialreperfusion injury, myocardial ischemia, renal ischemia, heart attack, cardiac hypertrophy, atherosclerosis and arteriosclerosis, organ hypoxia or platelet aggregation;

Skin diseases, conditions or disorders, including atopic dermatitis, eczema, psoriasis, scleroderma, itching or other pruritic conditions, vitiligo, hair loss;

Allergies, including mammalian allergic dermatitis (including equine allergic diseases, such as bite allergies), summer eczema, Culex mosquito itch syndrome (sweet itch), emphysema, inflammatory airway disease, recurrent airway obstruction, airway overreaction, or chronic obstructive pulmonary disease;

Asthma and other obstructive airway diseases, including chronic or refractory asthma, advanced asthma, bronchitis, bronchial asthma, allergic asthma, endogenous asthma, exogenous asthma or dusty asthma; and

Transplant rejection, including islet transplant rejection, bone marrow transplant rejection, graft versus host disease, organ and cell transplant rejection (for example bone marrow, cartilage, cornea, heart, intervertebral disc, pancreatic islets, kidney, limbs, liver, lung, muscle, myoblasts , nerve, pancreas, skin, small intestine or trachea) or xenotransplantation.

In a fourth aspect, the present disclosure provides a method for treating JAK-related diseases or disorders, the method comprising administrating a therapeutically effective amount of the compound as described above, or an optical isomer thereof, a geometric isomer thereof, a tautomer thereof or a mixture of various isomers, or a pharmaceutically acceptable salt thereof, or a prodrug thereof, or a metabolite thereof, or the composition as described above to patients in need. Among them, the patient is preferably a mammal, and more preferably a human patient. The route of administration can be oral, topical (including but not limited to external application, spraying, and the like), parenteral (including subcutaneous, intramuscular, cortical, and intravenous) administration, bronchial administration, or nasal administration. Among them, it is preferably administered orally or topically. It is more preferably administered orally.

Unexpectedly, the compound of the present disclosure demonstrated excellent efficacy as a JAK kinase inhibitor in experiments that is superior to existing JAK kinase inhibitors, such as Filgotinib, and had good safety potentially.

Preferably, the present disclosure provides the following embodiments:

Embodiment 1. a compound of Formula (I) as a JAK inhibitor

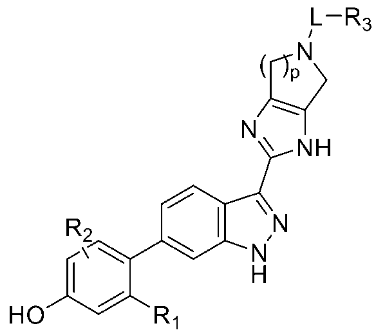

(I)

or an optical isomer thereof, a geometric isomer thereof, a tautomer thereof or a mixture of various isomers, or a pharmaceutically acceptable salt thereof, or a prodrug thereof, or a metabolite thereof,

wherein

L is C=O, O=S=O, $CH_2$ or a covalent bond; and

$R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom; and

$R_3$ is H, -N($R_{17}$)($R_{18}$), $C_{1-6}$ alkoxy, -$SR_{12}$, -$OR_{12}$, -CN, halogen, -$NO_2$, -$SF_5$, -S-$C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, or 5-11 membered bicyclic heteroalkyl, and $R_{13}$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, and 5-11 membered bicyclic heteroalkyl, and are optionally substituted by one or more substituents each independently selected from the group consisting of: -OH, -CN, -SH, halogen, -$NO_2$, -$SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl,-N($R_9$)($R_{10}$), -N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(=O)$_2$-N($R_9$)($R_{10}$), -$SR_{12}$, and -$OR_{12}$, wherein the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted by 1, 2, or 3 substituents each independently selected from the group consisting of: halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$),-C(=O)-$OR_{12}$, -C(=O)-H, -C(=O)-$R_{12}$, -C(=O)-N($R_9$)($R_{10}$), -N($R_{11}$)(S(=O)$_2R_{12}$), -S(=O)$_2$-N($R_9$)($R_{10}$), -$SR_{12}$, and -$OR_{12}$; or $R_{17}$, $R_{18}$, and the nitrogen atom to which they are attached together form a 3-14 membered ring.

0, 1, 2, 3 or 4 $R_2$(s) are present in Formula (I), and $R_2$ is selected from H, halogen, -OH, -$NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), -N($R_{11}$)(C(=O)$R_{12}$),-C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(= O)$_2$-N($R_9$)($R_{10}$), -$SR_{12}$ and -$OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$,-C(=O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; and

p is 1, 2 or 3; and

$R_a$ is selected from H, halogen, -OH, -$NO_2$, -CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15membered tricyclyl, $C_{5-11}$bicycloalkyl, 5-11 membered bicyclic hetero-

alkyl, $-N(R_9)(R_{10})$,$-N(R_{11})(C(=O)R_{12})$, $-C(=O)-N(R_9)(R_{10})$, $-C(=O)-R_{12}$, $-C(=O)-OR_{12}$, $-OC(=O)R_{12}$,$-N(R_{11})(S(=O)_2R_{12})$, $-S(=O)_2-N(R_9)(R_{10})$, $-SR_{12}$ and $-OR_{12}$, in which the $-S-C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_b$(s); and

$R_b$ is selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, $-N(R_5)(R_6)$,$-N(R_{11})(C(=O)R_{12})$, $-CON(R_7)(R_8)$, $-C(=O)-R_{12}$, $-C(=O)-OR_{12}$, $-OC(=O)R_{12}$,$-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen,-CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, $-C(=O)-N(R_9)(R_{10})$,$-N(R_{11})(S(=O)_2R_{12})$, $-S(=O)_2-N(R_9)(R_{10})$, $-SR_{12}$ and $-OR_{12}$; and

$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option in the group is optionally substituted with 1, 2, 3 or 4 substituent(s) each independently selected from the group consisting of halogen, $-CF_3$, -OH, $-NH_2$,$-NH(CH_3)$, $-N(CH_3)_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, $-S-C_{1-4}$ alkyl, -C(=O)H, $-C(=O)-C_{1-4}$ alkyl, $-C(=O)-O-C_{1-4}$ alkyl, $-C(=O)-NH_2$, $-C(=O)-N(C_{1-4}\ alkyl)_2$, $C_{1-4}$ haloalkyl, $C_{1-4}$ alkoxy and $C_{1-4}$ haloalkoxy.

Embodiment 2. The compound, or an optical isomer thereof, a geometric isomer thereof, a tautomer thereof or a mixture of various isomers, or a pharmaceutically acceptable salt thereof, or a prodrug thereof, or a metabolite thereof according to Embodiment 1, wherein L is C=O, O=S=O or $CH_2$.

Embodiment 3. The compound, or an optical isomer thereof, a geometric isomer thereof, a tautomer thereof or a mixture of various isomers, or a pharmaceutically acceptable salt thereof, or a prodrug thereof, or a metabolite thereof according to Embodiment 1 or 2, wherein $R_1$ is $-S-C_{1-4}$ alkyl, $C_{1-6}$ alkyl, $C_{1-6}$ alkoxy, or $C_{1-6}$ haloalkoxy, wherein at least one H atom on $R^1$ is replaced by a D atom, preferably all H atoms on $R^1$ are replaced by D atoms.

Embodiment 4. The compound, or an optical isomer thereof, a geometric isomer thereof, a tautomer thereof or a mixture of various isomers, or a pharmaceutically acceptable salt thereof, or a prodrug thereof, or a metabolite thereof according to Embodiment 1 or 2, wherein $R_1$ is $C_{1-6}$ alkyl, wherein at least one H atom on $R^1$ is replaced by a D atom, preferably all H atoms on $R^1$ are replaced by D atoms.

Embodiment 5. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein $R_3$ is H, $-N(R_{17})(R_{18})$, $C_{1-6}$ alkoxy, -OH, -SH, -CN, halogen,$-NO_2$, $-SF_5$, $-S-C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, or 5-11 membered bicyclic heteroalkyl, and $R_3$ is substituted with 0, 1, 2, 3, or 4 $R_a$(s);in which $R_{17}$ and $R_{18}$ are each independently selected from H, $C_{1-6}$ alkyl, $C_{1-6}$ alkoxy, $C_{3-7}$ haloalkyl, $C_{3-7}$ cycloalkyl, $C_{3-7}$ heterocycloalkyl, $C_{5-7}$ aryl, and 5-7 membered heteroaryl, and are optionally substituted by one or more of -OH,-CN, -SH, halogen, $-NO_2$, and $-SF_5$.

Embodiment 6. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein $R_3$ is $-N(R_{17})(R_{18})$, $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 5-11 membered bicyclic heteroalkyl, and $R_3$ is substituted with 0, 1, 2, 3, or 4 $R_a$(s).

Embodiment 7. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein $R_3$ is $-N(R_{17})(R_{18})$, $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_3$ is substituted with 0, 1, 2, 3, or 4 $R_a$(s).

Embodiment 8. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, phenyl, or 5-6 membered heteroaryl, and $R_3$ is substituted with 0, 1, 2, 3, or 4 $R_a$(s).

Embodiment 9. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein $R_3$ is $C_{3-7}$ cycloalkyl, or 3-7 membered heterocycloalkyl and are optionally substituted by one or more of -OH, -CN, -SH, halogen, $-NO_2$, and $-SF_5$.

Embodiment 10. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein $R_3$ is -N($R_{17}$)($R_{18}$), and $R_{17}$ and $R_{18}$ are each independently selected from H, $C_{1-6}$ alkyl, $C_{1-6}$ alkoxy, $C_{3-7}$ haloalkyl, and $C_{3-7}$ cycloalkyl, and are optionally substituted by one or more of -OH, -CN, -SH, halogen, $-NO_2$, and $-SF_5$

Embodiment 11. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein $R_3$ is -N($R_{17}$)($R_{18}$), and $R_{17}$, $R_{18}$, and the nitrogen atom to which they are attached together form a 4-10-membered ring, and the ring is optionally substituted by one or more of -OH, -CN, -SH, halogen, $-NO_2$, and $-SF_5$.

Embodiment 12. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 4, wherein L is C=O; $R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl,, wherein at least one H atom on $R_1$ is replaced by a D atom; and $R_3$ is -N($R_{17}$)($R_{18}$), $C_{1-6}$ alkoxy, $C_{1-6}$ alkyl, $C_{3-7}$ cycloalkyl, 3-7 membered heterocycloalkyl, $C_{5-7}$ aryl, 5-7 membered heteroaryl, $C_{5-11}$ bicyclic alkyl, $C_{7-11}$ bicyclic aryl, 7-11 membered bicyclic heteroaryl, or 5-11 membered bicyclic heteroalkyl, and $R_3$ is substituted with 0, 1, 2, 3, or 4 $R_a$(s); wherein $R_{17}$ and $R_{18}$ are each independently selected from H, $C_{1-6}$ alkyl, $C_{1-6}$ alkoxy, $C_{1-6}$ alkoxy, $C_{3-7}$ haloalkyl, $C_{3-7}$ heterocycloalkyl, $C_{5-7}$ aryl, and 5-7 membered heteroaryl, and optionally substituted with one or more of -OH, -CN, -SH, halogen, $-NO_2$, $-SF_5$; or $R_{17}$, $R_{18}$, and the N atom to which they are attached together form a 3-14 membered ring, and the ring is optionally substituted with one or more of -OH, -CN, -SH, halogen, $-NO_2$, and $-SF_5$.

Embodiment 13. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 12, wherein one, two or three $R_2$(s) are present and $R_2$ is selected from H, halogen, -OH, $-NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the -S-$C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy.

Embodiment 14. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 12, wherein one, two or three $R_2$(s) are present and $R_2$ is selected from halogen, $C_{1-6}$ alkyl and $C_{3-6}$ cycloalkyl , in which $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy.

Embodiment 15. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 12, wherein one or two $R_2$(s) are present and $R_2$ is selected from halogen and $C_{1-6}$ alkyl.

Embodiment 16. The compound, or an optical isomer thereof, a geometric isomer thereof, a tautomer thereof or a mixture of various isomers, or a pharmaceutically acceptable salt thereof, or a prodrug thereof, or a metabolite thereof

according to any one of Embodiments 1 to 12, wherein $R_3$ is substituted with 0 or 1 $R_a$, and $R_a$ is selected from halogen, -OH, -CN, $C_{1-6}$ alkyl, $C_{3-7}$ cycloalkyl, and 5-7 membered heterocycloalkyl, wherein the $C_{1-6}$ alkyl, $C_{3-7}$ cycloalkyl, 5-7 membered heterocycloalkyl are optionally substituted with 1, 2, or 3 $R_b$(s).

Embodiment 17. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 12, wherein the compound is selected from:

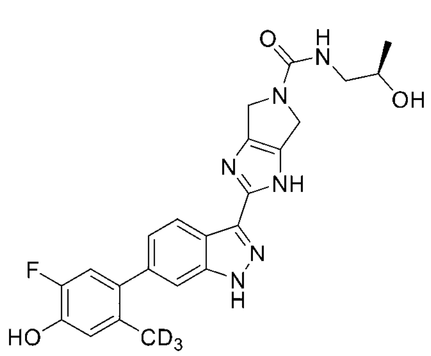

,

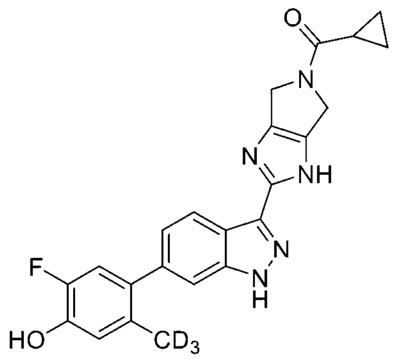

,

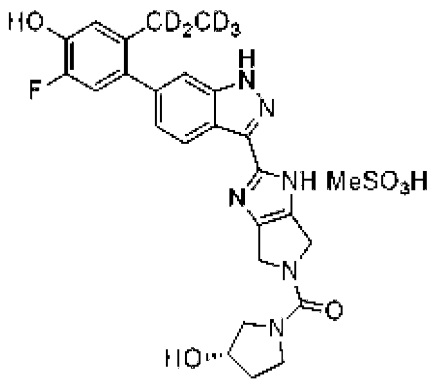

,

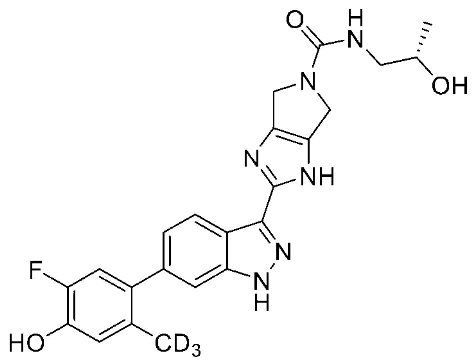

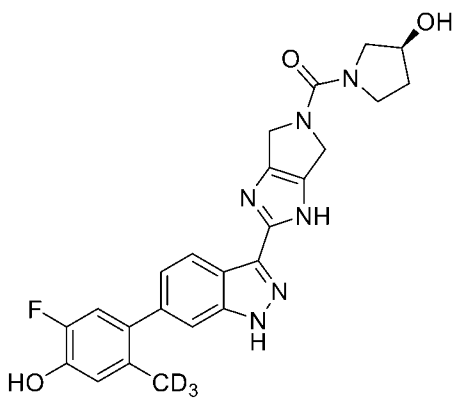

,

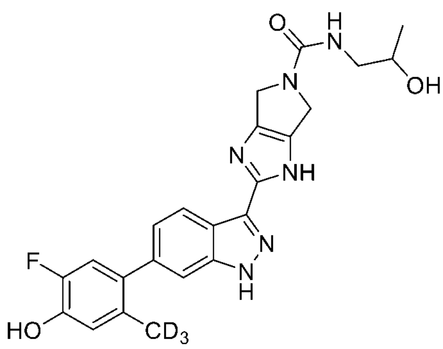

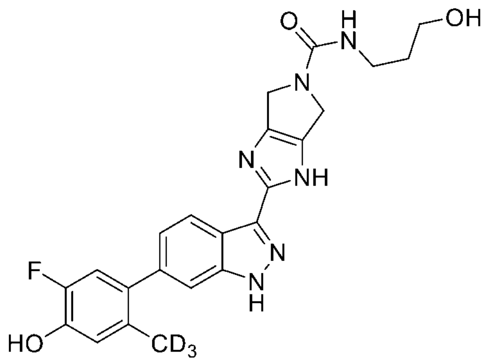
,
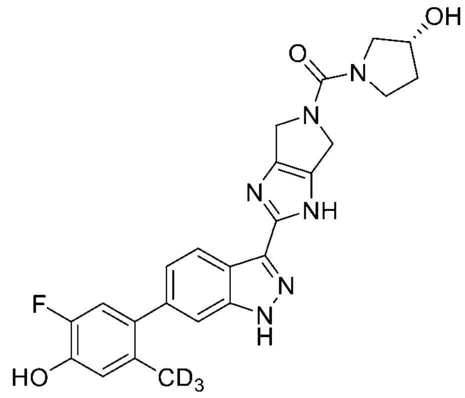

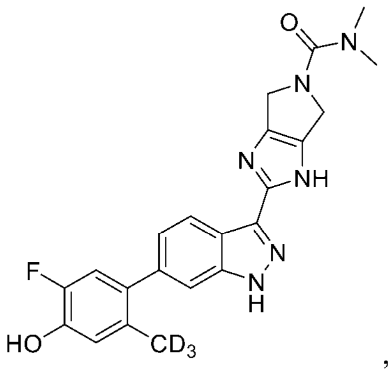
, and
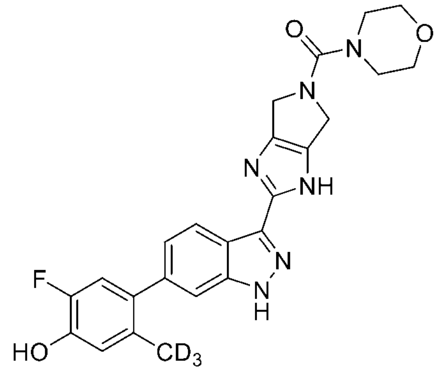
.

Embodiment 18. The compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiments 1 to 12, wherein the compound is selected from:

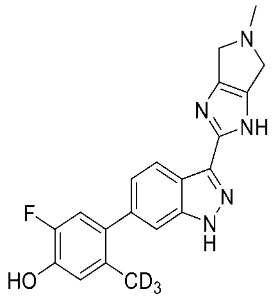
12
,
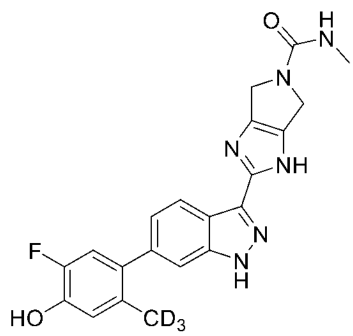
,
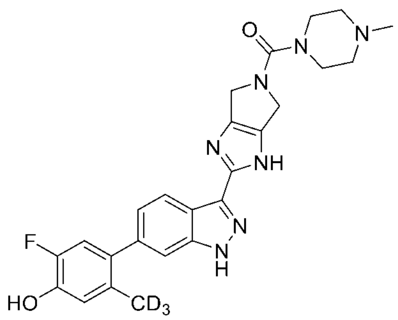
,

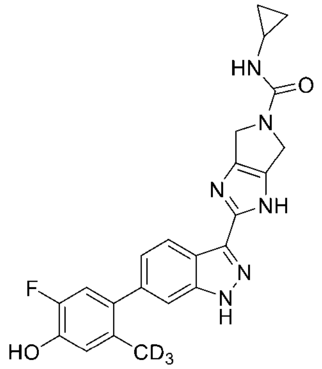
,
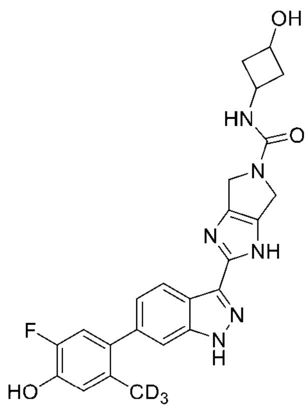
,
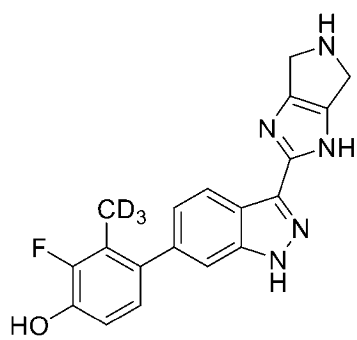
,

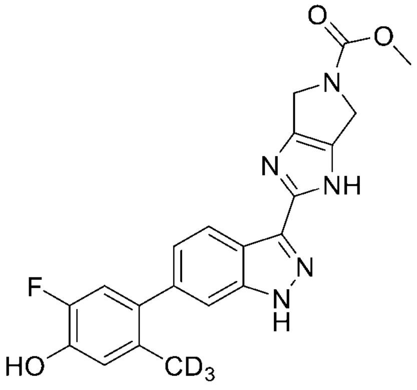,
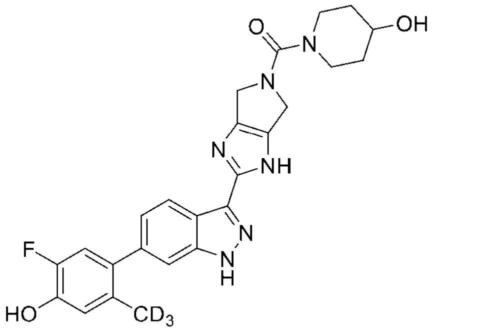,

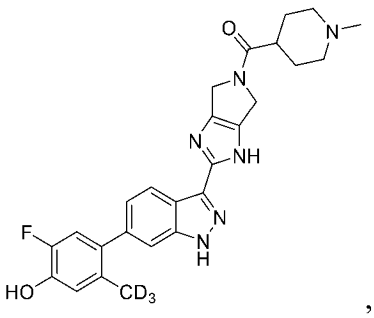,
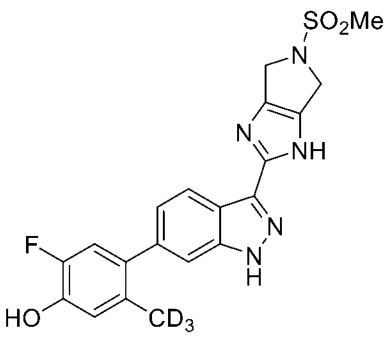,
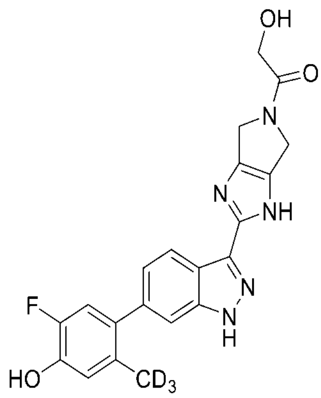,

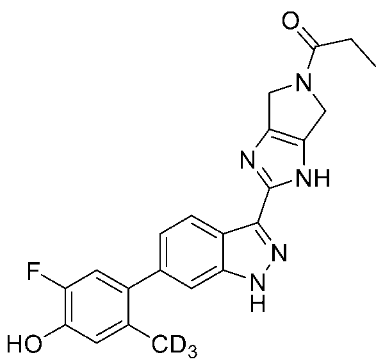,
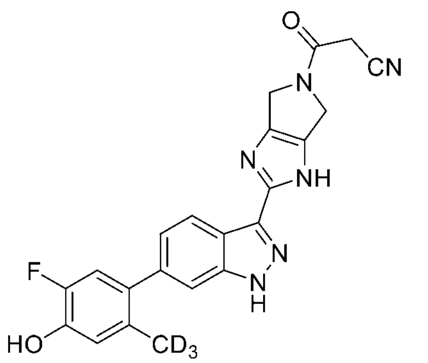,

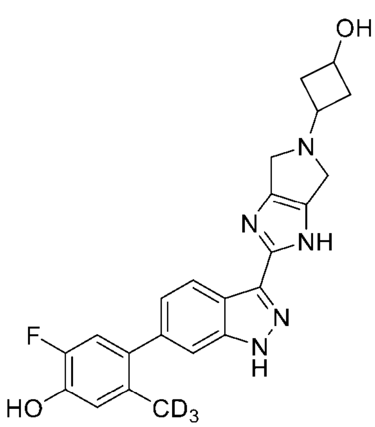,
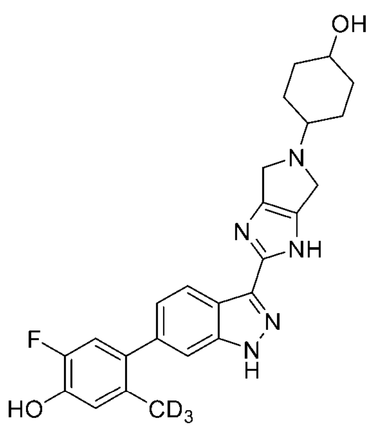,
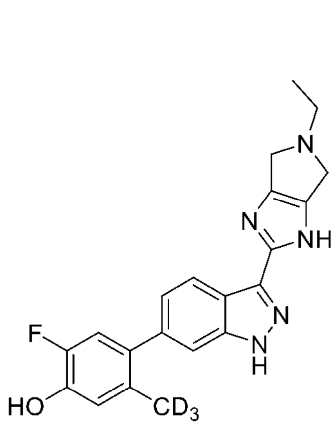,

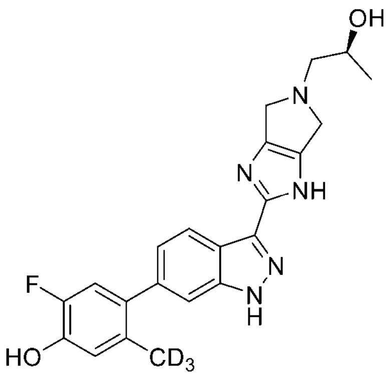

,

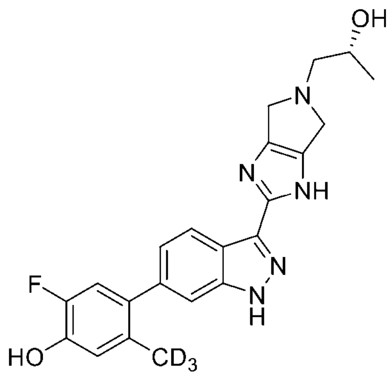

,

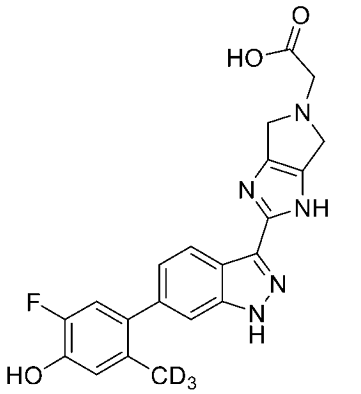

,

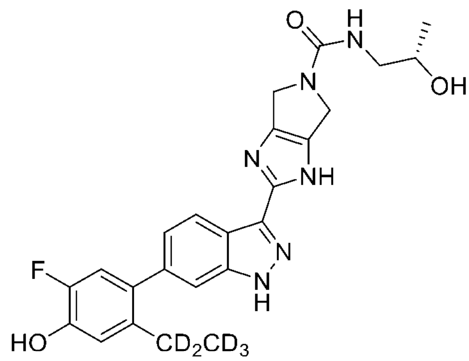

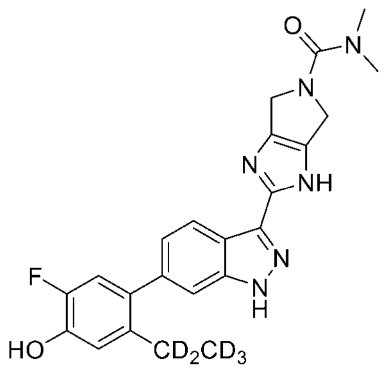

,

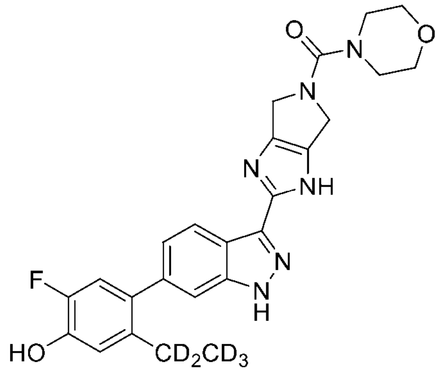

,

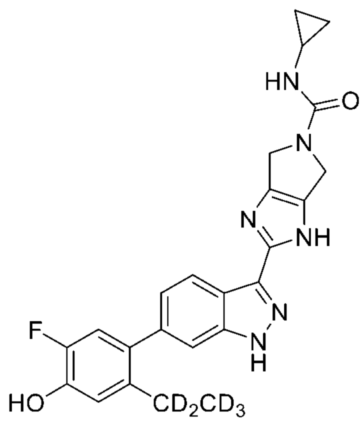

,

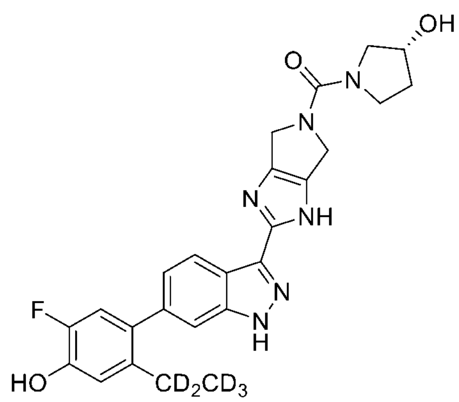

and

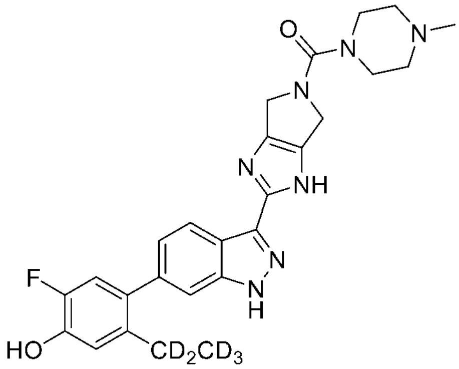

.

Embodiment 19. A pharmaceutical composition, comprising the compound, or an optical isomer thereof, a geometric isomer thereof, a tautomer thereof or a mixture of various isomers, or a pharmaceutically acceptable salt thereof, or a prodrug thereof, or a metabolite thereof according to any one of Embodiemnts 1 to 18, and one or more pharmaceutically acceptable carriers, adjuvants or excipients.

Embodiment 20. Use of the compound, or optical isomer thereof, geometric isomer thereof, a tautomer thereof or a mixture of various isomers, or a pharmaceutically acceptable salt thereof, or a prodrug thereof, or a metabolite thereof according to any one of Embodiments 1 to 18 or the pharmaceutical composition of Embodiment 19 in the manufacture of a medicament for the treatment and/or prevention of a JAK-related disease or disorder.

Embodiment 21. The use according to Embodiment 20, wherein the JAK-related disease or disorder is selected from the group consisting of arthritis, autoimmune diseases or disorders, cancer or tumor, diabetes, eye diseases, disorders or conditions, intestinal inflammation, allergies or conditions, neurodegenerative diseases, skin diseases, conditions or disorders, allergies, asthma and other obstructive airway diseases, and transplant rejection.

Embodiment 22. A method for treating a JAK-related disease or disorder, comprising administrating to a patient in need a therapeutically effective amount of the compound, or optical isomer thereof, geometric isomer thereof, a tautomer thereof or a mixture of various isomers, or a pharmaceutically acceptable salt thereof, or a prodrug thereof, or a metabolite thereof according to any one of Embodiments 1 to 18 or the pharmaceutical composition of Embodiment 19.

Embodiment 23. The method according to Embodiment 22, wherein the JAK-related disease or disorder is selected from the group consisting of arthritis, autoimmune diseases or disorders, cancer or tumor, diabetes, eye diseases, disorders or conditions, intestinal inflammation, allergies or conditions, neurodegenerative diseases, skin diseases, conditions or disorders, allergies, asthma and other obstructive airway diseases, and transplant rejection.

The present disclosure will be further illustrated and described below in conjunction with the drawings and specific examples.

## EXAMPLES

The compounds of formula (I) of the present disclosure can be synthesized by various methods familiar to those skilled in the art of organic synthesis. The following specific examples give some exemplary synthesis methods of the compounds of formula (I), and these methods are well-known in the field of synthetic chemistry. Obviously, referring to the exemplary embodiments of the present application, those skilled in the art can appropriately adjust reactants, reaction conditions, and protective groups to easily design other synthetic routes for compounds of formula (I).

**Synthesis of Intermediate Int 1:** Tert-butyl 2-(6-Bromo-1-(tetrahydro-2H-**pyran-2-yl)-1H-indazol-3-yl)-1-((2-(trimethylsilyl)ethoxy)methyl)-4,6-dihydropyrrolo[3,4-d]imidazole-5(1H)-carboxylate**

**Synthetic Route**

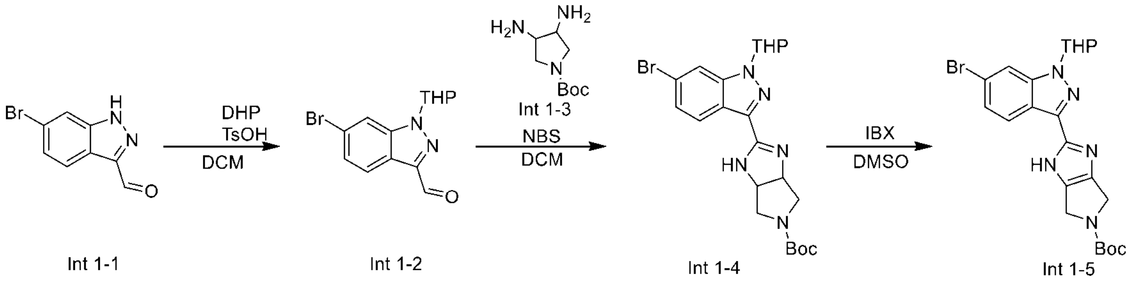


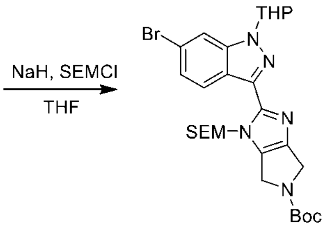


Int 1

**Synthetic Method**

**Synthesis of 6-bromo-1-(tetrahydro-2H-pyran-2-yl)-1H-indazole-3-carbaldehyde (Int 1-2)**

To a clean 500 L reactor were added 467 kg of dichloromethane, 35.4 kg of 6-bromo-1H-indazole-3-carbaldehyde (Int 1-1), and 5.30 kg of TsOH (p-toluenesulfonic acid). The mixture was cooled to 0-10°C, to which 15.91 kg of DHP (dihydropyran) was added in batches, maintaining the temperature within a range of 0-10°C. After the addition was complete, the temperature was raised to 20-30°C and the reaction was carried out for 3±1 hours. After completion of the reaction, the reaction mixture was slowly added to 44.2 kg of an 8% sodium bicarbonate aqueous solution, and the resulting mixture was stirred for 0.5 hours while controlling the temperature between 10-20°C, and filtered. The filtrate was allowed to stand for phase separation. The organic phase was dried over sodium sulfate and concentrated to dryness. The residue was then slurried with 39.6 kg of ethyl acetate and 120 kg of n-heptane at 15-25°C for 2 hours, filtered, and the filter cake was washed once with 12.0 kg of n-heptane. The filter cake was dried to yield 34.0 kg of the target product Int 1-2.

**Synthesis of tert-butyl 2-(6-bromo-1-(tetrahydro-2H-pyran-2-yl)-1H-indazol-3-yl)-3a,4,6,6a-tetrahydropyrrolo [3,4-d]imidazole-5(1H)-carboxylate (Int 1-4)**

To a clean 500 L reactor were added 198 kg of dichloromethane, followed by 15.0 kg of Int 1-2 and 15.0 kg of tert-butyl 3,4-diaminopyrrolidine-1-carboxylate (Int 1-3). 9.45 kg of NBS (N-bromosuccinimide) was added to the reactor in batches, maintaining the temperature within 15-30°C. After the addition was complete, the mixture was stirred for 4 hours, controlling the temperature between 20-30°C. Subsequently, 2.40 kg of Int 1-3 was added, followed by 1.50 kg of NBS added in batches, maintaining the temperature within 15-30°C. After completion of addition, the mixture was stirred for 1 hour for reaction while controlling the temperature between 20-30°C. Another 2.40 kg of Int 1-3 was added and then 1.50 kg of NBS was added in batches, maintaining the temperature within 15-30°C. After completion of addition, the mixture was stirred for another hour while controlling the temperature between 20-30°C. The reaction liquid was slowly poured into 49.95 kg of a 5% sodium sulfite aqueous solution. Then, 43.80 kg of a 10% potassium carbonate aqueous solution was added to adjust the pH to ≥9, and the mixture was stirred for 0.5 hours while controlling the temperature between 10-20°C. The mixture was allowed to stand for phase separation. The aqueous phase was extracted once with 48.3 kg of dichloromethane. The combined organic phases were dried over magnesium sulfate and concentrated to dryness. The residue was slurried with 55.5 kg of tert-butyl methyl ether at 5-15°C for 2 hours, filtered, and the filter cake was washed once with 5.00 kg of tert-butyl methyl ether. The filter cake was vacuum-dried at 35-45°C under a vacuum of ≤-0.07 MPa for 12 hours to yield 18.6 kg of the target product Int 1-4. LC-MS m/z (ESI) $[M+H]^+$ calcd for $C_{22}H_{29}BrN_5O_3$: 490.2; found: 490.1.

**Synthesis of tert-butyl 2-(6-bromo-1-(tetrahydro-2H-pyran-2-yl)-1H-indazol-3-yl)-4,6-dihydropyrrolo[3,4-d]imidazole-5(1H)-carboxylate (Int 1-5)**

To a clean 500 L reactor were added 143.2 kg of DMSO (dimethyl sulfoxide) and 18.6 kg of Int 1-4. The mixture was stirred and heated to 35-40°C, and a pre-prepared solution of 16.0 kg of 2-iodoxybenzoic acid (IBX) in 61.4 kg of DMSO was added dropwise, maintaining the temperature between 35-40°C. After the addition, the mixture was stirred at this temperature for 5 hours, then cooled to 20-30°C. The reaction mixture was slowly added to 233 kg of a 3.3% sodium sulfite aqueous solution. Subsequently, 38.9 kg of a 10% potassium carbonate aqueous solution and 98.4 kg of tert-butyl methyl ether were added. After stirring, the mixture was filtered, and the filter cake was washed once with 57.5 kg of tert-butyl methyl ether. The filtrate was allowed to stand for phase separation. The aqueous phase was extracted once with the wash filtrate. The combined organic phases were washed once with 116 kg of water, dried over sodium sulfate, and concentrated to yield 18.4 kg of brown oily Int 1-5, which was used directly in the next step.

**Synthesis of tert-butyl 2-(6-bromo-1-(tetrahydro-2H-pyran-2-yl)-1H-indazol-3-yl)-1-((2-(trimethylsilyl)ethoxy)methyl)-4,6-dihydropyrrolo[3,4-d]imidazole-5(1H)-carboxylate (Int 1)**

To a clean 500 L reactor were added 65.5 kg of THF (tetrahydrofuran) under nitrogen protection. The mixture was stirred and cooled to 0-10°C, and 1.76 kg of sodium hydride (60%) was added in batches, venting during addition and protecting with a nitrogen flow, while maintaining the temperature between 0-10°C. After stirring for 0.5 hours, a pre-prepared solution of Int 1-5 (18.4 kg) in 49.1 kg of THF was added dropwise, maintaining the temperature between 0-10°C under a nitrogen flow. After the addition, 7.36 kg of (2-(trimethylsilyl)ethoxy)methoxy chloride (SEMCl) was added dropwise, maintaining the temperature between 0-10°C under a nitrogen flow. After completion of addition, the temperature was then raised to 10-20°C and the mixture was stirred for 1 hour. After completion of reaction, the mixture was cooled to 0-10°C and slowly added under nitrogen protection to 115 kg of a 20% ammonium chloride aqueous solution, controlling the temperature between 0-20°C. The mixture was stirred for 0.5 hours and then phase-separated. The aqueous phase was extracted once with 56.7 kg of tert-butyl methyl ether. The combined organic phases were washed once with 38.5 kg of a 20% sodium chloride aqueous solution, dried over sodium sulfate, and concentrated to dryness. To the residue, 72.7 kg of methanol was added and stirred for 2 hours at 5-15°C. The mixture was filtered, and the filter cake was recrystallized by refluxing with 35.1 kg of acetonitrile at 70-80°C until clear. After cooling to 20-30°C, the mixture was filtered, and the filter cake was washed once with 7.36 kg of acetonitrile. The filter cake was vacuum-dried at 35-45°C under a vacuum of ≤ -0.07 MPa for 7 hours to yield 10.1 kg of off-white powdery solid Int 1. LC-MS m/z (ESI) $[M+H]^+$ calcd for $C_{28}H_{41}BrN_5O_4Si$: 618.2; found: 618.2.

**Synthesis of Intermediate Int 2: tert-butyl 2-(6-Bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (Int 2)**

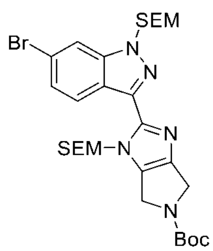

Int 2

Int 2 can be obtained via a synthetic method analogous to that used for the preparation of Int 1, using suitable starting materials. LC-MS m/z (ESI) $[M+H]^+$ calcd for $C_{29}H_{47}BrN_5O_4Si_2$: 664.2; found: 664.2.

<u>**Example 1: (R)-2-(6-(5-Fluoro-4-hydroxy-2-(methyl-d3)phenyl)-1H-indazol-3-yl)-N-(2-hydroxypropyl)-4,6-dihydropyrrolo[3,4-d]imidazole-5(1H)-carboxamide (Compound 1)**</u>

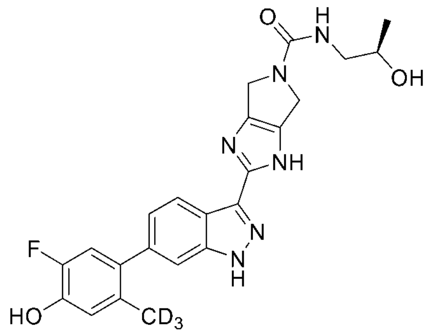

1

**Synthetic Route for Compound 1:**

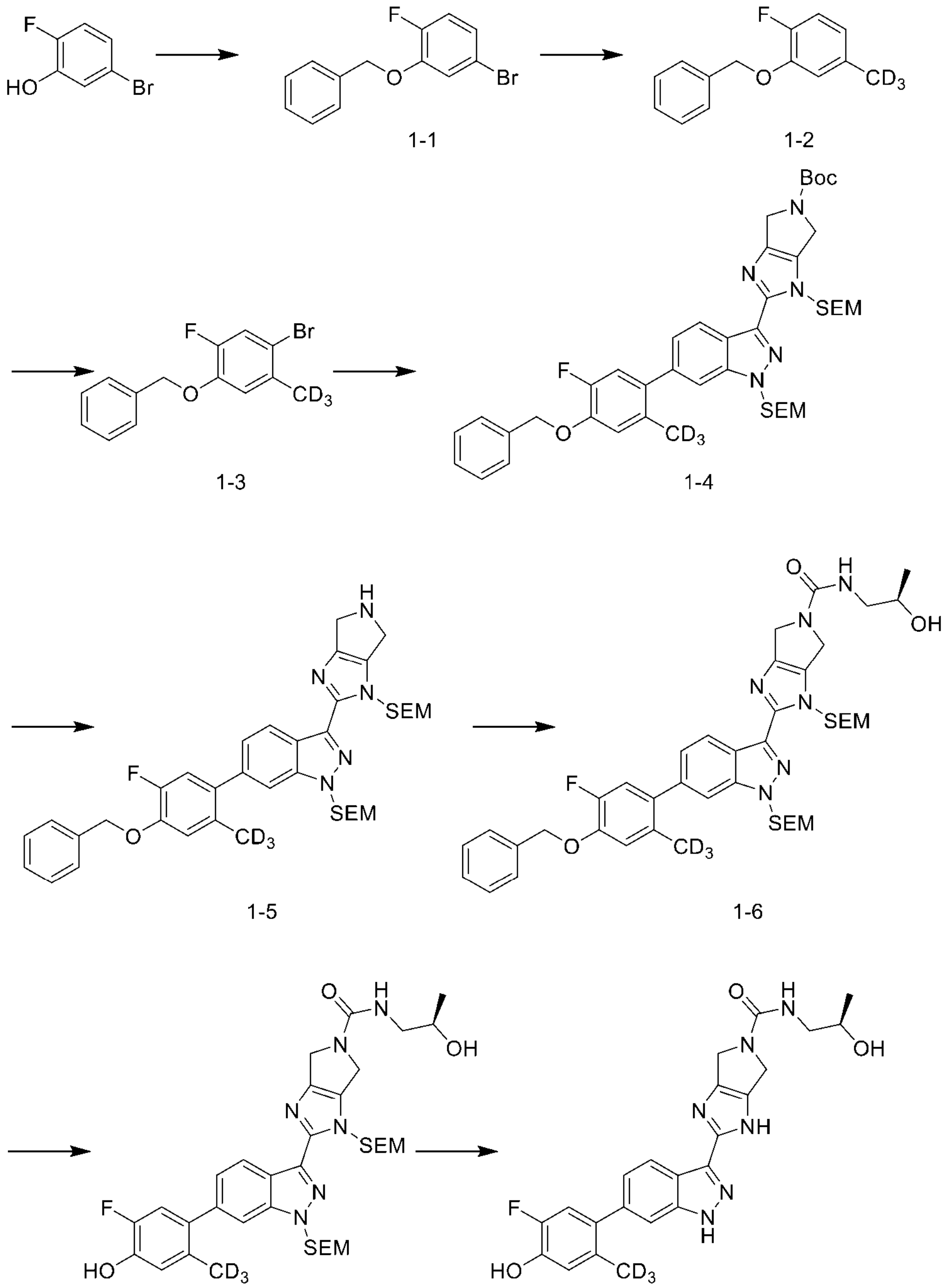

1-1 1-2

1-3 1-4

1-5 1-6

1-7 1

**Synthetic Method:**

**Synthesis of Intermediate 1-1: 2-(Benzyloxy)-4-bromo-1-fluorobenzene**

5-Bromo-2-fluorophenol (2.0 g, 10.47 mmol) and benzyl bromide (2.2 g, 12.57 mmol) were dissolved in 20 ml of acetonitrile. Potassium carbonate (2.2 g, 15.71 mmol) was added, and the mixture was stirred at room temperature for 3 h. After completion of reaction, water was added to the reaction mixture, and it was extracted twice with ethyl acetate. The combined organic phases were washed with saturated brine, dried over anhydrous sodium sulfate, concentrated, and purified by silica gel column chromatography to yield 2.8 g of intermediate 1-1, in a yield of 95.1%.

$^1H$ NMR (400 MHz, $CDCl_3$) δ 7.52-7.32 (m, 5H), 7.17 (dd, J = 7.5 Hz, J = 2.3 Hz, 1H), 7.10-6.92 (m, 2H), 5.14 (s, 2H).

**Synthesis of Intermediate 1-2: 2-(Benzyloxy)-1-fluoro-4-(methyl-d3)benzene**

Intermediate 1-1 (230.0 mg, 0.82 mmol) was placed in a three-necked flask, to which tetrakis(triphenylphosphine)palladium (94.5 mg, 0.082 mmol) was added, and the atmosphere was replaced with nitrogen. 8 ml of dioxane was added for dissolution, to which a solution of deuterated methylmagnesium iodide in diethyl ether (2.5 ml, 2.45 mmol) was added dropwise slowly at room temperature. After the addition, the reaction mixture was placed in an 80°C oil bath and stirred for 16 hours. After completion, the reaction mixture was cooled to room temperature, quenched with water, and extracted twice with ethyl acetate. The combined organic phases were washed with saturated brine, dried over anhydrous sodium sulfate, concentrated, and purified by silica gel column chromatography, eluting with pure petroleum ether, to yield 190.3 mg of crude intermediate 1-2, in a yield of 105.9 %.

$^1$H NMR (400 MHz, $CDCl_3$) δ 7.52-7.31 (m, 5H), 6.99 (dd, J = 11.3 Hz, J = 8.2 Hz, 1H), 6.85 (dd, J = 8.1 Hz, J = 2.1 Hz, 1H), 6.72 (ddd, J = 8.2 Hz, J = 4.3 Hz, J = 2.1 Hz, 1H), 5.14 (s, 2H).

**Synthesis of Intermediate 1-3: 1-(Benzyloxy)-4-bromo-2-fluoro-5-(methyl-d3)benzene**

Intermediate 1-2 (190.3 mg, 0.87 mmol) was dissolved in 10 ml of acetonitrile, to which copper(II) bromide (579.1 mg, 2.60 mmol) was added. It was allowed to react at room temperature for 16 hours. After completion of reaction, water was added and stirred until the mixture cleared. The resulting mixture was then extracted twice with ethyl acetate. The combined organic phases were washed with saturated brine, dried over anhydrous sodium sulfate, concentrated, and purified by silica gel column chromatography, eluting with pure petroleum ether, to yield 135.2 mg of crude intermediate 1-3, in a yield of 52.3 %.

**Synthesis of Intermediate 1-4: tert-butyl 2-(6-(4-(Benzyloxy)-5-fluoro-2-(methyl-d3)phenyl)-1-((2-(trimethylsilyl) ethoxy)methyl)-1H-indazol-3-yl)-1-((2-(trimethylsilyl)ethoxy)methyl)-4,6-dihydropyrrolo[3,4-d]imidazole-5(1H)-carboxylate**

Intermediate 1-3 (135.2 mg, 0.45 mmol) and bis(pinacolato)diboron(126.6 mg, 0.50 mmol) were dissolved in 8 ml of dioxane, to which $Pd(dppf)Cl_2 \cdot CH_2Cl_2$ (37.1 mg, 0.05 mmol) and potassium acetate (133.1 mg, 1.36 mmol) were added, and the atmosphere was replaced with nitrogen. It was allowed to react at 100°C for 5.5 hours. After cooling to room temperature, 4 ml of water was added to the reaction solution. Tert-butyl 2-(6-Bromo-1-((2-(trimethylsilyl)ethoxy) methyl)-1H-indazol-3-yl)-1-((2-(trimethylsilyl)ethoxy)methyl)-4,6-dihydropyrrolo[3,4-d]imidazole-5(1H)-carboxylate (Int 2) was dissolved in 4 ml of dioxane and added to the reaction solution. Then, $Pd(dppf)Cl_2 \cdot CH_2Cl_2$ (37.1 mg, 0.05 mmol) and potassium carbonate (187.3 mg, 1.36 mmol) were added and the atmosphere was replaced with nitrogen. It was allowed to react at 100°C for 16 hours. After completion, water was added, and the resulting mixture was extracted twice with ethyl acetate. The combined organic phases were washed with saturated brine, dried over anhydrous sodium sulfate, concentrated, and purified by silica gel column chromatography to yield 103.4 mg of intermediate 1-4, in a yield of 28.3 %.

$^1$H NMR (400 MHz, $CDCl_3$) δ 8.53-8.46 (m, 1H), 7.63-7.56 (m, 1H), 7.53-7.50 (m, 2H), 7.47-7.30 (m, 5H), 7.27-7.19 (m, 1H), 5.97-5.93 (m, 2H), 5.82-5.77 (m, 2H), 5.24 (d, J = 20.7 Hz, 2H), 4.76-4.54 (m, 4H), 3.67-3.56 (m, 4H), 1.57 (s, 9H), 0.97-0.90 (m, 4H), -0.03 - -0.06 (m, 18H).

**Synthesis of Intermediate 1-5: 6-(4-(Benzyloxy)-5-fluoro-2-(methyl-d3)phenyl)-1-((2-(trimethylsilyl)ethoxy) methyl)-3-(1-((2-(trimethylsilyl)ethoxy)methyl)-1,4,5,6-tetrahydropyrrolo[3,4-d]imidazol-2-yl)-1H-**indazole

Intermediate 1-4 (103.4 mg, 0.13 mmol) was dissolved in 8 ml of dichloromethane, to which zinc bromide (143.4 mg, 0.64 mmol) was added. It was allowed to react at room temperature for 16 hours. After completion, aqueous ammonia was added and stirred until the mixture cleared. The mixture was extracted twice with dichloromethane. The combined organic phases were washed with saturated brine, dried over anhydrous sodium sulfate, and concentrated to yield 89.8 mg of crude intermediate 1-5, in a yield of 98.8 %.

**Synthesis of Intermediate 1-6: (R)-2-(6-(4-(Benzyloxy)-5-fluoro-2-(methyl-d3)phenyl)-1-((2-(trimethylsilyl) ethoxy)methyl)-1H-indazol-3-yl)-N-(2-hydroxypropyl)-1-((2-(trimethylsilyl)ethoxy)methyl)-4,6-dihydropyrrolo [3,4-d]imidazole-5(1H)-carboxamide**

Intermediate 1-5 (35.0 mg, 0.05 mmol) was dissolved in 8 ml of dichloromethane, to which triphosgene (14.7 mg, 0.05 mmol) was added at 0°C. The mixture was stirred for 1 min. Triethylamine (0.1 ml, 0.50 mmol) was added dropwise, producing white fumes. After stirring for 5 minutes, TLC showed the disappearance of the starting materials. The reaction mixture was warmed to room temperature, to which (R)-1-aminopropan-2-ol was added. It was allowed to react for 1 hour. After completion, water was added, and the resulting mixture was extracted twice with dichloromethane. The combined

organic phases were washed with saturated brine, dried over anhydrous sodium sulfate, concentrated, and purified by silica gel column chromatography to yield 28.3 mg of intermediate 1-6, in a yield of 69.9 %.

**Synthesis of Intermediate 1-7: (R)-2-(6-(5-Fluoro-4-hydroxy-2-(methyl-d3)phenyl)-1-((2-(trimethylsilyl)ethoxy) methyl)-1H-indazol-3-yl)-N-(2-hydroxypropyl)-1-((2-(trimethylsilyl)ethoxy)methyl)-4,6-dihydropyrrolo[3,4-d]imidazole-5(1H)-carboxamide**

Intermediate 1-6 (28.3 mg, 0.03 mmol) was dissolved in 4 ml of methanol, to which 10 mg of palladium on carbon was added. It was allowed to react at 45°C for 1 hour. After completion, the reaction mixture was filtered through a pad of Celite, and the filtrate was concentrated to yield 23.8 mg of crude intermediate 1-7, in a yield of 92.6 %.

**Synthesis of Final Product 1: (R)-2-(6-(5-Fluoro-4-hydroxy-2-(methyl-d3)phenyl)-1H-indazol-3-yl)-N-(2-hydroxypropyl)-4,6-dihydropyrrolo[3,4-d]imidazole-5(1H)-carboxamide**

Intermediate 1-7 (23.8 mg, 0.03 mmol) was dissolved in 4 ml of methanol to which 2 ml of concentrated hydrochloric acid was added. It was allowed to react at 50°C for 6 hours. After completion, the reaction mixture was concentrated. The residue was dissolved in methanol, and aqueous ammonia was added to adjust the pH to alkaline. The resulting mixture was concentrated and purified by silica gel plate to yield 3.2 mg of final product 1, in a yield of 20.5 %. LC-MS m/z (ESI) $[M+H]^+$ calcd for $C_{23}H_{20}D_3FN_6O_3$: 454.2; found: 454.3.

**Example 2: Cyclooropyl (2-(6-(5-fluoro-4-hydroxy-2-(methyl-d3)ohenyl)-1H-indazol-3-yl)-4,6-dihydropyrrolo [3,4-d]imidazol-5(1H)-yl) ketone (Compound 2)**

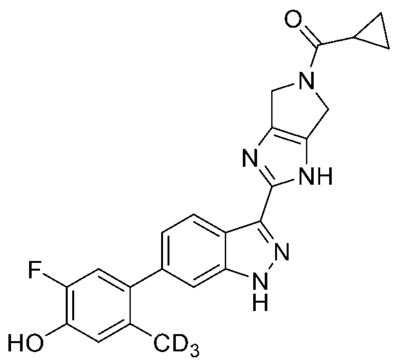

2

**Synthetic Route for Compound 2:**

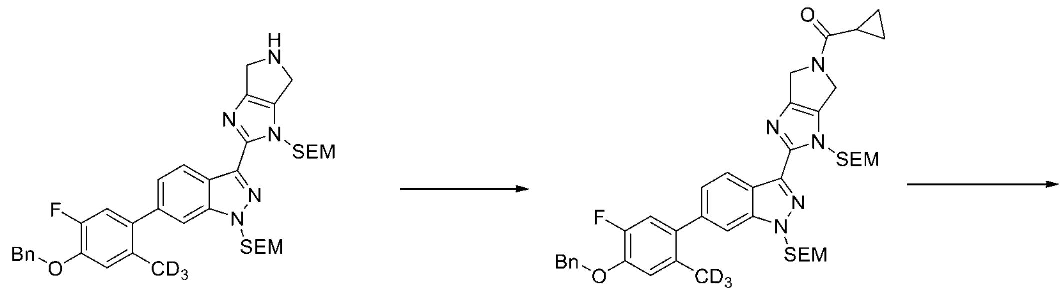

1-5 2-1

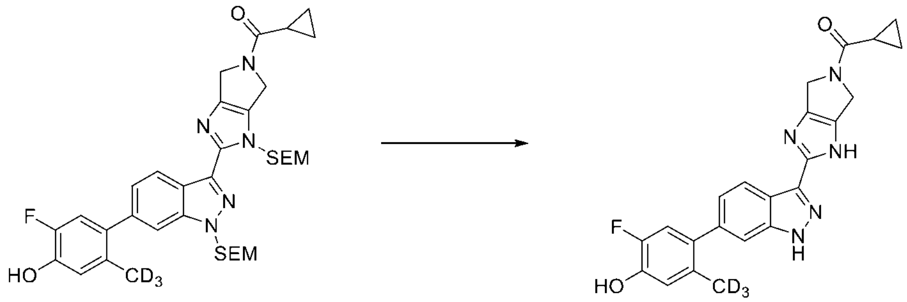

2-2 2

**Synthetic Method:**

**Synthesis of Intermediate 2-1: (2-(6-(4-(Benzyloxy)-5-fluoro-2-(methyl-d3)phenyl)-1-((2-(trimethylsilyl)ethoxy)methyl)-1H-indazol-3-yl)-1-((2-(trimethylsilyl)ethoxy)methyl)-4,6-dihydropyrrolo [3,4-d]imidazol-5(1H)-yl) (cyclopropyl) ketone**

Intermediate 1-5 (50.0 mg, 0.07 mmol) was dissolved in 10 ml of dichloromethane (DCM) to which triethylamine (10.8 mg, 0.11 mmol) was added at room temperature, followed by cyclopropanecarbonyl chloride (9.6 mg, 0.09 mmol). The mixture was stirred at room temperature for 0.5 hours. After completion, water was added to quench the reaction. The mixture was extracted with DCM. The organic phase was washed with saturated brine, dried over anhydrous sodium sulfate, concentrated, and purified by preparative TLC to yield 40.0 mg of intermediate 2-1, in a yield of 72.9%.

**Synthesis of Intermediate 2-2: Cyclopropyl (2-(6-(5-fluoro-4-hydroxy-2-(methyl-d3)phenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl) ketone**

Intermediate 2-1 (40.0 mg, 0.05 mmol) was dissolved in 3 ml of methanol, to which 4 mg of palladium on carbon was added, and the atmosphere was replaced with hydrogen. It was allowed to react at 40°C for 2 hours. After completion, the mixture was filtered, and the filtrate was concentrated to yield 28.0 mg of intermediate 2-2, in a yield of 79.2%.

**Synthesis of Compound 2: Cyclopropyl (2-(6-(5-fluoro-4-hydroxy-2-(methyl-d3)phenyl)-1H-indazol-3-yl)-4,6-dihydropyrrolo[3,4-d]imidazol-5(1H)-yl) ketone**

Intermediate 2-2 (28.0 mg, 0.04 mmol) was dissolved in 4 ml of methanol, to which 2 ml of concentrated hydrochloric acid was added. It was allowed to react at 50°C for 6 hours. The mixture was concentrated and dissolved in 5 ml of methanol, to which 0.5 ml of ammonia water was added. The resulting mixture was concentrated and the residue was purified by reverse-phase silica gel column chromatography to yield 4.2 mg of the final product, in a yield of 24.3%. LC-MS m/z (ESI) $[M+H]^+$ calcd for $C_{23}H_{17}D_3FN_5O_3$: 421.2; found: 421.2.

**Example 3: (S)-(2-(6-(2-(Ethyl-d5)-5-fluoro-4-hydroxyphenyl)-1H-indazol-3-yl)-4,6-dihydropyrrolo[3,4-d]imidazol-5(1H)-yl) (3-hydroxypyrrolidin-1-yl) ketone methanesulfonate (Compound 3)**

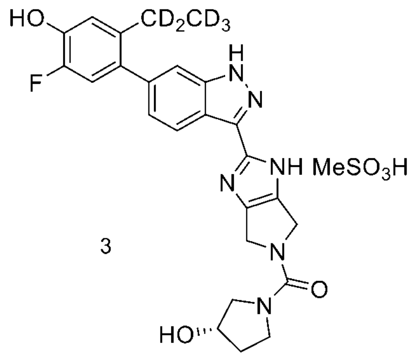

3

**Synthetic Route for Compound 3:**

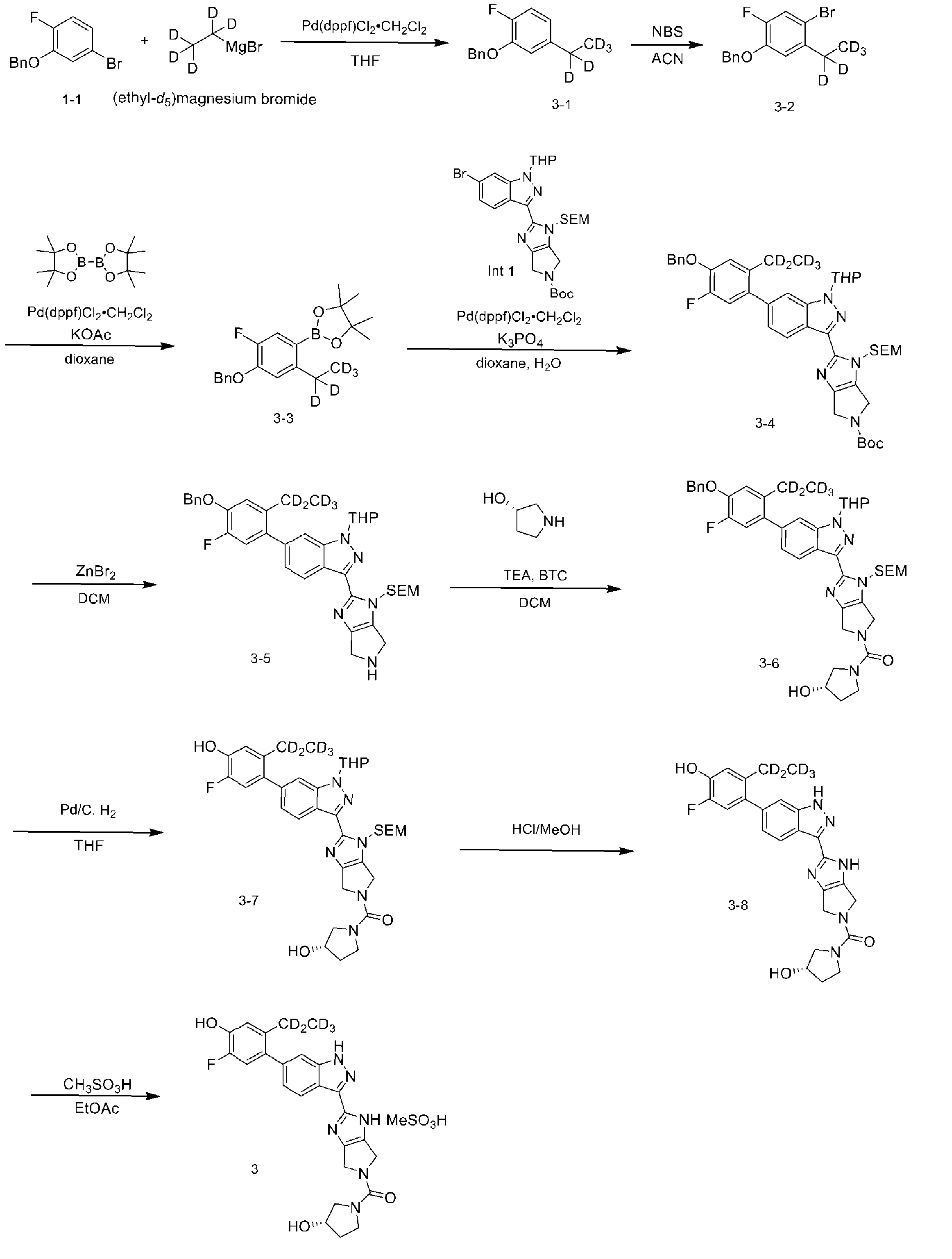


**Synthetic Method:**

**Synthesis of Intermediate 3-1: 2-(Benzyloxy)-4-(ethyl-d5)-1-fluorobenzene**

THF (80.0 mL), compound 1-1 (13.0 g, 1.00 eq), and $Pd(dppf)Cl_2•CH_2Cl_2$ (780 mg) were added to a reaction flask. At 20-30 °C, (ethyl-d5) magnesium bromide (9.59 g, 1.50 eq) was added to the flask. It was allowed to react at 65 °C

for 12 hours with stirring. The reaction mixture was quenched by adding 100 mL of 1 N hydrochloric acid (HCl), then extracted with 100 mL of ethyl acetate. The organic phase was concentrated. The concentrate was dissolved in 200 mL of ethyl acetate and purified by passing through a silica gel pad. Concentration yielded 11.0 g of compound 3-1 as a yellow oil.

$^1H$ NMR: (400 MHz, $CDCl_3$) δ 7.39-7.48 (m, 5H), 6.99-7.03 (m, 1H), 6.85-6.87 (m, 1H), 6.75 (m, 1H), 5.15 (s, 2H)

**Synthesis of Intermediate 3-2: 1-(Benzyloxy)-4-bromo-5-(ethyl-d5)-2-fluorobenzene**

Compound 3-1 (11.0 g, 1.00 eq) and acetonitrile (ACN, 100 mL) were added to a reaction flask. At 20-30 °C, NBS (8.32 g, 1.00 eq) was added to the flask. It was allowed to react at 20-30 °C for 1 hour. The reaction mixture was concentrated, to which 100 mL of water was added, and the resulting mixture was extracted with ethyl acetate (100 mL × 2). The combined organic phases were concentrated under reduced pressure. The concentrate was dissolved in 200 mL of ethyl acetate and purified by passing through a silica gel pad. Concentration yielded 15.0 g of compound 3-2 as a yellow oil.

$^1H$ NMR (400 MHz, $CDCl_3$) δ 7.24-7.35 (m, 5H), 7.16-7.19 (m, 1H), 6.77-6.81 (m, 1H), 5.03 (s, 2H).

**Synthesis of Intermediate 3-3: 2-(4-(Benzyloxy)-2-(ethyl-d5)-5-fluorophenyl)-4,4,5,5-tetramethyl-1,3,2-dioxaborolane**

Compound 3-2 (14.0 g, 1.00 eq), $B_2Pin_2$ (12.1 g, 1.00 eq), $Pd(dppf)Cl_2$•$CH_2Cl_2$ (780 mg, 0.02 eq), and potassium acetate (AcOK, 14.1 g, 3.00 eq) were added to dioxane (150 mL). Under nitrogen protection, it was allowed to react at 110-120 °C for 2 hours. The reaction mixture was filtered, and the filtrate was concentrated. 100 mL of water was added to the concentrate, and then the resulting mixture was extracted with 100 mL of methyl tert-butyl ether (MTBE). The organic phase was concentrated to yield 17.0 g of compound 3-3 as a yellow oil.

$^1H$ NMR (400 MHz, $CDCl_3$): δ 7.34-7.46 (m, 6H), 6.84-6.86 (m, 1H), 5.18 (s, 2H), 1.35 (s, 12H).

**Synthesis of Intermediate 3-4: tert-butyl 2-(6-(4-(Benzyloxy)-2-(ethyl-d5)-5-fluorophenyl)-1-(tetrahydro-2H-pyran-2-yl)-1H-indazol-3-yl)-1-((2-(trimethylsilyl)ethoxy)methyl)-4,6-dihydropyrrolo[3,4-d]imidazole-5(1H)-carboxylate**

Compound 3-3 (12.0 g, 1.11 eq), compound Int 1 (18.5 g, 1.00 eq), $K_3PO_4$ (12.7 g, 2.00 eq), and 44.0 mL of water were added to dioxane (150 mL). Under nitrogen protection, $Pd(dppf)Cl_2$•$CH_2Cl_2$ (244 mg, 0.01 eq) was added to the reaction flask. It did not dissolve completely. It was allowed to react by heating to 70-80 °C for 2 hours. The reaction mixture was concentrated. 250 mL of MTBE was added to the concentrate for extraction. The organic phase was dried over anhydrous sodium sulfate and filtered, and the filtrate was concentrated. The concentrate was loaded onto silica gel and purified by a silica gel pad, eluting first with petroleum ether, then with petroleum ether:ethyl acetate = 1:1. The eluate containing the product was collected and rotary evaporated to yield 14.0 g of compound 3-4 as a brown paste. LC-MS m/z (ESI) $[M+H]^+$ calcd for $C_{43}H_{50}D_5FN_5O_5Si$: 773.4; found: 773.3.

$^1H$ NMR (400 MHz, $CDCl_3$): δ 8.43-8.48 (m, 1H), 7.47-7.51 (m, 2H), 7.40-7.42 (m, 3H), 6.95-7.08 (m, 3H), 6.01-6.04 (m, 1H), 5.87-5.93 (m, 1H), 5.74-5.77 (m, 1H), 5.21 (s, 1H), 4.52-4.65 (m, 4H), 4.02-4.03 (m, 1H), 3.76 (m, 1H), 3.56-3.62 (m, 2H), 2.59 (m, 1H), 2.12-2.19 (m, 2H), 1.71-1.74 (m, 4H), 1.55 (s, 9H), -0.07~-0.05 (s, 9H).

**Synthesis of Intermediate 3-5: 6-(4-(Benzyloxy)-2-(ethyl-d5)-5-fluorophenyl)-1-(tetrahydro-2H-pyran-2-yl)-3-(1-((2-(trimethylsilyl)ethoxy)methyl)-1,4,5,6-tetrahydropyrrolo[3,4-d]imidazol-2-yl)-1H-indazole**

Compound 3-4 (14.0 g, 1.00 eq) was added to DCM (70.0 mL). At 20-30 °C, ZnBr2 (16.3 g, 4.00 eq) was added. After addition, the solution cleared. It was allowed to react at 20-30 °C for 3.5 hours with stirring. To the reaction mixture, 37.8 mL of 28% ammonia water was added, and the mixture was stirred at 20-30 °C for 10 minutes. The mixture emulsified heavily. DCM was removed by rotary evaporation. The residue was extracted with 155 mL of MTBE. The mixture was filtered, and the filtrate was concentrated. 100 mL of water was added to the concentrate, and the resulting mixture was extracted with 100 mL of MTBE. The organic phase was dried over anhydrous sodium sulfate and filtered, and the filtrate was concentrated to yield 10.0 g of compound 3-5 as a brown paste, used directly in the next step.

**Synthesis of Intermediate 3-6: (2-(6-(4-(Benzyloxy)-2-(ethyl-d5)-5-fluorophenyl)-1-(tetrahydro-2H-pyran-2-yl)-1H-indazol-3-yl)-1-((2-(trimethylsilyl)ethoxy)methyl)-4,6-dihydropyrrolo[3,4-d]imidazol-5(1H)-yl) ((S)-3-hydroxypyrrolidin-1-yl) ketone**

Triphosgene (1.80 g, 0.41 eq) was added to DCM (20.0 mL) and cooled to 0-5 °C. Under nitrogen protection, a solution of compound 3-5 (10.0 g, 1.00 eq) in 80.0 mL of DCM was added dropwise to the above reaction mixture, controlling the temperature within 0-5 °C. After addition, it was allowed to react for 10 minutes with stirring. Maintaining the

temperature at 0-5 °C, triethylamine (TEA, 12.0 g, 8.00 eq) was added dropwise. After addition, it was allowed to react for 10 minutes with stirring. (S)-Pyrrolidin-3-ol hydrochloride (2.75 g, 1.50 eq) was added to the reaction mixture in one portion and it was allowed to react by heating raised to 20-30 °C with stirring for 1 hour. 100 mL of water was added to quench and dilute the reaction. The mixture was stirred at 20-30 °C for 30 minutes and then allowed to stand for phase separation. The organic phase was dried over anhydrous sodium sulfate and rotary evaporated to dryness. The residue was filtered through a silica gel pad, and the eluate was evaporated to yield 6.00 g of compound 3-6 as brown foam. LC-MS m/z (ESI) $[M+H]^+$ calcd for $C_{43}H_{49}D_5FN_6O_5Si$: 786.4; found: 786.2.

**Synthesis of Intermediate 3-7: (2-(6-(2-(Ethyl-d5)-5-fluoro-4-hydroxyphenyl)-1-(tetrahydro-2H-pyran-2-yl)-1H-indazol-3-yl)-1-((2-(trimethylsilyl)ethoxy)methyl)-4,6-dihydropyrrolo[3,4-d]imidazol-5(1H)-yl) ((S)-3-hydroxypyrrolidin-1-yl) ketone**

Compound 3-6 (6.00 g, 1.00 eq) was added to THF (60.0 mL). Under nitrogen protection, wet palladium on carbon (600 mg) was added. The atmosphere was replaced with hydrogen three times, and then it was allowed to react at 20-30 °C for 3 hours under a hydrogen atmosphere. After completion of reaction, the mixture was filtered through a Celite pad. The filter cake was washed with 100 mL of THF. The filtrates were combined, to which another 600 mg of wet palladium on carbon was added. The atmosphere was replaced with hydrogen three times, and then it was allowed to react at 20-30 °C for 7.5 hours under a hydrogen atmosphere. After completion of reaction, the mixture was filtered through a Celite pad. The filter cake was washed with 100 mL of THF. The filtrates were combined, to which another 600 mg of wet palladium on carbon was added. The atmosphere was replaced with hydrogen three times, and then it was allowed to react at 20-30 °C for 4 hours under a hydrogen atmosphere. The reaction was stopped, and the mixture was filtered through a Celite pad. The filtrate was rotary evaporated to dryness. 12.0 mL ofMTBE was added to the residue and the resuling mixture was concentrated to yield 5.00 g of crude compound 3-7, used directly in the next step.

**Synthesis of Intermediate 3-8: (S)-(2-(6-(2-(Ethyl-d5)-5-fluoro-4-hydroxyphenyl)-1H-indazol-3-yl)-4,6-dihydropyrrolo[3,4-d]imidazol-5(1H)-yl)(3-hydroxypyrrolidin-1-yl) ketone**

40.0 g of methanol was saturated with hydrogen chloride gas (27.0 g), giving an HCl/MeOH solution with a mass fraction of 40.3%. Compound 3-7 (5.00 g, 1.00 eq) was added to the HCl/MeOH solution and it was allowed to react at 20-30 °C for 17.5 hours. The reaction mixture was filtered. The filter cake was suspended in 50.0 mL of ethanol. At 20-30 °C, 6.00 mL of ammonia water (28%) was added dropwise to liberate the product. During the process, the solution first cleared and then precipitated solid again. Stirring was continued at 20-30 °C for 30 minutes. The suspension was filtered, and the filter cake was evaporated to dryness to yield 2.00 g of off-white solid. 60.0 mL of methanol was added, and the mixture was slurried at 60-65 °C for 5.5 hours. After cooling to 20-30 °C, it was filtered, and the filter cake was evaporated to dryness to yield 1.50 g of compound 3-8 as an off-white solid. LC-MS m/z (ESI) $[M+H]^+$ calcd for $C_{25}H_{21}D_5FN_6O_3$: 482.2; found: 482.1.

**Synthesis of Compound 3: (S)-(2-(6-(2-(Ethyl-d5)-5-fluoro-4-hydroxyphenyl)-1H-indazol-3-yl)-4,6-dihydropyrrolo[3,4-d]imidazol-5(1H)-yl)(3-**hydroxypyrrolidin-1-yl) ketone methanesulfonate

Compound 3-8 (1.50 g, 1.00 eq) was added to ethyl acetate (30.0 mL). Under nitrogen protection, $CH_3SO_3H$ (360 mg, 1.20 eq) was added dropwise at 10-20 °C. After addition, it was allowed to react at 10-20 °C for 3 hours. The reaction mixture was filtered, and the filter cake was washed with 2.00 mL of ethyl acetate and evaporated to dryness to yield 1.60 g of compound 3 as an off-white solid.

$_1$H NMR: (400 MHz, DMSO) δ 14.00 (s, 1H), 8.25-8.27 (m, 1H), 7.55 (s, 1H), 7.27-7.29 (m, 1H), 7.03-7.06 (m, 1H), 6.92-6.94 (m, 1H), 4.75-4.78 (m, 2H), 4.58 (m, 2H), 4.29 (s, 1H), 3.52-3.55 (m, 3H), 3.41-3.43 (m, 2H), 3.25 (m, 1H), 2.32-2.34 (m, 3H), 1.80-1.91 (m, 2H).

**Example 4: 2-Fluoro-5-(methyl-d3)-4-(3-(5-methyl-1,4,5,6-tetrahydropyrrolo[3,4-d]imidazol-2-yl)-1H-indazol-6-yl)phenol (Compound 12)**

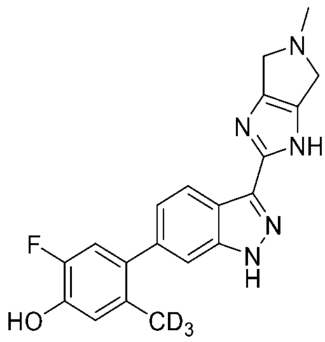

12

**Synthetic Route for Compound 12:**

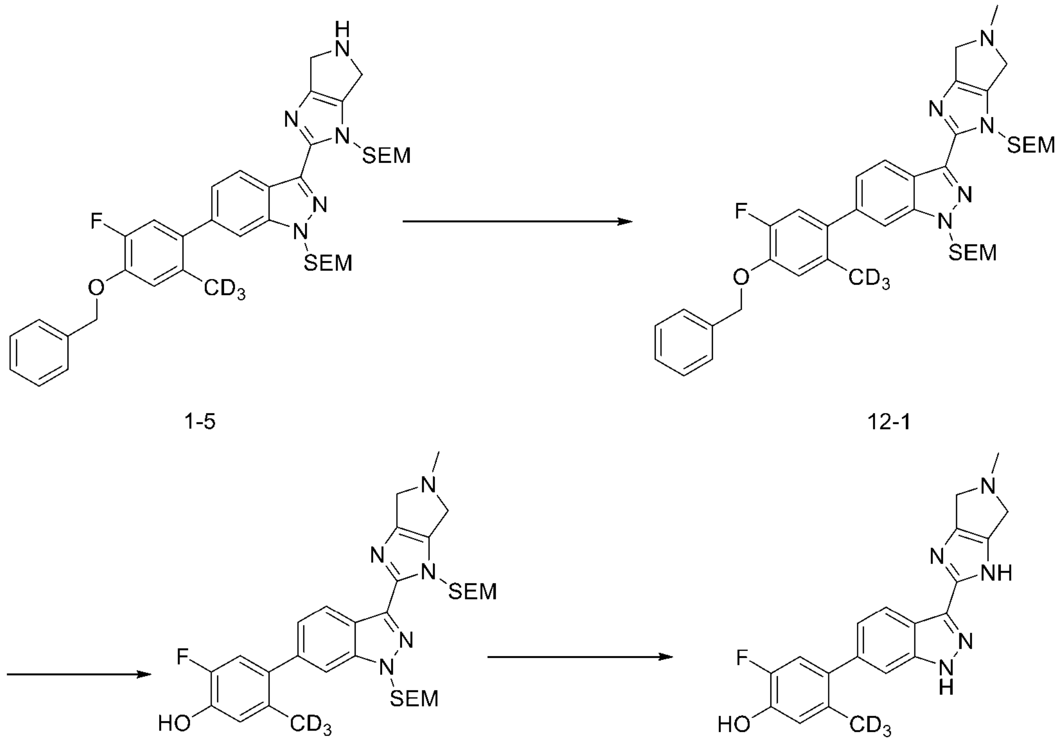

1-5

12-1

12-2

12

**Synthetic Method:**

**Synthesis of Intermediate 12-1: 6-(4-(Benzyloxy)-5-fluoro-2-(methyl-d3)phenyl)-3-(5-methyl-1-((2-(trimethylsilyl)ethoxy)methyl)-1,4,5,6-tetrahydropyrrolo[3,4-d]imidazol-2-yl)-1-((2-trimethylsilyl)ethoxymethyl)-1H-indazole**

Intermediate 1-5 (100.0 mg, 0.14 mmol) was dissolved in 10 ml of DCM, to which formaldehyde solution (40%) (21 mg, 0.24 mmol) was added at room temperature and the resulting mixture was stirred for 0.5 hours. Then sodium triacetoxyborohydride (89 mg, 0.42 mmol) was added and the resulting mixture was stirred at room temperature for 2 hours. After completion, water was added to quench the reaction. The mixture was extracted with DCM. The organic phase was washed with saturated brine, dried over anhydrous sodium sulfate, concentrated, and purified by preparative TLC to yield 80.0 mg of intermediate 12-1, in a yield of 78.4%.

**Synthesis of Intermediate 12-2: 2-Fluoro-5-(methyl-d3)-4-(3-(5-methyl-1-((2-(trimethylsilyl)ethoxy) methyl)-1,4,5,6-tetrahydropyrrolo[3,4-d]imidazol-2-yl)-1-((2-(trimethylsilyl)ethoxy)methyl)-1H-indazol-6-yl)phenol**

Intermediate 12-1 (20.0 mg, 0.03 mmol) was dissolved in 3 ml of methanol, to which 4 mg of palladium on carbon was added, and the atmosphere was replaced with hydrogen. It was allowed to react at 40°C for 2 hours. After completion, the mixture was filtered, and the filtrate was concentrated to yield 10.0 mg of intermediate 12-2, in a yield of 57%.

**Synthesis of Compound 12: 2-Fluoro-5-(methyl-d3)-4-(3-(5-methyl-1,4,5,6-tetrahydropyrrolo[3,4-d]imidazol-2-yl)-1H-indazol-6-yl)phenol**

Intermediate 12-2 (10.0 mg, 0.02 mmol) was dissolved in 4 ml of methanol, to which 2 ml of concentrated hydrochloric acid was added. It was allowed to react at 50°C for 6 hours. The mixture was concentrated, and the concentrate was dissolved in 5 ml of methanol. To the mixture, 0.5 ml of ammonia water was added. The resulting mixture was concentrated and purified by reverse-phase silica gel column chromatography to yield 2.0 mg of the final product, in a yield of 34.5%. LC-MS m/z (ESI) $[M+H]^+$ calcd for $C_{20}H_{16}D_3FN_5O$: 367.2; found: 367.2.

**Table 1:** The following compounds were prepared according to the synthetic route described in Example 1 using suitable starting materials.

| Compounds | LC-MS calcd $(M+1)^+$ | LC-MS found $(M+1)^+$ |
|---|---|---|
| 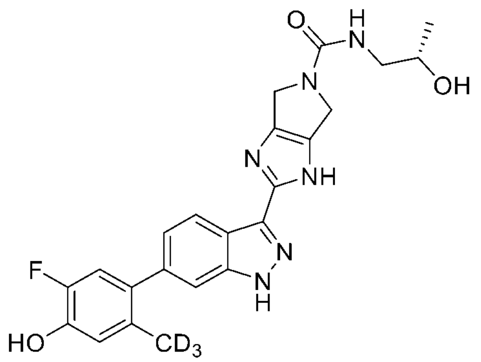 4 (S)-2-(6-(5-Fluoro-4-hydroxy-2-(methyl-d3)phenyl)-1H-indazol-3-yl)-N-(2-hydroxypropyl)-4,6-dihydropyrrolo[3,4-d]imidazole-5(1H)-carboxamide | 454.2 | 454.3 |
| 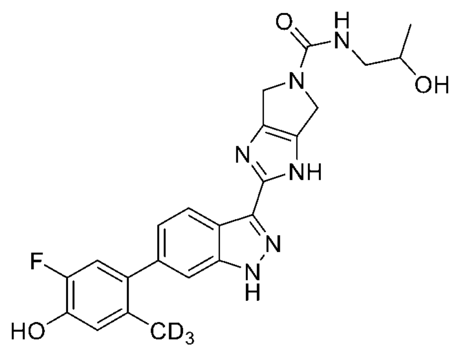 5 2-(6-(5-Fluoro-4-hydroxy-2-(methyl-d3)phenyl)-1H-indazol-3-yl)-N-(2-hydroxypropyl)-4,6-dihydropyrrolo[3,4-d]imidazole-5(1H)-carboxamide | 454.2 | 454.2 |

(continued)

| Compounds | LC-MS calcd (M+1)+ | LC-MS found (M+1)+ |
|---|---|---|
| 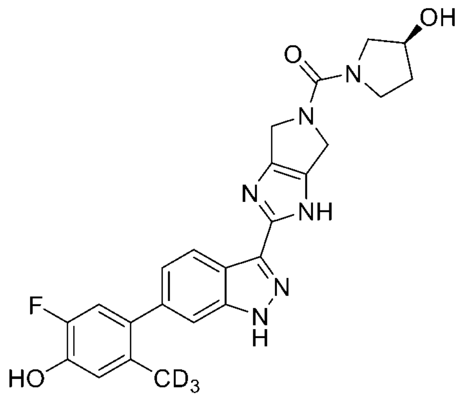 6 (S)-(2-(6-(5-Fluoro-4-hydroxy-2-(methyl-d3)phenyl)-1H-indazol-3-yl)-4,6-dihydropyrrolo [3,4-d]imidazole-5(1H)-yl) (3-hydroxypyrrolidin-1-yl) ketone | 466.2 | 466.2 |
| 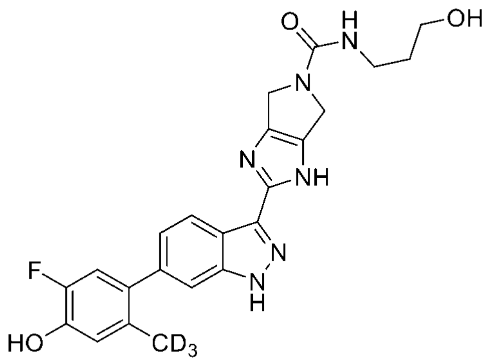 7 2-(6-(5-Fluoro-4-hydroxy-2-(methyl-d3)phenyl)-1H-indazol-3-yl)-N-(3-hydroxypropyl)-4,6-dihydropyrrolo[3,4-d]imidazole-5(1H)-carboxamide | 454.2 | 454.3 |
| 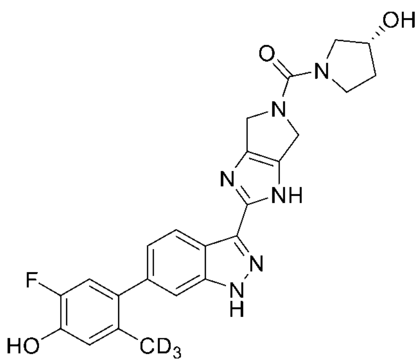 8 (R)-(2-(6-(5-Fluoro-4-hydroxy-2-(methyl-d3)phenyl)-1H-indazol-3-yl)-4,6-dihydropyrrolo [3,4-d]imidazole-5(1H)-yl) (3-hydroxypyrrolidin-1-yl) ketone | 466.2 | 466.2 |

(continued)

| Compounds | LC-MS calcd $(M+1)^+$ | LC-MS found $(M+1)^+$ |
|---|---|---|
| 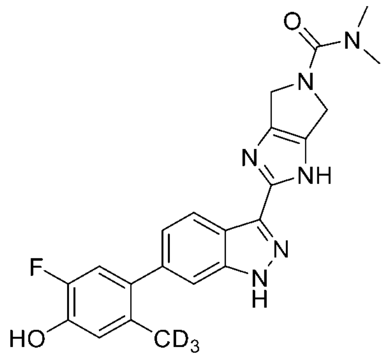 9 2-(6-(5-Fluoro-4-hydroxy-2-(methyl-d3)phenyl)-1H-indazol-3-yl)-N,N-dimethyl-4,6-dihydropyrrolo[3,4-d]imidazole-5(1H)-carboxamide | 424.2 | 424.1 |
| 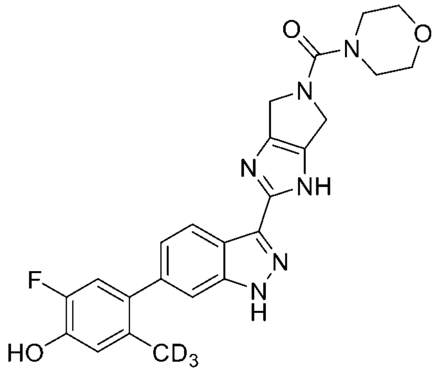 10 (2-(6-(5-Fluoro-4-hydroxy-2-(methyl-d3)phenyl)-1H-indazol-3-yl)-4,6-dihydropyrrolo[3,4-d]imidazole-5(1H)-yl) (morpholino) ketone | 466.2 | 466.2 |
| 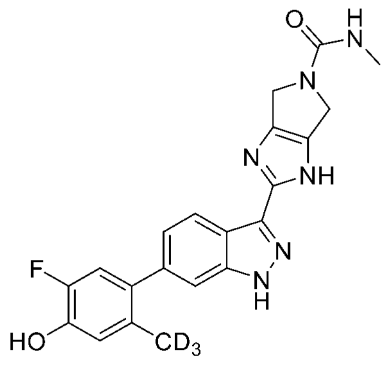 13 2-(6-(5-Fluoro-4-hydroxy-2-(methyl-d3)phenyl)-1H-indazol-3-yl)-N-methyl-4,6-dihydropyrrolo[3,4-d]imidazole-5(1H)-carboxamide | 410.0 | 410.0 |

(continued)

| Compounds | LC-MS calcd (M+1)$^+$ | LC-MS found (M+1)$^+$ |
|---|---|---|
| 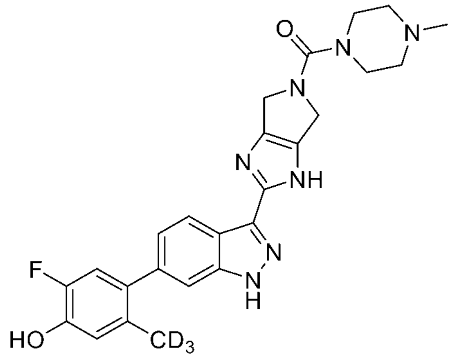<br>14<br>(2-(6-(5-Fluoro-4-hydroxy-2-(methyl-d3)phenyl)-1H-indazol-3-yl)-4,6-dihydropyrrolo[3,4-d]imidazole-5(1H)-yl)(4-methylpiperazin-1-yl)ketone | 479.1 | 479.1 |
| 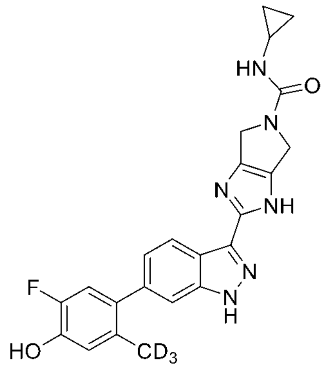<br>15<br>N-Cyclopropyl-2-(6-(5-fluoro-4-hydroxy-2-(methyl-d3)phenyl)-1H-indazol-3-yl)-4,6-dihydropyrrolo[3,4-d]imidazole-5(1H)-carboxamide | 436.2 | 436.2 |
| 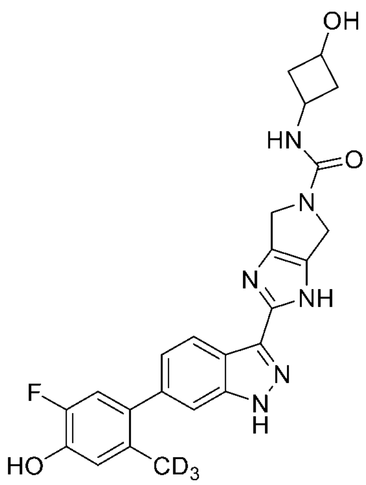<br>16<br>2-(6-(5-Fluoro-4-hydroxy-2-(methyl-d3)phenyl)-1H-indazol-3-yl)-N-(3-hydroxycyclobutyl)-4,6-dihydropyrrolo[3,4-d]imidazole-5(1H)-carboxamide | 466.2 | 466.2 |

(continued)

| Compounds | LC-MS calcd $(M+1)^+$ | LC-MS found $(M+1)^+$ |
|---|---|---|
| 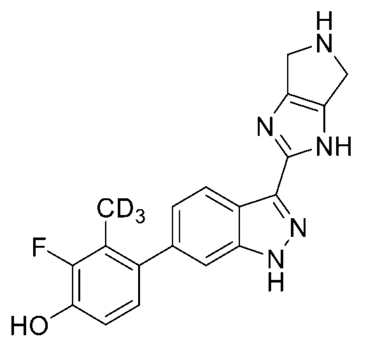 17 2-Fluoro-3-(methyl-d3)-4-(3-(1,4,5,6-tetrahydropyrrolo[3,4-d]imidazol-2-yl)-1H-indazol-6-yl)phenol | 353.2 | 353.2 |
| 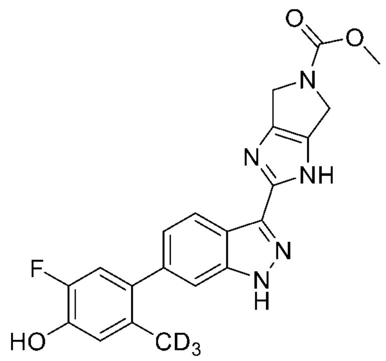 18 Methyl 2-(6-(5-fluoro-4-hydroxy-2-(methyl-d3)phenyl)-1H-indazol-3-yl)-4,6-dihydropyrrolo[3,4-d]imidazole-5(1H)-carboxylate | 411.0 | 411.0 |
| 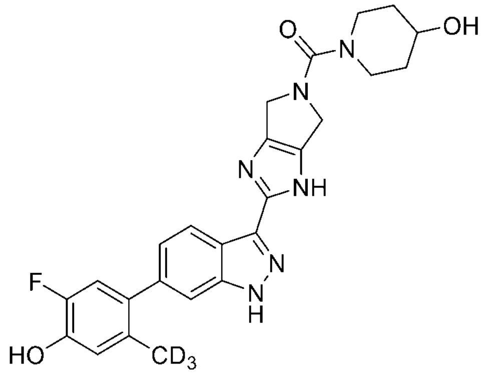 19 (2-(6-(5-Fluoro-4-hydroxy-2-(methyl-d3)phenyl)-1H-indazol-3-yl)-4,6-dihydropyrrolo[3,4-d]imidazole-5(1H)-yl)(4-hydroxypiperidin-1-yl)ketone | 480.2 | 480.1 |

**Table 2:** The following compounds were prepared according to the synthetic route described in Example 2 using suitable starting materials.

| Compounds | LC-MS calcd $(M+1)^+$ | LC-MS found $(M+1)^+$ |
|---|---|---|
| 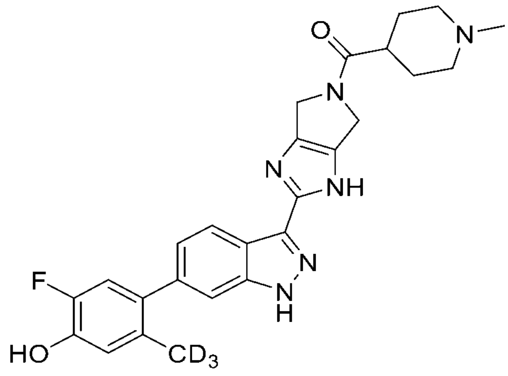<br>20<br>(2-(6-(5-Fluoro-4-hydroxy-2-(methyl-d3)phenyl)-1H-indazol-3-yl)-4,6-dihydropyrrolo[3,4-d]imidazol-5(1H)-yl) (1-methylpiperidin-4-yl) ketone | 478.2 | 478.2 |
| 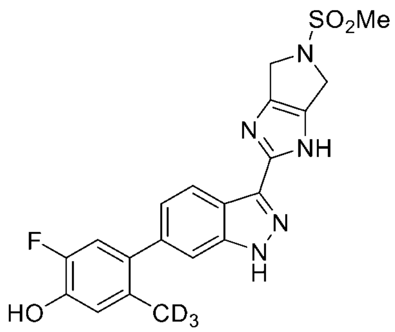<br>21<br>2-Fluoro-5-(methyl-d3)-4-(3-(5-(methylsulfonyl)-1,4,5,6-tetrahydropyrrolo[3,4-d]imidazol-2-yl)-1H-indazol-6-yl)phenol | 431.1 | 431.1 |
| 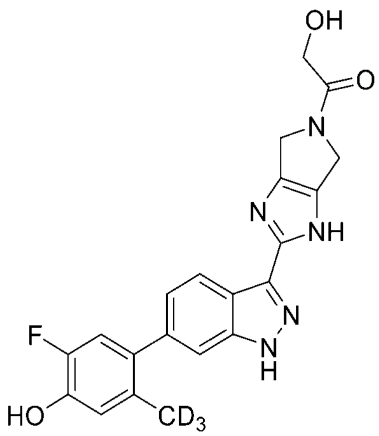<br>22<br>1-(2-(6-(5-Fluoro-4-hydroxy-2-(methyl-d3)phenyl)-1H-indazol-3-yl)-4,6-dihydropyrrolo [3,4-d]imidazol-5(1H)-yl)-2-hydroxyethan-1-one | 411.2 | 411.2 |

(continued)

| Compounds | LC-MS calcd (M+1)+ | LC-MS found (M+1)+ |
|---|---|---|
| 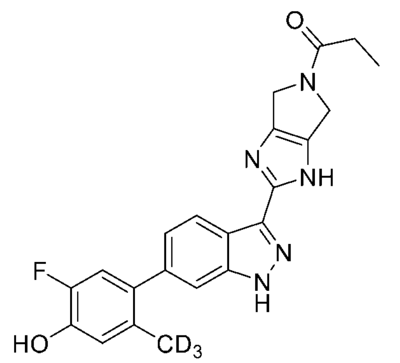<br>23<br>1-(2-(6-(5-Fluoro-4-hydroxy-2-(methyl-d3)phenyl)-1H-indazol-3-yl)-4,6-dihydropyrrolo [3,4-d]imidazol-5(1H)-yl)propan-1-one | 409.2 | 409.2 |
| 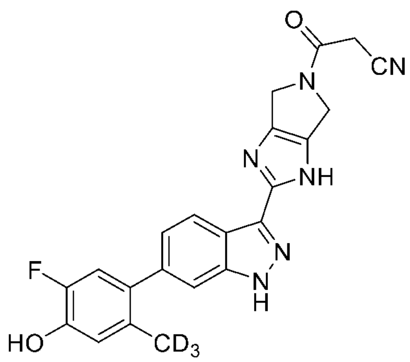<br>24<br>3-(2-(6-(5-Fluoro-4-hydroxy-2-(methyl-d3)phenyl)-1H-indazol-3-yl)-4,6-dihydropyrrolo [3,4-d]imidazol-5(1H)-yl)-3-oxopropanenitrile | 420.1 | 420.1 |

**Table 3:** The following compounds were prepared according to the synthetic route described in Example 4 using suitable starting materials.

| Compounds | LC-MS calcd $(M+1)^+$ | LC-MS found $(M+1)^+$ |
|---|---|---|
| 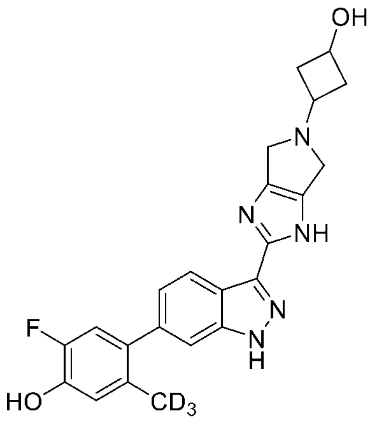 25 2-Fluoro-4-(3-(5-(3-hydroxycyclobutyl)-1,4,5,6-tetrahydropyrrolo[3,4-d]imidazol-2-yl)-1H-indazol-6-yl)-5-(methyl-d3)phenol | 423.2 | 423.2 |
| 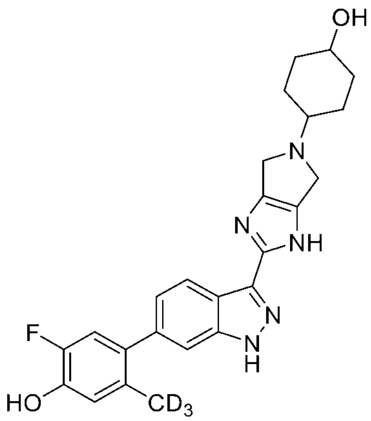 26 2-Fluoro-4-(3-(5-(4-hydroxycyclohexyl)-1,4,5,6-tetrahydropyrrolo[3,4-d]imidazol-2-yl)-1H-indazol-6-yl)-5-(methyl-d3)phenol | 451.2 | 451.2 |
| 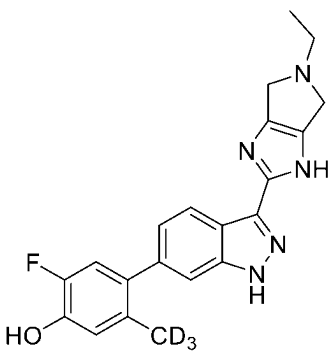 27 4-(3-(5-Ethyl-1,4,5,6-tetrahydropyrrolo[3,4-d]imidazol-2-yl)-1H-indazol-6-yl)-2-fluoro-5-(methyl-d3)phenol | 381.2 | 381.2 |

(continued)

| Compounds | LC-MS calcd (M+1)+ | LC-MS found (M+1)+ |
|---|---|---|
| 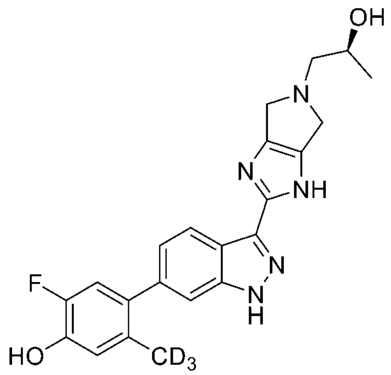 28 (S)-2-Fluoro-4-(3-(5-(2-hydroxypropyl)-1,4,5,6-tetrahydropyrrolo[3,4-d]imidazol-2-yl)-1H-indazol-6-yl)-5-(methyl-d3)phenol | 411.2 | 411.0 |
| 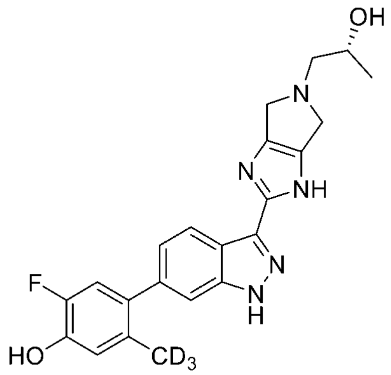 29 (R)-2-Fluoro-4-(3-(5-(2-hydroxypropyl)-1,4,5,6-tetrahydropyrrolo[3,4-d]imidazol-2-yl)-1H-indazol-6-yl)-5-(methyl-d3)phenol | 411.2 | 411.1 |
| 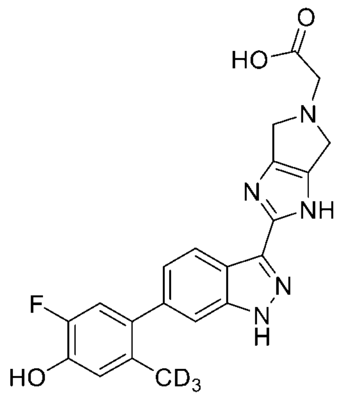 30 2-(2-(6-(5-Fluoro-4-hydroxy-2-(methyl-d3)phenyl)-1H-indazol-3-yl)-4,6-dihydropyrrolo [3,4-d]imidazol-5(1H)-yl)acetic acid | 411.1 | 411.1 |

**Table 4:** The following compounds were prepared according to the synthetic route described in Example 3 using suitable starting materials.

| Compounds | LC-MS calcd $(M+1)^+$ | LC-MS found $(M+1)^+$ |
|---|---|---|
| 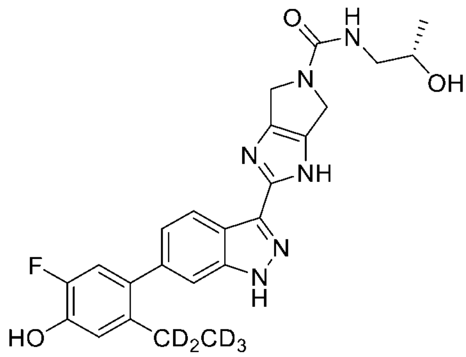 31 (S)-2-(6-(2-(Ethyl-d5)-5-fluoro-4-hydroxyphenyl)-1H-indazol-3-yl)-N-(2-hydroxypropyl)-4,6-dihydropyrrolo[3,4-d]imidazole-5(1H)-carboxamide | 470.2 | 470.3 |
| 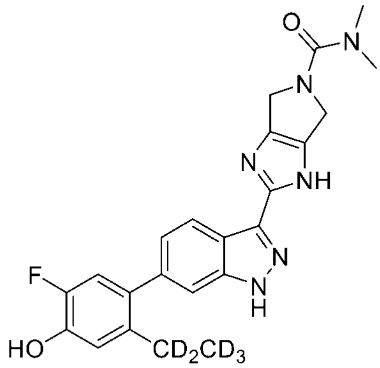 32 2-(6-(2-(Ethyl-d5)-5-fluoro-4-hydroxyphenyl)-1H-indazol-3-yl)-N,N-dimethyl-4,6-dihydropyrrolo[3,4-d]imidazole-5(1H)-carboxamide | 440.2 | 440.3 |
| 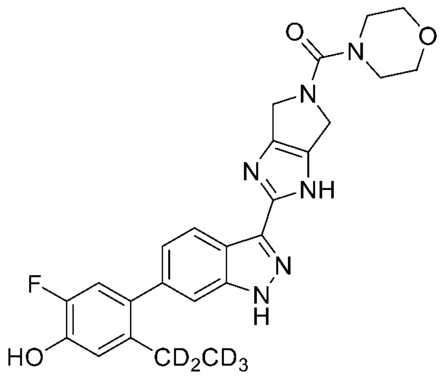 33 (2-(6-(2-(Ethyl-d5)-5-fluoro-4-hydroxyphenyl)-1H-indazol-3-yl)-4,6-dihydropyrrolo[3,4-d]imidazole-5(1H)-yl)(morpholino)ketone | 482.2 | 482.3 |

(continued)

| Compounds | LC-MS calcd $(M+1)^+$ | LC-MS found $(M+1)^+$ |
|---|---|---|
| 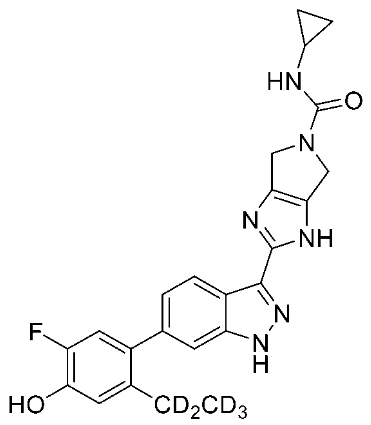<br>34<br>N-Cyclopropyl-2-(6-(2-(Ethyl-d5)-5-fluoro-4-hydroxyphenyl)-1H-indazol-3-yl)-4,6-dihydropyrrolo[3,4-d]imidazole-5(1H)-carboxamide | 452.2 | 452.3 |
| 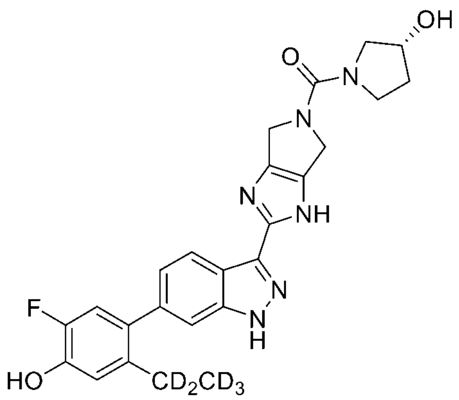<br>35<br>(R)-(2-(6-(2-(Ethyl-d5)-5-fluoro-4-hydroxyphenyl)-1H-indazol-3-yl)-4,6-dihydropyrrolo[3,4-d]imidazole-5(1H)-yl)(3-hydroxypyrrolidin-1-yl)ketone | 482.2 | 482.3 |
| 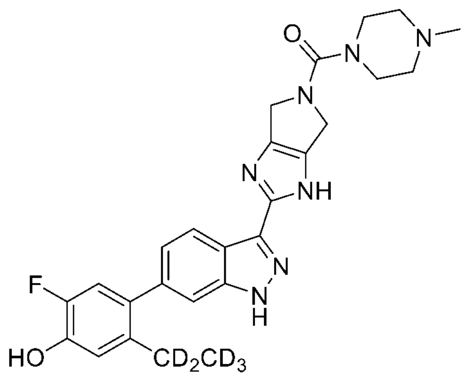<br>36<br>(2-(6-(2-(Ethyl-d5)-5-fluoro-4-hydroxyphenyl)-1H-indazol-3-yl)-4,6-dihydropyrrolo[3,4-d]imidazole-5(1H)-yl)(4-methylpiperazin-1-yl)ketone | 495.2 | 495.2 |

(continued)

| Compounds | LC-MS calcd $(M+1)^+$ | LC-MS found $(M+1)^+$ |
|---|---|---|
| 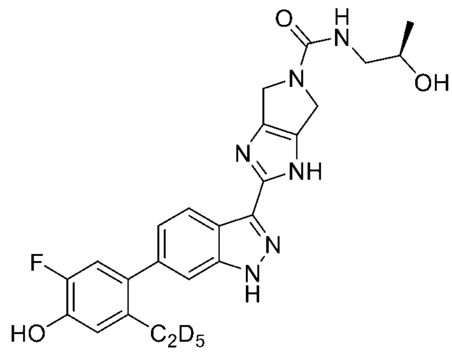 37 (R)-2-(6-(2-(Ethyl-d5)-5-fluoro-4-hydroxyphenyl)-1H-indazol-3-yl)-N-(2-hydroxypropyl)-4,6-dihydropyrrolo[3,4-d]imidazole-5(1H)-carboxamide | 470.2 | 470.1 |

## Example 11: Evaluation of Pharmacological Activity

### 1. Experimental principle

A drug screening system based on kinases JAK1, JAK2, JAK3, and TYK2 was used to detect the inhibitory ability of small molecule compounds on kinase activity. A kinase undergoes an enzymatic reaction with its substrates IRS1, IGF1Rtide, and Poly (4:1 Glu, Tyr), consuming ATP to produce ADP, wherein the ADP-Glo reagent and luminescence method were used to detect the amount of the product to reflect the activity of the kinase.

### 2. Experimental approach

### 2.1 Experimental materials and instruments

| Name | Source/Supplier | Catalogue No. |
|---|---|---|
| Hepes | Thermo Fisher | 15630080 |
| Brij35 | Millipore | 1018940100 |
| EGTA | Sigma | E3889 |
| ADP-Glo Kinase Assay | Promega | V9103 |
| MgCl2 | Sigma | M1028 |
| DTT | MCE | HY-15917 |
| DMSO | Sigma | D4540 |
| ATP | Promega | V915B |
| JAK1 | Carna | 08-144 |
| JAK2 | Carna | 08-045 |
| JAK3 | Carna | 08-046 |
| TYK2 | ThermoFisher | PR8440C |
| IGF1R tide | GenScript | PE0403 |
| Poly tide | SingalChem | P61-58 |

(continued)

| Name | Source/Supplier | Catalogue No. |
|---|---|---|
| IRS1 tide | GenScript | PE0335 |
| Tofacitinib | MCE | HY-40354 |
| 384-well plate, white, low-volume round-bottom | Greiner | 784075 |
| 96-well polypropylene plate | Nunc | 249944 |
| Centrifuge | Cence | TDZ5-WS |
| ECHO® 655 SYSTEM | Beckman | 655 |
| 384-well plate, PP2.0, microplate, Echo Qualified | Beckman | PP-0200 |
| Plate reader | BMG | PHERAstar FSX |

2. Kinase Information

2.1 Kinase Reaction Buffer:

| Name | Working concentration |
|---|---|
| Hepes | 50 mM |
| $MgCl_2$ | 10 mM |
| Brij35 | 0.01% |
| EGTA | 1 mM |
| DTT | 2 mM |
| $ddH_2O$ | / |

2.2 Kinase Reaction Conditions:

| Kinase | Kinase | | Substrate | | ATP |
|---|---|---|---|---|---|
| | **Stock concentration (nM)** | **Working concentration (nM)** | **Name** | **Working concentration (mg/mL)** | **Working concentration (μM)** |
| JAK1 | 13242 | 25.00 | IRS1 tide | 0.05 | 20 |
| JAK2 | 4256 | 0.50 | IGF1R tide | 0.5 | 1 |
| JAK3 | 3024 | 2.00 | Poly tide | 0.03 | 10 |
| TYK2 | 6977 | 2.50 | IRS1 tide | 0.01 | 20 |

2. Experimental approach

2.3 Compound Screening Procedure:

Prepare 2× ATP/substrate solution and 2× kinase solution using the kinase reaction buffer.

For JAK1/2/3 inhibition activity testing, Tofacitinib was used at a starting concentration of 1 μM with 1:4 serial dilution. For TYK2 inhibition activity testing, the starting concentration was 10 μM with 1:4 serial dilution. Test compounds were used at a starting concentration of 0.5 μM with 1:4 serial dilution.

Transfer 40 nL of compound dilution to a 384-well assay plate using Echo 655. After centrifuge, 2 μL of 2× kinase

solution was added to the 384-well plate, which was centrifuged at 1000 rpm for 1 minute, and then incubated at 25 °C for 10 minutes.

Add 2 μL of 2× substrate and ATP solution to the 384-well plate, which was centrifuged at 1000 rpm for 1 minute, and incubated at 25 °C for 60 minutes.

Transfer 4 μL of ADP-Glo reagent to the 384-well plate, which was centrifuged at 1000 rpm for 1 minute, and incubated at 25 °C for 40 minutes.

Transfer 8 μL of the test solution to the 384-well plate, which was centrifuged at 1000 rpm for 1 minute, and incubated at 25 °C for 40 minutes.

Read luminescence signals using a multimode microplate reader.

**3. Data Analysis**

3.1 Inhibition rate was calculated as follows:

% Inhibition = 100% - [(Compound - Positive Control) / (Negative Control - Positive Control)] × 100%

Negative Control: DMSO

Positive Control: Tofacitinib

3.2 Calculate $IC_{50}$ and plot the compound inhibition curve:

The following nonlinear regression equation was used to determine the compound's $IC_{50}$ (half-maximal inhibitory concentration). Data analysis was performed using GraphPad Prism 7.0 software.

$$Y = Bottom + (Top - Bottom) / [1 + 10^{\wedge}((LogIC_{50} - X) \times Hill\ Slope)]$$

X: Logarithm of compound concentration; and Y: Inhibition rate (% Inhibition)

**4. Summary of Compound Test Results**

| Compounds to be tested | $IC_{50}$ (nM) | | | |
|---|---|---|---|---|
| | JAK1 | JAK2 | JAK3 | TYK2 |
| Tofacitinib | 1.3 | 0.74 | 0.98 | 43.5 |
| 1 | 0.096 | 0.013 | 0.20 | 0.80 |
| 2 | 0.19 | 0.017 | 0.28 | 1.2 |
| 3 | 0.0072 | 0.015 | 0.044 | 0.022 |
| 4 | 0.10 | 0.015 | 0.26 | 1.0 |
| 5 | 0.20 | 0.010 | 0.30 | 1.6 |
| 6 | 0.086 | 0.013 | 0.19 | 0.32 |
| 7 | 0.070 | 0.014 | 0.25 | 0.53 |
| 8 | 0.045 | 0.004 | 0.16 | 0.19 |
| 9 | 0.071 | 0.008 | 0.32 | 0.43 |
| 10 | 0.092 | 0.0097 | 0.30 | 0.37 |
| 12 | 1.80 | N/A | N/A | N/A |
| 13 | 0.077 | 0.012 | 0.22 | 0.43 |
| 14 | 0.042 | 0.0096 | 0.13 | 0.17 |

(continued)

| Compounds to be tested | $IC_{50}$ (nM) | | | |
|---|---|---|---|---|
| | JAK1 | JAK2 | JAK3 | TYK2 |
| 15 | 0.11 | 0.17 | 0.32 | 1.1 |
| 16 | 0.14 | 0.24 | 0.48 | 1.7 |
| 17 | 2.3 | 6.8 | 81 | 359 |
| 18 | N/A | 0.61 | N/A | N/A |
| 19 | 0.032 | 0.0050 | 0.18 | 0.30 |
| 20 | 0.81 | 0.12 | 2.4 | 5.6 |
| 21 | 0.98 | 0.22 | 2.0 | 2.7 |
| 23 | 0.46 | 0.020 | 0.47 | 1.4 |
| 24 | 0.45 | 0.24 | 0.72 | 3.1 |
| 25 | 0.27 | 0.54 | 0.92 | 5.19 |
| 26 | 0.29 | 0.46 | 1.1 | 5.1 |
| 27 | 2.0 | N/A | N/A | N/A |
| 28 | 0.43 | 0.050 | 1.0 | 6.4 |
| 29 | 0.60 | 0.083 | 0.75 | 4.7 |
| 30 | 0.19 | N/A | N/A | N/A |
| 31 | 0.022 | 0.022 | 0.19 | 0.48 |
| 32 | 0.025 | 0.032 | 0.20 | 0.30 |
| 33 | 0.017 | 0.019 | 0.12 | 0.12 |
| 34 | 0.043 | 0.036 | 0.19 | 0.77 |
| 35 | 0.0075 | 0.0088 | 0.095 | 0.085 |
| 36 | 0.0082 | 0.0099 | 0.065 | 0.066 |

The above experimental results show that all compounds of the present disclosure tested in assay can inhibit JAK1, JAK2, JAK3, and TYK2 at very low concentrations and the inhibitory activities of these compounds are much higher than that of Tofacitinib. Compared to the corresponding non-deuterated compounds, the compounds of the present invention further exhibited surprisingly and significantly higher inhibitory activity, which was difficult to anticipate prior to the present disclosure.

Although specific embodiments of the present disclosure have been illustrated and described, it does not mean that these embodiments illustrate and describe all possible implementation forms of the present disclosure. More precisely, the language used in this specification are only descriptive words and not restrictive. It will be obvious to those skilled in the art that various kinds of changes and modifications can be made without departing from the general scope of the present disclosure. Therefore, the appended claims are intended to include all these changes and modifications within the scope of the present disclosure.

## Claims

**1.** A compound of Formula (I) as a JAK inhibitor

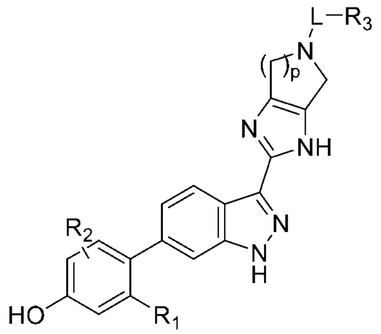

(I)

or an optical isomer thereof, a geometric isomer thereof, a tautomer thereof or a mixture of various isomers, or a pharmaceutically acceptable salt thereof, or a prodrug thereof, or a metabolite thereof,
wherein
L is C=O, O=S=O, $CH_2$ or a covalent bond; and
$R_1$ is -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, or 7-11 membered bicyclic heteroaryl, wherein at least one H atom on $R_1$ is replaced by a D atom; and
$R_3$ is H, -N($R_{17}$)($R_{18}$), $C_{1-6}$ alkoxy, -$SR_{12}$, -$OR_{12}$, -CN, halogen, -$NO_2$, -$SF_5$, -S-$C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, or 5-11 membered bicyclic heteroalkyl, and $R_{13}$ is substituted with 0, 1, 2, 3, or 4 $R_a$(s); in which $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, and 5-11 membered bicyclic heteroalkyl, and are optionally substituted by one or more substituents each independently selected from the group consisting of -OH, -CN, -SH, halogen, -$NO_2$, -$SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl,-N($R_9$)($R_{10}$), -N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(=O)$_2$-N($R_9$)($R_{10}$), -$SR_{12}$, and -$OR_{12}$, wherein the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted by 1, 2, or 3 substituents each independently selected from the group consisting of: halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$),-C(=O)-$OR_{12}$, -C(=O)-H, -C(=O)-$R_{12}$, -C(=O)-N($R_9$)($R_{10}$), -N($R_{11}$)(S(=O)$_2R_{12}$), -S(=O)$_2$-N($R_9$)($R_{10}$), -$SR_{12}$, and -$OR_{12}$; or $R_{17}$, $R_{18}$, and the nitrogen atom to which they are attached together form a 3-14 membered ring.
0, 1, 2, 3 or 4 $R_2$(s) are present in Formula (I), and $R_2$ is selected from H, halogen, -OH, -$NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), -N($R_{11}$)(C(=O)$R_{12}$),-C(=O)-N($R_9$)($R_{19}$), -C(=O)-$R_{12}$, -C(=O)-$OR_{12}$, -OC(=O)$R_{12}$, -N($R_{11}$)(S(=O)$_2R_{12}$), -S(= O)$_2$-N($R_9$)($R_{10}$), -$SR_{12}$ and -$OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$,-C(=O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; and
p is 1, 2 or 3; and
$R_a$ is selected from H, halogen, -OH, -$NO_2$, -CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, 5-11 membered bicyclic heteroalkyl, -N($R_9$)($R_{10}$),-N($R_{11}$)(C(=O)$R_{12}$), -C(=O)-N($R_9$)($R_{19}$), -C(=O)-$R_{12}$, -C(=O)-$OR_{12}$, -OC(=O)$R_{12}$,-N($R_{11}$)(S(=O)$_2R_{12}$), -S(=O)$_2$-N($R_9$)($R_{10}$), -$SR_{12}$ and -$OR_{12}$, in which the -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_b$ (s); and
$R_b$ is selected from H, halogen, -OH, -$NO_2$, -CN, -$SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl,

7-11 membered bicyclic heteroaryl, $-N(R_5)(R_6)$,$-N(R_{11})(C(=O)R_{12})$, $-CON(R_7)(R_8)$, $-C(=O)-R_{12}$, $-C(=O)-OR_{12}$, $-OC(=O)R_{12}$,$-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen,-CN, -OH, $C_{1-4}$ alkyl, $C_{1-6}$ alkoxy, $C_{1-4}$ haloalkyl, $C_1$-4 haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, $-C(=O)-N(R_9)(R_{10})$,$-N(R_{11})(S(=O)_2R_{12})$, $-S(=O)_2-N(R_9)(R_{10})$, $-SR_{12}$ and $-OR_{12}$; and

$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each option in the group is optionally substituted with 1, 2, 3 or 4 substituent(s) each independently selected from the group consisting of halogen, $-CF_3$, -OH, $-NH_2$, - $NH(CH_3)$, $-N(CH_3)_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C(=O)-N($C_{1-4}$ alkyl$)_2$, $C_{1-4}$ haloalkyl, $C_{1-4}$ alkoxy and $C_{1-4}$ haloalkoxy.

2. The compound, or an optical isomer thereof, a geometric isomer thereof, a tautomer thereof or a mixture of various isomers, or a pharmaceutically acceptable salt thereof, or a prodrug thereof, or a metabolite thereof according to claim 1, wherein L is C=O, O=S=O or $CH_2$.

3. The compound, or an optical isomer thereof, a geometric isomer thereof, a tautomer thereof or a mixture of various isomers, or a pharmaceutically acceptable salt thereof, or a prodrug thereof, or a metabolite thereof according to claim 1 or 2, wherein $R_1$ is -S-$C_{1-4}$ alkyl, $C_{1-6}$ alkyl, $C_{1-6}$ alkoxy, or $C_{1-6}$ haloalkoxy, wherein at least one H atom on $R^1$ is replaced by a D atom, preferably all H atoms on $R^1$ are replaced by D atoms.

4. The compound, or an optical isomer thereof, a geometric isomer thereof, a tautomer thereof or a mixture of various isomers, or a pharmaceutically acceptable salt thereof, or a prodrug thereof, or a metabolite thereof according to claim 1 or 2, wherein $R_1$ is $C_{1-6}$ alkyl, wherein at least one H atom on $R^1$ is replaced by a D atom, preferably all H atoms on $R^1$ are replaced by D atoms.

5. The compound, or an optical isomer thereof, a geometric isomer thereof, a tautomer thereof or a mixture of various isomers, or a pharmaceutically acceptable salt thereof, or a prodrug thereof, or a metabolite thereof according to any one of claims 1 to 4, wherein $R_3$ is H, $-N(R_{17})(R_{18})$, $C_{1-6}$ alkoxy, -OH, -SH, -CN, halogen, $-NO_2$, $-SF_5$, -S-$C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl, or 5-11 membered bicyclic heteroalkyl, and $R_3$ is substituted with 0, 1, 2, 3, or 4 $R_a$(s); in which $R_{17}$ and $R_{18}$ are each independently selected from H, $C_{1-6}$ alkyl, $C_{1-6}$ alkoxy, $C_{3-7}$ haloalkyl, $C_{3-7}$ cycloalkyl, $C_{3-7}$ heterocycloalkyl, $C_{5-7}$ aryl, and 5-7 membered heteroaryl, and are optionally substituted by one or more of -OH, -CN, -SH, halogen, $-NO_2$, and $-SF_5$.

6. The compound, or an optical isomer thereof, a geometric isomer thereof, a tautomer thereof or a mixture of various isomers, or a pharmaceutically acceptable salt thereof, or a prodrug thereof, or a metabolite thereof according to any one of claims 1 to 4, wherein $R_3$ is $C_{3-7}$ cycloalkyl, or 3-7 membered heterocycloalkyl and are optionally substituted by one or more of -OH, -CN, -SH, halogen, $-NO_2$, and $-SF_5$.

7. The compound, or an optical isomer thereof, a geometric isomer thereof, a tautomer thereof or a mixture of various isomers, or a pharmaceutically acceptable salt thereof, or a prodrug thereof, or a metabolite thereof according to any one of claims 1 to 4, wherein $R_3$ is $-N(R_{17})(R_{18})$, and $R_{17}$ and $R_{18}$ are each independently selected from H, $C_{1-6}$ alkyl, $C_{1-6}$ alkoxy, $C_{3-7}$ haloalkyl, and $C_{3-7}$ cycloalkyl, and are optionally substituted by one or more of -OH, - CN, -SH, halogen, $-NO_2$, and $-SF_5$

8. The compound, or an optical isomer thereof, a geometric isomer thereof, a tautomer thereof or a mixture of various isomers, or a pharmaceutically acceptable salt thereof, or a prodrug thereof, or a metabolite thereof according to any one of claims 1 to 7, wherein the compound is selected from:

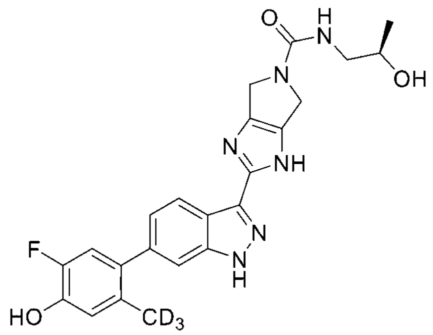

,

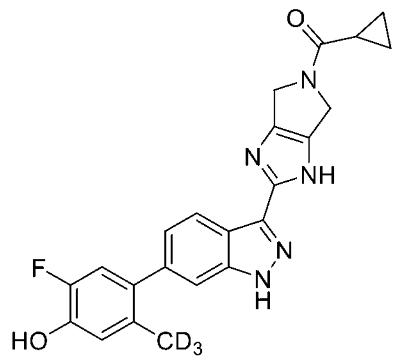

,

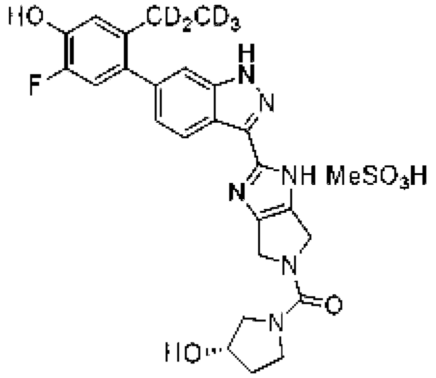

,

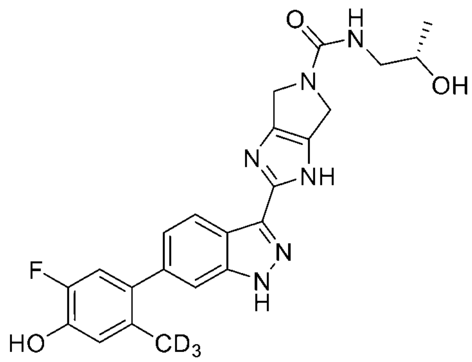

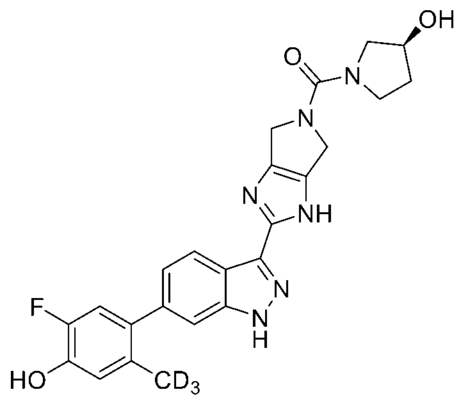

,

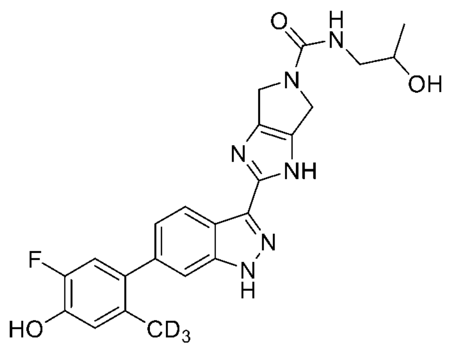

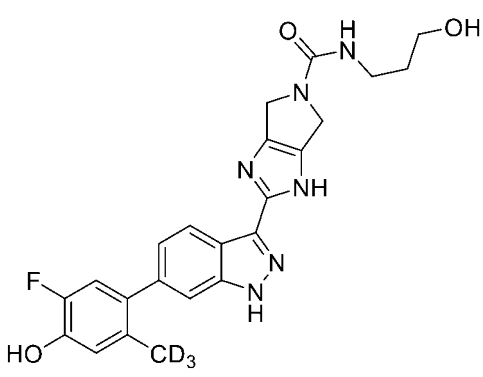

,

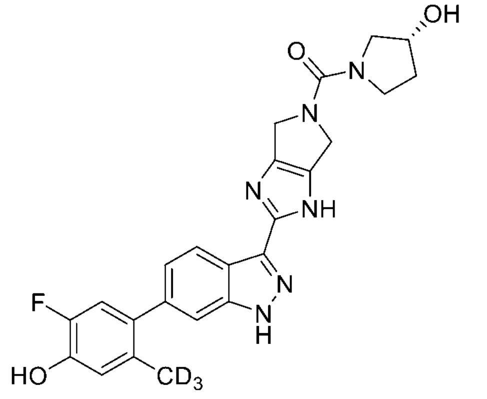

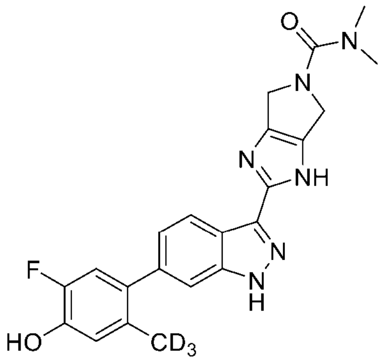
, and
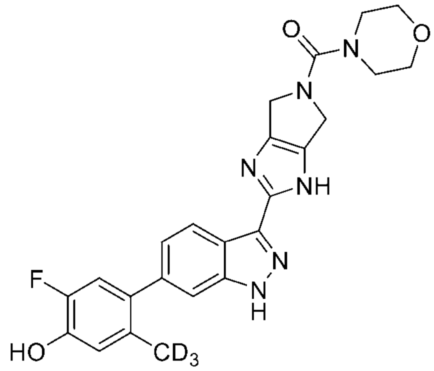
.

9. The compound, or an optical isomer thereof, a geometric isomer thereof, a tautomer thereof or a mixture of various isomers, or a pharmaceutically acceptable salt thereof, or a prodrug thereof, or a metabolite thereof according to any one of claims 1 to 7, wherein the compound is selected from:

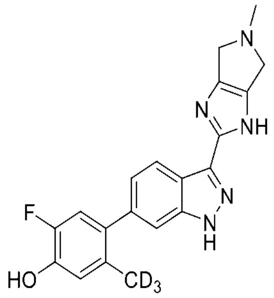
12
,
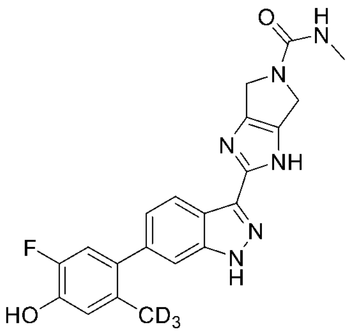
,
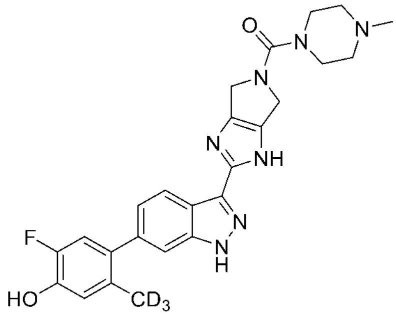
,

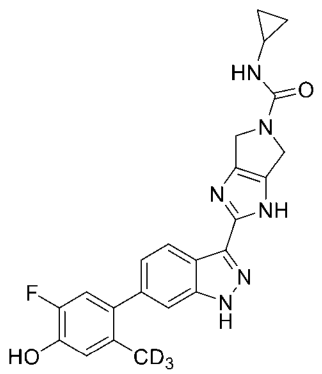
,
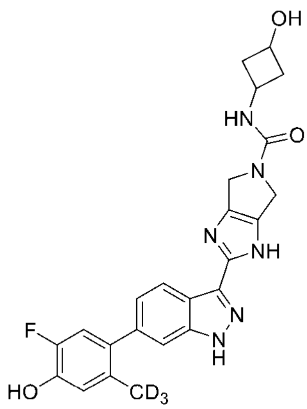
,
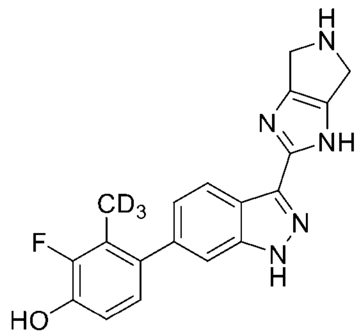
,

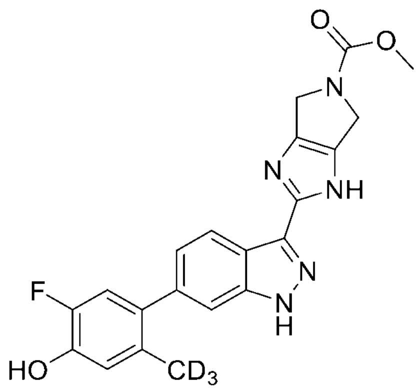
,
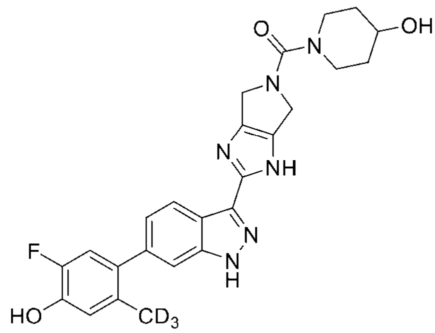
,

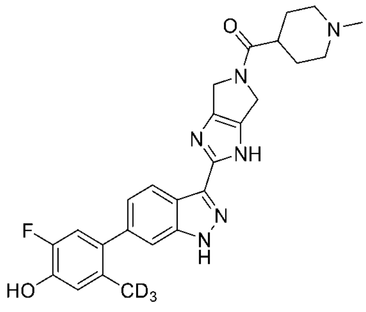 , 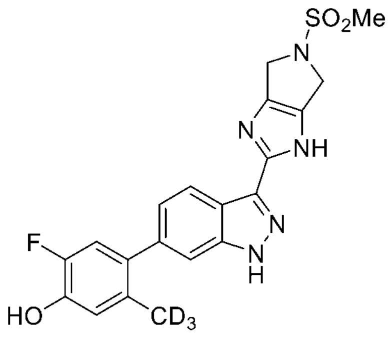 , 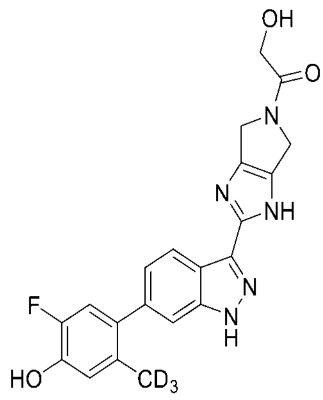 ,

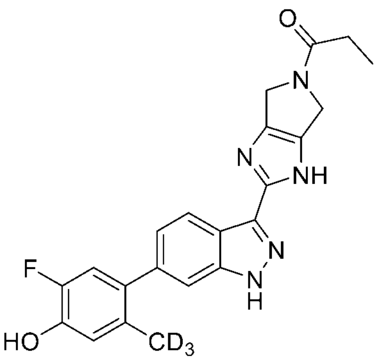 , 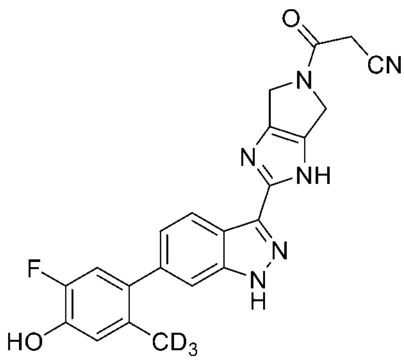 ,

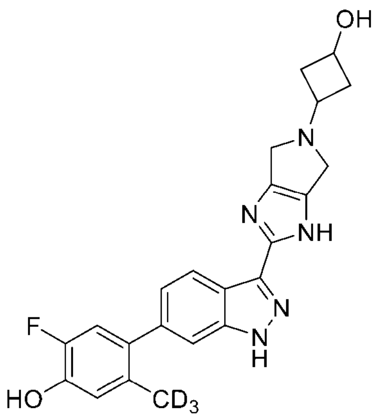 , 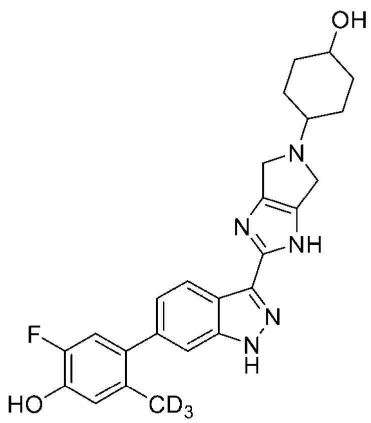 , 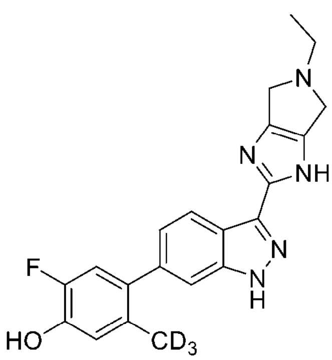 ,

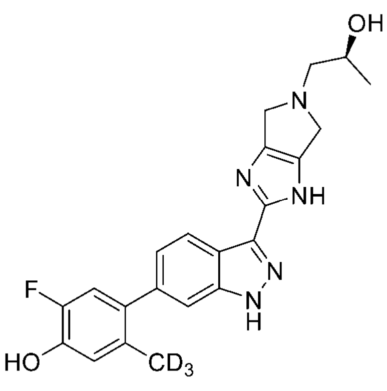 , 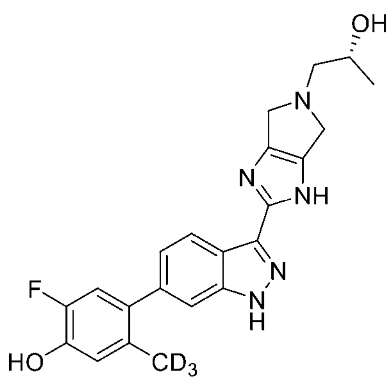 ,

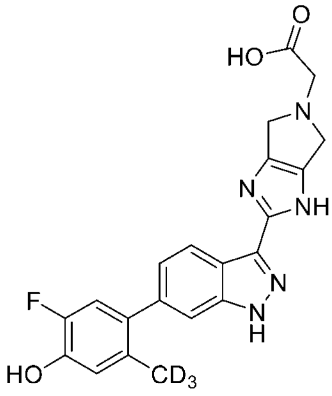

,

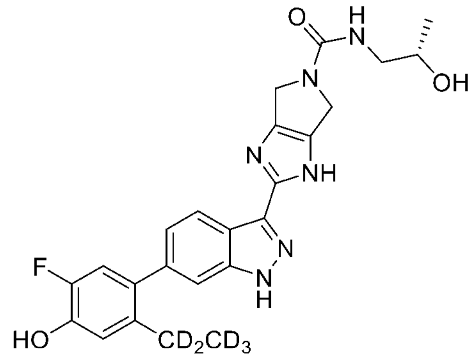

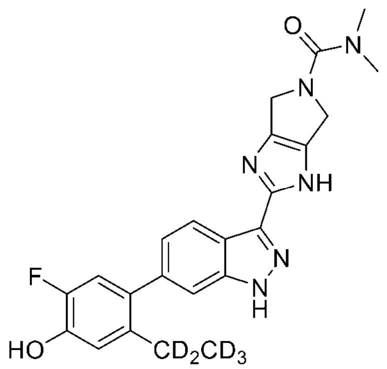

,

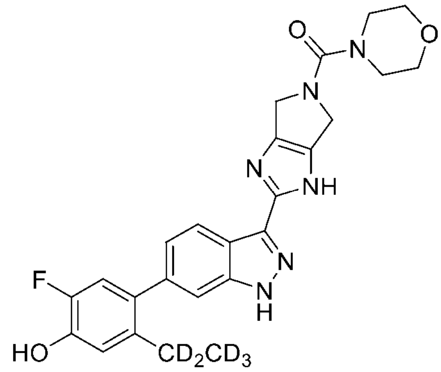

,

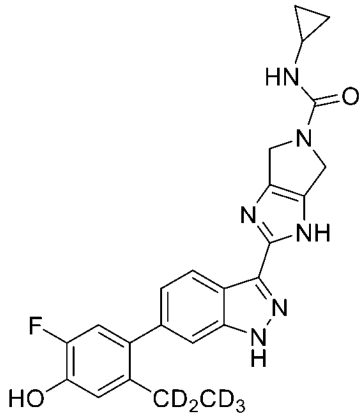

,

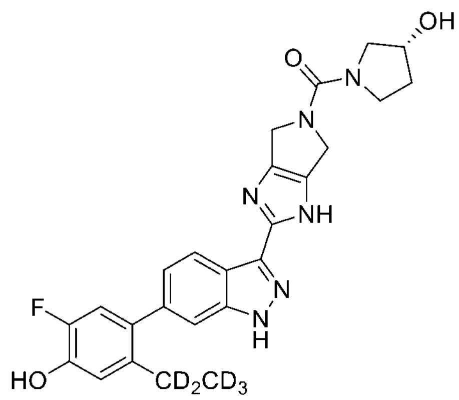

and

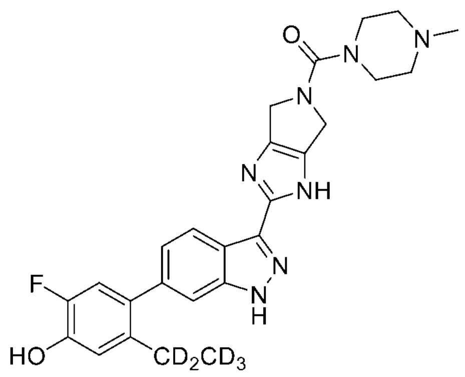

.

**10.** A pharmaceutical composition, comprising the compound, or an optical isomer thereof, a geometric isomer thereof, a tautomer thereof or a mixture of various isomers, or a pharmaceutically acceptable salt thereof, or a prodrug thereof, or a metabolite thereof according to any one of claims 1 to 10, and one or more pharmaceutically acceptable carriers, adjuvants or excipients.

**11.** Use of the compound, or optical isomer thereof, geometric isomer thereof, a tautomer thereof or a mixture of various isomers, or a pharmaceutically acceptable salt thereof, or a prodrug thereof, or a metabolite thereof according to any one of claims 1 to 9 or the pharmaceutical composition of claim 10 in the manufacture of a medicament for the treatment and/or prevention of a JAK-related disease or disorder, preferably, the JAK-related disease or disorder is selected from the group consisting of arthritis, autoimmune diseases or disorders, cancer or tumor, diabetes, eye diseases, disorders or conditions, intestinal inflammation, allergies or conditions, neurodegenerative diseases, skin diseases, conditions or disorders, allergies, asthma and other obstructive airway diseases, and transplant rejection.

**INTERNATIONAL SEARCH REPORT**

International application No.
**PCT/CN2024/111108**

**A. CLASSIFICATION OF SUBJECT MATTER**

C07D487/04(2006.01)i; C07D519/00(2006.01)i; A61K31/497(2006.01)i; A61K31/5377(2006.01)i; A61K31/4188(2006.01)i; A61K31/454(2006.01)i; A61K31/501(2006.01)i; A61K31/496(2006.01)i; A61P37/02(2006.01)i; A61P35/00(2006.01)i; A61P3/10(2006.01)i; A61P27/02(2006.01)i; A61P1/00(2006.01)i; A61P37/08(2006.01)i; A61P25/00(2006.01)i; A61P17/00(2006.01)i; A61P37/06(2006.01)i; A61P11/00(2006.01)i; A61P11/06(2006.01)i

According to International Patent Classification (IPC) or to both national classification and IPC

**B. FIELDS SEARCHED**

Minimum documentation searched (classification system followed by classification symbols)

C07D 487/-,C07D 519/-,A61K,A61P

Documentation searched other than minimum documentation to the extent that such documents are included in the fields searched

Electronic data base consulted during the international search (name of data base and, where practicable, search terms used)

CNKI, CNABS, CNTXT, ENTXT, DWPI, REGISTRY, CAPLUS, MARPAT: JAK, 吡喃, 吲唑, 吡咯, 咪唑, pyran, indazole, pyrrole, imidazole, 根据式I进行的结构式限制检索, structural formula range search conducted according to formula I.

**C. DOCUMENTS CONSIDERED TO BE RELEVANT**

| Category* | Citation of document, with indication, where appropriate, of the relevant passages | Relevant to claim No. |
|---|---|---|
| PX | WO 2024008088 A1 (HENAN MEDINNO PHARMACEUTICAL TECHNOLOGY CO., LTD.) 11 January 2024 (2024-01-11)<br>entire document | 1-11 |
| X | CA. "STN"<br>*STN REGISTRY database*, 06 July 2023 (2023-07-06),<br>RN: 2941096-28-0, 2941096-27-9, 2941096-26-8, 2941096-25-7, 2941096-24-6, 2941096-23-5, 2941096-22-4, 2941096-21-3, 2941096-20-2, 2941096-19-9, 2941096-18-8 (06.07.2023); RN: 2761301-85-1 (04.03.2022) | 1, 2, 5, 6 |
| X | WO 2020173400 A1 (HENAN MEDINNO PHARMACEUTICAL TECHNOLOGY CO., LTD.) 03 September 2020 (2020-09-03)<br>embodiments 1-34, 39-49, and 51-58, and claims 39-41 | 1-11 |
| X | WO 2022033562 A1 (HENAN MEDINNO PHARMACEUTICAL TECHNOLOGY CO., LTD.) 17 February 2022 (2022-02-17)<br>embodiments 1-33, 38-46, 48-54, and 55 | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2024** | **27 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/111108**

**C. DOCUMENTS CONSIDERED TO BE RELEVANT**

| Category* | Citation of document, with indication, where appropriate, of the relevant passages | Relevant to claim No. |
|---|---|---|
| X | WO 2022033563 A1 (HENAN MEDINNO PHARMACEUTICAL TECHNOLOGY CO., LTD.) 17 February 2022 (2022-02-17)<br>1-2, 5-6, and claims 7-11 | 1-11 |
| A | WO 2013014567 A1 (PFIZER LTD.; COE JOTHAM WADSWORTH; DEHNHARDT CHRISTOPH MARTIN; JONES PETER; KORTUM STEVEN WADE; SABNIS YOGESH ANIL; WAKENHUT FLORIAN MICHEL; WHITLOCK GAVIN ALISTAIR) 31 January 2013 (2013-01-31)<br>entire document | 1-11 |
| A | WO 2018204233 A1 (THERAVANCE BIOPHARMA R&D IP, LLC.) 08 November 2018 (2018-11-08)<br>entire document | 1-11 |

Form PCT/ISA/210 (second sheet) (July 2022)

**INTERNATIONAL SEARCH REPORT**
**Information on patent family members**

International application No.
**PCT/CN2024/111108**

| Patent document cited in search report | Publication date (day/month/year) | Patent family member(s) | Publication date (day/month/year) |
|---|---|---|---|
| WO 2024008088 A1 | 11 January 2024 | CN 117343063 A | 05 January 2024 |
| WO 2020173400 A1 | 03 September 2020 | KR 20210132666 A | 04 November 2021 |
| | | FI 3932919 T3 | 30 August 2024 |
| | | SI 3932919 T1 | 30 September 2024 |
| | | AU 2020228799 A1 | 07 October 2021 |
| | | IL 285783 A | 31 October 2021 |
| | | IL 285783 B1 | 01 August 2024 |
| | | MX 2021010242 A | 21 September 2021 |
| | | NZ 779955 A | 31 May 2024 |
| | | EP 3932919 A1 | 05 January 2022 |
| | | EP 3932919 A4 | 14 December 2022 |
| | | EP 3932919 B1 | 26 June 2024 |
| | | US 2022073524 A1 | 10 March 2022 |
| | | US 11629148 B2 | 18 April 2023 |
| | | CL 2021002244 A1 | 18 March 2022 |
| | | DK 3932919 T3 | 15 July 2024 |
| | | PE 20212069 A1 | 26 October 2021 |
| | | CA 3131293 A1 | 03 September 2020 |
| | | BR 112021016751 A2 | 19 October 2021 |
| | | BR 112021016751 B1 | 07 November 2023 |
| | | BR 112021016751 B8 | 21 November 2023 |
| | | HRP 20240994 T1 | 25 October 2024 |
| | | JP 2022523274 A | 21 April 2022 |
| | | JP 7514025 B2 | 10 July 2024 |
| | | PT 3932919 T | 30 July 2024 |
| | | ZA 202106511 B | 31 August 2022 |
| | | US 2023192711 A1 | 22 June 2023 |
| | | CN 111606908 A | 01 September 2020 |
| | | CN 113563345 A | 29 October 2021 |
| WO 2022033562 A1 | 17 February 2022 | EP 4186907 A1 | 31 May 2023 |
| | | TW 202206434 A | 16 February 2022 |
| | | JP 2023536891 A | 30 August 2023 |
| WO 2022033563 A1 | 17 February 2022 | EP 4186908 A1 | 31 May 2023 |
| | | EP 4186908 A4 | 03 January 2024 |
| | | TW 202206435 A | 16 February 2022 |
| | | JP 2023536892 A | 30 August 2023 |
| | | JP 7554007 B2 | 19 September 2024 |
| WO 2013014567 A1 | 31 January 2013 | DOP 2014000016 A | 15 July 2014 |
| | | CU 20140009 A7 | 26 March 2014 |
| | | MX 2014001004 A | 13 May 2014 |
| | | NI 201400005 A | 23 April 2014 |
| | | BR 112014001801 A2 | 14 February 2017 |
| | | EP 2736907 A1 | 04 June 2014 |
| | | EP 2736907 B1 | 07 October 2015 |
| | | KR 20140026627 A | 05 March 2014 |
| | | AR 087348 A1 | 19 March 2014 |
| | | CO 6862160 A2 | 10 February 2014 |
| | | AP 201407372 D0 | 31 January 2014 |
| | | TW 201313712 A | 01 April 2013 |
| | | TWI 462922 B | 01 December 2014 |

Form PCT/ISA/210 (patent family annex) (July 2022)

**INTERNATIONAL SEARCH REPORT**
**Information on patent family members**

International application No.
**PCT/CN2024/111108**

| Patent document cited in search report | Publication date (day/month/year) | Patent family member(s) | Publication date (day/month/year) |
|---|---|---|---|
| | | JP 5579351 B1 | 27 August 2014 |
| | | JP 2014522865 A | 08 September 2014 |
| | | AU 2012288491 A1 | 30 January 2014 |
| | | ECSP 14013222 A | 31 March 2014 |
| | | UY 34220 A | 28 February 2013 |
| | | CA 2841882 A1 | 31 January 2013 |
| | | CR 20140042 A | 19 March 2014 |
| | | GT 201400016 A | 19 February 2015 |
| | | UA 108442 C2 | 27 April 2015 |
| | | EA 201490357 A1 | 30 May 2014 |
| | | IL 230662 A0 | 31 March 2014 |
| | | US 2013029968 A1 | 31 January 2013 |
| | | US 8575336 B2 | 05 November 2013 |
| | | MA 35285 B1 | 03 July 2014 |
| | | CL 2014000122 A1 | 18 August 2014 |
| | | US 2014024634 A1 | 23 January 2014 |
| | | US 8895544 B2 | 25 November 2014 |
| | | AP 2014007372 A0 | 31 January 2014 |
| | | MD 20140002 A2 | 30 June 2014 |
| | | PE 20141059 A1 | 30 August 2014 |
| | | TN 2014000033 A1 | 01 July 2015 |
| WO 2018204233 A1 | 08 November 2018 | AU 2018261588 A1 | 31 October 2019 |
| | | PH 12019502345 A1 | 07 December 2020 |
| | | CA 3059785 A1 | 08 November 2018 |
| | | EP 3609498 A1 | 19 February 2020 |
| | | SG 11201909019 SA | 30 October 2019 |
| | | US 2020121669 A1 | 23 April 2020 |
| | | US 11160800 B2 | 02 November 2021 |
| | | RU 2019138463 A | 02 June 2021 |
| | | RU 2019138463 A3 | 03 August 2021 |
| | | RU 2764979 C2 | 24 January 2022 |
| | | NZ 758109 A | 24 September 2021 |
| | | US 2018311226 A1 | 01 November 2018 |
| | | US 10406148 B2 | 10 September 2019 |
| | | CL 2019003126 A1 | 24 January 2020 |
| | | ZA 201906817 B | 27 October 2021 |
| | | US 2019350916 A1 | 21 November 2019 |
| | | US 10548886 B2 | 04 February 2020 |
| | | BR 112019022665 A2 | 19 May 2020 |
| | | JP 2022120147 A | 17 August 2022 |
| | | MX 2019012950 A | 16 December 2019 |
| | | US 2022008403 A1 | 13 January 2022 |
| | | US 11786517 B2 | 17 October 2023 |
| | | JP 2020518579 A | 25 June 2020 |
| | | JP 7153031 B2 | 13 October 2022 |
| | | KR 20190140062 A | 18 December 2019 |
| | | KR 102568333 B1 | 18 August 2023 |
| | | TW 201900170 A | 01 January 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311017417
- US 6313 A
- US 129 A


### Non-patent literature cited in the description


- **KISSELEVA et al.** *Gene*, 2002, vol. 285, 1
- **YAMAOKA et al.** *Genome Biology*, 2004, vol. 5, 253
- **SANTOS et al.** *Blood*, 2010, vol. 115, 1131
- **BAROSI G** ; **ROSTI V.** *Curr. Opin. Hematol.*, 2009, vol. 16, 129
- **ATALLAH E** ; **VERSOTVSEK S.** *Exp. Rev. Anticancer Ther*, 2009, vol. 9, 663
- **BORIE et al.** *Curr. Opin. Investigational Drugs*, 2003, vol. 4, 1297
- **STAHL** ; **WERMUTH**. Handbook of Pharmaceutical Salts: Properties, Selection and Use. Wiley-VCH, 2002